(19) Europäisches Patentamt / European Patent Office / Office européen des brevets

(11) **EP 4 108 779 A1**

(12) **EUROPEAN PATENT APPLICATION**
published in accordance with Art. 153(4) EPC

(43) Date of publication:
**28.12.2022 Bulletin 2022/52**

(21) Application number: **20918272.4**

(22) Date of filing: **13.10.2020**

(51) International Patent Classification (IPC):
***C12Q 1/68*** [(2018.01)]

(52) Cooperative Patent Classification (CPC):
**C12Q 1/68; C12Q 1/6883; G16H 50/20**

(86) International application number:
**PCT/CN2020/120654**

(87) International publication number:
**WO 2021/159722 (19.08.2021 Gazette 2021/33)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.02.2020 CN 202010084924**

(71) Applicant: **Beijing Euler Technology Limited
Company
Beijing 102206 (CN)**

(72) Inventors:
• **ZHANG, Yi
Changping District, Beijing 102206 (CN)**
• **WU, Kai
Changping District, Beijing 102206 (CN)**
• **JIN, Wan
Changping District, Beijing 102206 (CN)**

(74) Representative: **Bardehle Pagenberg
Partnerschaft mbB
Patentanwälte Rechtsanwälte
Prinzregentenplatz 7
81675 München (DE)**

Remarks:
The complete document including Reference Table(s) and the Sequence Listing(s) can be downloaded from the EPO website

(54) **METHOD FOR EVALUATING AND PREDICTING PLACENTA-DERIVED DISEASES AND KIT**

(57) The present disclosure relates to a method for evaluating and predicting a placenta-derived disease and a kit. Specifically, the invention of the present disclosure provides a method for detecting the methylation modification level of at least one DNA methylation modification difference locus or at least one DNA methylation haplotype in a given genomic position in a sample to be tested, a kit, and a corresponding detection system, which can be used for accurately screening, *in vitro,* whether a subject has a placenta-derived gestational disorder.

FIG. 16

Processed by Luminess, 75001 PARIS (FR)

**Description**

**TECHNICAL FIELD**

The present disclosure relates to the field of biological detection. Specifically, the present disclosure relates to a method for predicting and evaluating the risk of pregnancy complications associated with placental dysplasia, and a kit. More specifically, the present disclosure relates to a method for evaluating and predicting, at early pregnancy (12 to 16 weeks of gestation), the risk of pregnant women being afflicted with gestational hypertension and other types of placenta-derived diseases in the perinatal period, and a kit. More specifically, the present disclosure relates to a method for evaluating and predicting the risk of preeclampsia, and a kit.

**BACKGROUND**

Formation of a placenta is a vital guarantee for normal mammalian pregnancy and healthy fetal growth. The placenta is a maternal-fetal interface and plays important roles in, e.g., providing necessary nutrition support for a fetus and regulating maternal immune tolerance. From the standpoint of embryonic development, the placenta originates from the extraembryonic ectoderm (ExE). Its formation course starts with implantation of the blastocyst, i.e., 6 to 7 days after fertilization, and its development course continues throughout the gestation. The blastocyst ready for implantation has differentiated into the inner cell mass (ICM) with the potential to develop into an embryo and the trophoectoderm (TE) with the potential to differentiate into a placental tissue. Placental growth and development involves such biological processes as degradation of extracellular matrix, trophoblastic invasion, cell differentiation of placental lineage, promotion of angiogenesis, cell migration, and regulation of tolerance of the maternal immune system. In addition, the placental development is also accompanied by dynamic epigenetic processes, such as a remodeling process of whole-genome methylation and regulation of expression levels by genomic imprinting, which plays important roles in determining the fate of the cell lineage and regulating the expression of placental development-associated genes and the embryonic development.

DNA methylation modification is to transfer a methyl group to the fifth carbon atom of cytosine under the action of methyltransferases to form 5-methylcytosine. Most of DNA methylation modifications occur at CpG loci, with very few occurring at CHG or CHH loci (H represents C, T, or A). DNA methylation modifications are roughly similar in cells from the same developmental lineage. When cells undergo differentiation, transdifferentiation, canceration, etc., the DNA methylation modifications will be altered. Under different physiological and pathological conditions, the DNA methylation modification to the same cell may also be altered. Therefore, the pattern of the DNA methylation modification may be used to deduce the origin of its tissue and to evaluate the pathological and physiological status of the tissue cell where it is found.

During the development of zygote, the DNA methylation modification is a highly dynamic process. In a few hours after zygote formation, an erasure of DNA methylation occurs progressively within a whole-genome range to thereby reduce the mean whole-genome methylation level from approximately 41% after fertilization to 9% at the 2-cell stage. The genome of blastocyst is almost completely hypo-methylated prior to implantation. The unerased methylation modification is known as methylation imprinting. After implantation of blastocyst, the *de novo* methylation of DNA will occur, which is closely related to the regulation of the expression of embryonic development-associated genes. During the *de novo* methylation, intra- and extra-embryonic tissues show different patterns, with 80% of methylated loci being differentially modified at the whole-genome level in the intra- and extra-embryonic tissues; for an individual methylated locus, the DNA methylation modification in the intra-embryonic tissue is in an "all-or-none" pattern, i.e., a state that a particular methylated locus is completely methylated or completely hypo-methylated, whereas a majority of CpG loci in the extra-embryonic tissues are hemi-methylated.

For the vast majority of genes on autosomes, alleles inherited from both paternal and maternal sources are expressed simultaneously. However, there are a few genes, in which either the paternal or maternal allele is expressed. Such monoallelic expression, i.e., a genomic imprinting phenomenon, is realized by asymmetric epigenetic modifications between different parents. The occurrence of imprinted gene is of significance to normal tissue differentiation and embryonic development. For example, fetal growth restriction (FGR) is associated with the loss of genomic imprinting on *IGF2,* which, in turn, leads to an undersized placenta or villous hypoplasia. The genomic imprinting can be realized by differential methylation modifications of the paternal and maternal genomes. The methylation modifications to imprinted genes are regulated by an imprinting control region (ICR). The ICR may remain methylated during the whole-genome erasure of methylations in the early development of a zygote, and the methylated ICR may affect surrounding genes such that they are methylation modified as well. Most of the ICRs in the oocyte are methylated, and they are mostly distributed in the CpG island (CGI) containing dense methylated loci. In the early stage of embryonic development, genes are transcribed with an alternative promoter. The alternative promoters are upstream of the classic promoter on the CGI, and are mostly retrotransposons with a long terminal repeat (LTR), also referred to as endoretroviruses (ERVs).

ERVs make up approximately 8% of the human genome. Human-derived ERVs lose their retrotransposon activity but retain their function as alternative promoters. For example, the transcription of the *CYP19A1* gene in the placenta is driven by a tissue-specific promoter -- MER21 LTR, which belongs to the ERV family and enables placental cells to express tissue-specific transcripts through an alternative splicing mechanism.

Preeclampsia (PE) is a common pregnancy complication that may seriously threaten lives of mother and fetus in the perinatal period. In China, the morbidity of preeclampsia is approximately 2% to 6%, and preeclampsia is the second leading cause of maternal death. In clinic, preeclampsia is typically characterized by first onset of hypertension and proteinuria after 20 weeks of gestation, often accompanied by edema and hyperuricemia. Onset of preeclampsia will cause fatal damages to the liver function, kidney function, and coagulation function of pregnant women. Pregnant women with severe preeclampsia may develop microangiopathic hemolytic anemia, elevated lactate dehydrogenase and liver enzymes, headache, or other neurological symptoms.

It is now generally acceptable that preeclampsia is mainly caused by inadequate invasion of trophoblast into the uterine decidua and inadequate remodeling of distal uterine spiral arteries. However, there is still no clinical means of treating preeclampsia and the only cure is induction of labor. Several clinical trials have demonstrated that taking low-dose aspirin before 16 weeks of gestation may reduce the morbidity of preeclampsia. In order to relieve the pressure of clinical management in preeclampsia, strengthen the early prevention of preeclampsia, and reduce its morbidity, there is a need to strengthen early screening of preeclampsia. Previous scientific research has shown that Congo red dye could bind to the β-amyloid protein in urine of pregnant women with preeclampsia, and turns red. In the plasma of patients with preeclampsia, as the placental development-associated proteins, the PIGF level is decreased, while the sFlt1 level is increased. Therefore, the Congo red staining result and the PIGF/sFlt1 ratio may be used as indicators for clinical diagnosis and prediction of preeclampsia. However, the above biochemical assay is only applicable to people at 25 weeks of gestational age or more but not to people at less than 16 weeks of gestational age. Because aspirin intervention is only effective before 16 weeks of gestational age, the above method is relatively limited in guiding physicians on medication and preventing preeclampsia. Scientific findings have shown that there is a significant difference in DNA methylation modifications between the preeclamptic placenta and the placenta in normal pregnancy.

Cell-free DNA (cfDNA) is a single- or double-stranded DNA fragment present in human circulating blood, urine, and other body fluids. Most of cfDNAs are 200 bp or less in length, and they are present in plasma at very low concentrations (about 1 to about 100 ng/mL). cfDNA results mainly from apoptosis because its fragmentation pattern is very similar to DNA fragments generated by apoptosis. 90% or more of cfDNAs are derived from cells of the hematopoietic lineage. The cfDNAs in maternal plasma is a mixture of maternal and placental cfDNAs, of which fetal/placental cfDNAs are in a smaller proportion (proportion of placental cfDNAs: 3% to 15%). Existing methods for prenatal diagnosis have realized early screening for fetal genetic diseases, e.g., chromosomal polyploidization diseases such as Down's syndrome (T21), by deep sequencing of cfDNAs in maternal plasma.

In the prior art, a large number of clinical trials have been conducted to predict the risk of pregnancy complications associated with placental dysplasia by using, alone or in combination, a biochemical assay, a biophysical assay, and epidemiological statistics. However, subject to their sensitivity and specificity, these clinical trials also fail to achieve effective evaluation and prediction of the onset risks of the aforesaid diseases at an earlier gestational age. To date, there have been no proven and reliable screening methods used in clinical practice.

## SUMMARY

Technical Problem

In view of the deficiencies in the prior art, in one embodiment, the present disclosure provides use of a reagent for detecting a methylation modification level of at least one DNA methylation modification difference locus or at least one DNA methylation haplotype in a sample to be tested in the preparation of a reagent or kit for detecting, monitoring, and/or predicting whether a subject has a placenta-derived gestational disorder.

In another embodiment, the present disclosure provides a kit for detecting a methylation modification level of at least one DNA methylation modification difference locus or at least one DNA methylation haplotype in a sample.

In another embodiment, the present disclosure provides a polynucleotide usable for detecting presence of a DNA methylation modification.

In another embodiment, the present disclosure provides a method for detecting a methylation modification level of at least one DNA methylation modification difference locus or at least one DNA methylation haplotype in a sample to be tested.

In another embodiment, the present disclosure provides a method for detecting, monitoring, and/or predicting whether a subject has a placenta-derived gestational disorder.

In another embodiment, the present disclosure provides a device for detecting, monitoring, and/or predicting whether a subject has a placenta-derived gestational disorder, and a non-volatile computer readable storage medium.

In another embodiment, the present disclosure provides a system for detecting a methylation modification level of at least one DNA methylation modification difference locus or at least one DNA methylation haplotype in a sample to be tested.

In another embodiment, the present disclosure provides a system for detecting, monitoring, and/or predicting whether a subject has a placenta-derived gestational disorder.

Solution to Problem

The present disclosure provides the following inventions:

(1) Use of a reagent for detecting at least one specific DNA methylation modification or at least one specific DNA methylation haplotype carried in a specific human genomic region in a sample to be tested in the preparation of a reagent or kit for detecting, monitoring, or predicting whether a subject has a placenta-derived gestational disorder, wherein the specific DNA methylation modification or the specific DNA methylation haplotype is derived from methylation difference regions of a placenta of a pregnant woman in normal pregnancy and a placenta of a pregnant woman a placenta-derived gestational disorder.

(2) The use according to (1), wherein the sample to be tested is derived from the subject; the methylation difference region is a genomic region listed in Attached List 1;

the specific DNA methylation modification is a methylation modification at a specific CpG locus in a genomic region listed in Attached List 1;
the specific methylation haplotype is a DNA methylation haplotype covered by the genomic region listed in Attached List 1; and
the specific DNA methylation modification or the specific methylation haplotype is capable of distinguishing the placenta of the pregnant woman in normal pregnancy from the placenta of the pregnant woman with the placenta-derived gestational disorder.

(3) The use according to (1) or (2), wherein the sample to be tested is derived from the subj ect;
the specific DNA methylation modification or the specific DNA methylation haplotype detected is located in one or more regions selected from the following regions (i) to (iii):

(i) a region of LTR12 transposon family; preferably, the LTR12 transposon is an LTR12C transposon or an LTR12E transposon;
(ii) an FLT1 gene and a regulatory region thereof; preferably, the FLT1 gene and the regulatory region thereof are a region between human chromosome chr13: 28800000 bp and 302000000 bp; and
(iii) an LIFR gene and a regulatory region thereof; preferably, the LIFR gene and the regulatory region thereof are a region between human chromosome chr5: 38370000 bp and 38840000 bp.

(4) The use according to any one of (1) to (3), wherein the sample to be tested is selected from samples derived from blood, urine, feces, saliva, oral swabs, cervical secretions, cervical smears, amniocentesis, fetal villi, or fetal circulating cells; preferably, the blood is peripheral blood, and more preferably, the peripheral blood is plasma.

(5) The use according to any one of (1) to (4), wherein the placenta-derived gestational disorder is selected from one or more of gestational diabetes, twin-to-twin transfusion syndrome, fetal growth restriction, gestational hypertension, preeclampsia, severe preeclampsia, secondary preeclampsia, atypical preeclampsia, and HELLP syndrome; preferably, the gestational disorder is selected from one or more of preeclampsia, severe preeclampsia, secondary preeclampsia, and atypical preeclampsia.

(6) The use according to any one of (1) to (5), wherein the reagent for detecting the methylation difference regions derived from the placenta of the pregnant woman in normal pregnancy and the placenta of the pregnant woman with the placenta-derived gestational disorder is selected from a DNA methylation status indicator; or

a reagent for detecting presence of a DNA methylation modification in a sample from the subj ect;
preferably, the reagent for detecting the presence of the DNA methylation modification in the sample from the subject is a reagent required for enriching characteristic DNA.

(7) The use according to (6), wherein the DNA methylation status indicator is selected from an antibody or binding protein identifying methylated DNA, bisulfite, enzymes with DNA catalytic oxidation, enzymes with DNA deamination, or a methylation-sensitive enzyme, or a combination thereof; preferably, the methylation-sensitive enzyme is selected from a methylation sensitive restriction endonuclease, and more preferably, the methylation sensitive restriction endonuclease is selected from HpaII or BstUI, or a combination thereof.

(8) The use according to (6), wherein the reagent required for enriching the characteristic DNA is selected from a reagent required by a hybrid capture method, a reagent required by a polymerase chain reaction amplification method, a reagent required by an anchored nucleic acid amplification method, a reagent for sequencing by synthesis, or a reagent for single-molecule sequencing, or a combination thereof.

(9) The use according to (8), wherein the reagent required for enriching the characteristic DNA is a probe or probe set; preferably, the probe or probe set is an EP-007 probe or probe set.

(10) The use according to any one of (1) to (9), wherein the detection is prenatal detection; preferably, the prenatal detection is non-invasive prenatal detection; and more preferably, the prenatal detection is non-invasive prenatal detection in early pregnancy.

(11) The use according to any one of (1) to (10), wherein the DNA is gDNA or cfDNA.

(12) A kit for detecting at least one specific DNA methylation modification or at least one specific DNA methylation haplotype in a sample to be tested to detect, monitor, or predict whether a subject has a placenta-derived gestational disorder, the kit comprising:

> (a) a reagent for detecting presence of a DNA methylation modification;
> optionally, the kit further comprising:
> (b) an indicator for detecting a DNA methylation status;
> wherein the specific DNA methylation modification or the specific DNA methylation haplotype is derived from methylation difference regions of a placenta of a pregnant woman in normal pregnancy and a placenta of a pregnant woman with the placenta-derived gestational disorder.

(13) The kit according to (12), wherein the specific human genomic region is a genomic region listed in Attached List 1; the specific methylation modification is a methylation modification carried in a DNA fragment derived from the genomic region listed in Attached List 1; wherein the methylation modification carried may comprise a methylation modification at a specific CpG locus or a specific DNA methylation haplotype in the specific human genomic region; the methylation difference region is a genomic region listed in Attached List 1;

> the specific DNA methylation modification is a methylation modification at a specific CpG locus in a genomic region listed in Attached List 1;
> the specific methylation haplotype is a DNA methylation haplotype covered by the genomic region listed in Attached List 1; and
> the specific DNA methylation modification or the specific methylation haplotype is capable of distinguishing the placenta of the pregnant woman in normal pregnancy from the placenta of the pregnant woman with the placenta-derived gestational disorder.

(14) The kit according to (12) or (13), wherein the reagent for detecting the presence of the DNA methylation modification is a reagent required for enriching a characteristic DNA; preferably, the reagent required for enriching the characteristic DNA is a probe or probe set; and more preferably, the probe or probe set is an EP-007 probe or probe set.

(15) The kit according to any one of (12) to (14), wherein the DNA methylation status indicator is selected from an antibody or binding protein identifying methylated DNA, bisulfite, enzymes with DNA catalytic oxidation, enzymes with DNA deamination, or a methylation-sensitive enzyme, or a combination thereof; preferably, the methylation-sensitive enzyme is selected from a methylation sensitive restriction endonuclease, and more preferably, the methylation sensitive restriction endonuclease is selected from HpaII or BstUI, or a combination thereof.

(16) The kit according to any one of (12) to (15), wherein the sample to be tested is selected from samples derived from blood, urine, feces, saliva, oral swabs, cervical secretions, cervical smears, amniocentesis, fetal villi, or fetal circulating cells; preferably, the blood is peripheral blood, and more preferably, the peripheral blood is plasma.

(17) The kit according to any one of (12) to (16), wherein the detection is prenatal detection; preferably, the prenatal detection is non-invasive prenatal detection; and more preferably, the prenatal detection is non-invasive prenatal detection in early pregnancy.

(18) The kit according to any one of (12) to (17), wherein the DNA is gDNA or cfDNA.

(19) A polynucleotide capable of:

(i) hybridizing to a polynucleotide indicated by an EP-007 probe or probe set under high-stringency hybridization conditions or under very high-stringency hybridization conditions; or (ii) having a sequence reversely complementary to the sequence of the polynucleotide as shown in (i), wherein the polynucleotide is used for detecting at least one specific DNA methylation modification or at least one specific DNA methylation haplotype in a sample.

(20) The polynucleotide according to (19), which has a sequence having at least 90%, optionally at least 95%, preferably at least 97%, more preferably at least 98%, or most preferably at least 99% sequence identity to a nucleotide sequence indicated by the EP-007 probe or probe set or a sequence reversely complementary thereto.

(21) The polynucleotide according to (19) or (20), wherein the sequence of the polynucleotide comprises the nucleotide sequence indicated by the EP-007 probe or probe set; preferably, the sequence of the polynucleotide is the nucleotide sequence indicated by the EP-007 probe or probe set.

(22) The polynucleotide according to (19) or (20), wherein the polynucleotide is a DNA probe.

(23) A method for detecting whether at least one DNA fragment with a characteristic of a placenta-derived gestational disorder in a specific human genomic region is present in a sample to be tested, the method comprising a detecting step for detecting:

(a) whether at least one methylation modification at a specific CpG locus with a characteristic of the placenta-derived gestational disorder or an abundance of the modification thereof is present in the sample to be tested; or
(b) whether at least one DNA methylation haplotype with a characteristic of the placenta-derived gestational disorder or an abundance of the DNA methylation haplotype is present in the sample to be tested,

wherein the methylation modification at the specific CpG locus or the DNA methylation haplotype with the characteristic of the placenta-derived gestational disorder is derived from methylation difference regions of a placenta of a pregnant woman in normal pregnancy and a placenta of a pregnant woman with the placenta-derived gestational disorder.

(24) The method according to (23), wherein the methylation modification at the specific CpG locus with the characteristic of the placenta-derived gestational disorder takes place at a locus covered by a genomic region listed in Attached List 1; and
the DNA methylation haplotype is a DNA methylation haplotype covered by a genomic region listed in Attached List 1.

(25) The method according to (23) or (24), wherein the specific DNA methylation modification or the specific DNA methylation haplotype detected is located in one or more regions selected from the following regions (i) to (iii):

(i) a region of LTR12 transposon family; preferably, the LTR12 transposon is an LTR12C transposon or an LTR12E transposon;
(ii) an FLT1 gene and a regulatory region thereof; preferably, the FLT1 gene and the regulatory region thereof are a region between human chromosome chr13: 28800000 bp and 302000000 bp; and
(iii) an LIFR gene and a regulatory region thereof; preferably, the LIFR gene and the regulatory region thereof are a region between human chromosome chr5: 38370000 bp and 38840000 bp.

(26) The method according to any one of (23) to (25), which detects said (a) or (b) by a reagent for detecting presence of a DNA methylation modification, wherein the reagent for detecting the presence of the DNA methylation modification is a reagent required for enriching a characteristic DNA; preferably, the reagent required for enriching the characteristic DNA is a probe or probe set; and more preferably, the probe or probe set is an EP-007 probe or probe set.

(27) The method according to any one of (23) to (26), wherein the method further comprises a step of extracting DNA from the sample to be tested, and the sample to be tested is a peripheral blood sample; and preferably, the peripheral blood sample is a plasma sample.

(28) The method according to any one of (23) to (27), wherein the DNA is gDNA or cfDNA.

(29) A method for detecting, monitoring, or predicting whether a subject has a placenta-derived gestational disorder, the method comprising the following steps:

(1) a detecting step of:

(a1) detecting whether at least one methylation modification at a specific CpG locus with a characteristic of the placenta-derived gestational disorder or an abundance of the modification thereof is present in the sample to be tested, wherein the DNA methylation modification to the characteristic of the placenta-derived gestational disorder is derived from methylation difference regions of a placenta of a pregnant woman in normal pregnancy and a placenta of a pregnant woman with the placenta-derived gestational disorder; or
(b1) detecting whether a DNA methylation haplotype with a characteristic of the placenta-derived gestational disorder or an abundance of the DNA methylation haplotype is present in the sample to be tested, wherein the DNA methylation haplotype is derived from methylation difference regions of a placenta of a pregnant woman in normal pregnancy and a placenta of a pregnant woman with the placenta-derived gestational disorder;

(2) a comparing step of:

(a2) comparing a detection result of the methylation modification at the specific CpG locus in the sample to be tested or of the abundance of modification thereof with a detection result of a methylation modification at a specific CpG locus in a placental sample with or without a placenta-derived gestational disorder or of an abundance of modification thereof; or

(b2) comparing a detection result of the DNA methylation haplotype in the sample to be tested or of the abundance of the DNA methylation haplotype with a detection result of a DNA methylation haplotype in a placental sample with or without a placenta-derived gestational disorder or of an abundance of the DNA methylation haplotype;

(3) a determining step of:

(i) determining that the subject has the placenta-derived gestational disorder if in (a2) or (b2), a similarity between the detection result of the sample to be tested and the detection result of the placental sample without the placenta-derived gestational disorder is lower or a statistic value of the similarity is significantly lower than expected, or a similarity between the detection result of the sample to be tested and the detection result of the placental sample with the placenta-derived gestational disorder is higher or a statistic value of the similarity is significantly higher than expected;

(ii) determining that the subject does not have the placenta-derived gestational disorder if in (a2) or (b2), a similarity between the detection result of the sample to be tested and the detection result of the placental sample without the placenta-derived gestational disorder is higher or a statistic value of the similarity is significantly higher than expected, or a similarity between the detection result of the sample to be tested and the detection result of the placental sample with the placenta-derived gestational disorder is lower or a statistic value of the similarity is significantly lower than expected.

In a specific embodiment, the sample to be tested is derived from the subject.

(30) The method according to (29), wherein the methylation modification at the specific CpG locus with the characteristic of the placenta-derived gestational disorder takes place at a locus covered by a genomic region listed in Attached List 1; and the DNA methylation haplotype is a DNA methylation haplotype covered by a genomic region listed in Attached List 1.

(31) The method according to (29) or (30), wherein the methylation modification at the specific CpG locus or specific DNA methylation haplotype detected is located in one or more regions selected from the following regions (i) to (iii):

(i) a region of LTR12 transposon family; preferably, the LTR12 transposon is an LTR12C transposon or an LTR12E transposon;

(ii) an FLT1 gene and a regulatory region thereof; preferably, the FLT1 gene and the regulatory region thereof are a region between human chromosome chr13: 28800000 bp and 302000000 bp; and

(iii) an LIFR gene and a regulatory region thereof; preferably, the LIFR gene and the regulatory region thereof are a region between human chromosome chr5: 38370000 bp and 38840000 bp.

(32) The method according to any one of (29) to (31), wherein the placenta-derived gestational disorder is selected from one or more of gestational diabetes, twin-to-twin transfusion syndrome, fetal growth restriction, gestational hypertension, preeclampsia, severe preeclampsia, secondary preeclampsia, atypical preeclampsia, and HELLP syndrome; preferably, the gestational disorder is selected from one or more of preeclampsia, severe preeclampsia, secondary preeclampsia, and atypical preeclampsia.

(33) The method according to any one of (29) to (32), wherein a methylated region specific to the placenta-derived gestational disorder includes a hyper-methylated region and a hypo-methylated region.

(34) The method according to any one of (29) to (33), wherein the method for determining similarity statistics adopted in step (3) is selected from: correlation, t test, Z test, hypergeometric test, Fourier analysis, Wavelet analysis, Principal Component Analysis (PCA), t-Distributed Stochastic Neighbor Embedding (tSNE), Non-Negative Matrix Factorization (NMF), Support Vector Machine (SVM), k-Nearest Neighbor (KNN), k-means, Linear Model (LM), Generalized Linear Model (GLM), Gaussian Mixed Model (GMM), Neural Networks (NN), Random Forest (RF), Autoencoder, Deep Neural Networks (DNN) and variants thereof.

(35) The method according to any one of (29) to (34), wherein a reagent for detecting presence of a DNA methylation modification is used to detect the methylation modification at the specific CpG locus or the abundance of modification thereof; or to detect the specific DNA methylation haplotype or the abundance of the DNA methylation haplotype.

(36) The method according to (35), in which the detection is performed by a reagent for detecting presence of a DNA methylation modification, wherein the reagent for detecting the presence of the DNA methylation modification is a reagent required for enriching a characteristic DNA; preferably, the reagent required for enriching the characteristic DNA is a probe or probe set; and more preferably, the probe or probe set is an EP-007 probe or probe set.

(37) The method according to any one of (29) to (36), wherein the method further comprises a step of extracting DNA from the sample to be tested, and the sample to be tested is a peripheral blood sample; and preferably, the peripheral blood sample is a plasma sample.

(38) The method according to (37), wherein the DNA is gDNA or cfDNA.

(39) The method according to any one of (29) to (38), wherein the method further comprises a step of enriching the DNA or sequencing the DNA.

(40) A device for detecting, monitoring, or predicting whether a subject has a placenta-derived gestational disorder, comprising:

> a processor; and
> a memory configured to store processor-executable instructions,
> wherein the process is configured to, when executing the processor-executable instructions, implement the method according to any one of (23) to (39).

(41) A non-volatile computer readable storage medium having computer program instructions stored thereon, wherein the computer program instructions, when executed by a processor, implement the method according to any one of (23) to (39).

(42) A system for detecting at least one specific methylation modification at a specific CpG locus or at least one DNA methylation haplotype in a sample to be tested, the system comprising a detecting module configured to detect:

> (a) whether at least one methylation modification at a specific CpG locus with a characteristic of the placenta-derived gestational disorder or an abundance of the modification thereof is present in the sample to be tested; or
> (b) whether at least one DNA methylation haplotype with a characteristic of the placenta-derived gestational disorder or an abundance of the DNA methylation haplotype is present in the sample to be tested,

wherein the methylation modification at the specific CpG locus or the DNA methylation haplotype with the characteristic of the placenta-derived gestational disorder is derived from methylation difference regions of a placenta of a pregnant woman in normal pregnancy and a placenta of a pregnant woman with the placenta-derived gestational disorder.

(43) The system according to (42), wherein the methylation modification at the specific CpG locus with the characteristic of the placenta-derived gestational disorder takes place at a locus covered by a genomic region listed in Attached List 1; and the DNA methylation haplotype is a DNA methylation haplotype covered by a genomic region listed in Attached List 1.

(44) The system according to (42) or (43), wherein the specific DNA methylation modification or the specific DNA methylation haplotype detected is located in one or more regions selected from the following regions (i) to (iii):

> (i) a region of LTR12 transposon family; preferably, the LTR12 transposon is an LTR12C transposon or an LTR12E transposon;
> (ii) an FLT1 gene and a regulatory region thereof; preferably, the FLT1 gene and the regulatory region thereof are a region between human chromosome chr13: 28800000 bp and 302000000 bp; and
> (iii) an LIFR gene and a regulatory region thereof; preferably, the LIFR gene and the regulatory region thereof are a region between human chromosome chr5: 38370000 bp and 38840000 bp.

(45) A system for detecting, monitoring, and/or predicting whether a subject has a placenta-derived gestational disorder, the system comprising the following modules:

> (1) a detecting module configured to detect:

>> (a1) whether at least one methylation modification at a specific CpG locus with a characteristic of the placenta-derived gestational disorder or an abundance of the modification thereof is present in the sample to be tested, wherein the DNA methylation modification to the characteristic of the placenta-derived gestational disorder is derived from methylation difference regions of a placenta of a pregnant woman in normal pregnancy and a placenta of a pregnant woman with the placenta-derived gestational disorder; or
>> (b1) whether a DNA methylation haplotype with a characteristic of the placenta-derived gestational disorder or an abundance of the DNA methylation haplotype is present in the sample to be tested, wherein the DNA methylation haplotype is derived from methylation difference regions of a placenta of a pregnant woman in normal pregnancy and a placenta of a pregnant woman with the placenta-derived gestational disorder;

(2) a comparing module configured to compare:

(a2) a detection result of the methylation modification at the specific CpG locus in the sample to be tested or of the abundance of modification thereof with a detection result of a methylation modification at a specific CpG locus in a placental sample with or without a placenta-derived gestational disorder or of an abundance of modification thereof; or

(b2) a detection result of the DNA methylation haplotype in the sample to be tested or of the abundance of the DNA methylation haplotype with a detection result of a DNA methylation haplotype in a placental sample with or without a placenta-derived gestational disorder or of an abundance of the DNA methylation haplotype;

(3) a determining module configured to determine whether the subject has the placenta-derived gestational disorder:

(i) the subject has the placenta-derived gestational disorder if in (a2) or (b2), a similarity between the detection result of the sample to be tested and the detection result of the placental sample without the placenta-derived gestational disorder is lower or a statistic value of the similarity is significantly lower than expected, or a similarity between the detection result of the sample to be tested and the detection result of the placental sample with the placenta-derived gestational disorder is higher or a statistic value of the similarity is significantly higher than expected;

(ii) the subject does not have the placenta-derived gestational disorder if in (a2) or (b2), a similarity between the detection result of the sample to be tested and the detection result of the placental sample without the placenta-derived gestational disorder is higher or a statistic value of the similarity is significantly higher than expected, or a similarity between the detection result of the sample to be tested and the detection result of the placental sample with the placenta-derived gestational disorder is lower or a statistic value of the similarity is significantly lower than expected.

(46) The system according to (45), wherein the methylation modification at the specific CpG locus with the characteristic of the placenta-derived gestational disorder takes place at a locus covered by a genomic region listed in Attached List 1; and the DNA methylation haplotype is a DNA methylation haplotype covered by a genomic region listed in Attached List 1.

(47) The system according to (45) or (46), wherein the methylation modification at the specific CpG locus or specific DNA methylation haplotype detected is located in one or more regions selected from the following regions (i) to (iii):

(i) a region of LTR12 transposon family; preferably, the LTR12 transposon is an LTR12C transposon or an LTR12E transposon;

(ii) an FLT1 gene and a regulatory region thereof; preferably, the FLT1 gene and the regulatory region thereof are a region between human chromosome chr13: 28800000 bp and 302000000 bp; and

(iii) an LIFR gene and a regulatory region thereof; preferably, the LIFR gene and the regulatory region thereof are a region between human chromosome chr5: 38370000 bp and 38840000 bp.

(48) The method according to any one of (23) to (39), wherein the detection method comprises high-throughput sequencing.

(49) The method according to any one of (23) to (39), wherein the detection method comprises qPCR or digital PCR.

(50) The method according to any one of (23) to (39), wherein the detection method comprises methylation-specific PCR.

(51) The method according to any one of (23) to (39), wherein the detection method comprises methylation-sensitive enzyme digestion.

Effect of Invention

The invention of the present disclosure provides a method for detecting a methylation modification level of at least one DNA methylation modification difference locus or at least one DNA methylation haplotype in a sample to be tested, a kit, and a corresponding detection system, which can be used for accurately screening, *in vitro,* whether a subject has a placenta-derived gestational disorder.

**BRIEF DESCRIPTION OF THE DRAWINGS**

FIG. 1 shows a diagram for 0.8% agarose gel electrophoresis of gDNA from a placental tissue.

FIG. 2 shows the fragment size distribution of cfDNAs in plasma.

FIG. 3 shows the fragment size distribution of the DNA methylation library products for Tequila V3.

FIG. 4 shows the fragment size of a library captured by a SeqCap EpiGiant probe.

FIG. 5 shows the differentially methylated regions of the preeclamptic placentas and the placenta in normal pregnancy.

FIG. 6 shows a diagram for whole-genome distribution of preeclampsia-specific differentially methylated regions.

FIG. 7 shows enrichment of LTR12C transposons in the preeclampsia-specific hyper-methylated region.

FIG. 8 shows schematic diagrams, in which the methylation level of LTR12C in a hypo-methylated region specific to *Homo sapiens* sperms changes with a change in chromatin accessibility; the exemplary region of chromatin is chr1:212,028,092-212,163,353 (GRCh37 version).

FIG. 9 shows that the chromatin accessibility in vicinity of the preeclampsia-specific hyper-methylated region is specifically increased at the 8-cell stage.

FIG. 10 shows the *de novo* methylation defect in the preeclamptic placenta.

FIG. 11 shows the *de novo* methylation defect in the LIFR gene of the preeclamptic placenta.

FIG. 12 shows the *de novo* methylation defect in the FLT1 gene regulatory region of the preeclamptic placenta.

FIG. 13 shows that the chromatin accessibility in vicinity of a preeclampsia-specific hypo-methylated region is increased in blastocyst and placenta.

FIG. 14 shows that the expression level of the gene in vicinity of a preeclampsia-specific hypo-methylated region is significantly elevated in the extravillous trophoblast.

FIG. 15 shows that the DNA methylation modification level of the preeclampsia-specific methylated region can distinguish embryos (or tissues) at different developmental stages and different pregnancy complications.

FIG. 16 shows the preeclampsia-specific methylation haplotype.

FIG. 17 shows the design principle and verification of the EP-007 probe or probe set.

FIG. 18 shows the comparison results of the on target rates and the mean coverage in the sequencing results obtained after capture by the EP-007 probe or probe set at different combinations of hybridization and eluting temperatures.

FIG. 19 shows an influence of different hybridization time on the on target rate in the sequencing results.

FIG. 20 shows the fragment sizes of the libraries captured by EP-007 probes or probe sets.

FIG. 21 shows the results of training a model for predicting the risk of preeclampsia.

FIG. 22 shows the performance results of sensitivity and specificity of a preeclampsia risk prediction model in a test set.

FIG. 23 shows that a preeclampsia risk prediction model can distinguish different severities of subtypes of preeclampsia.

FIG. 24 shows that a preeclampsia risk prediction model can predict the highest systolic blood pressure during the first stage of labor.

## DETAILED DESCRIPTION

Definitions

When used in combination with the term "including" in the claims and/or specification, the word "a" or "an" may refer to "one", or refer to "one or more", "at least one", and "one or more than one".

As used in the claims and specification, the term "including", "having", "comprising", or "containing" is intended to be inclusive or open-ended, and does not exclude additional or unrecited elements or methods and steps.

Throughout the application document, the term "about" means that a value includes the error or standard deviation caused by the device or method used to measure the value.

It is applicable to the content disclosed herein that the term "or" is defined only as alternatives and "and/or", but the term "or" used herein refers to "and/or" unless otherwise expressly stated to be only alternatives or mutual exclusion between alternatives.

In the present disclosure, unless otherwise specified, all of the positions of human chromosomes described herein are numbered as per the GRCh37 version.

In the present disclosure, "fetus" refers to an embryo after 8 weeks of gestational age. "Gestational age" is a measure of the age of gestation, starting from a woman's last menstrual period (LMP) or a corresponding age estimated by an additional method. In obstetrics, the gestational age refers to the embryonic or fetal age + two weeks. For human beings, it is common that labor occurs within 37 to 42 weeks of gestational age, but it usually occurs at about 40 weeks of gestational age.

In the present disclosure, "early pregnancy" refers to the gestational age at or before 16 weeks.

In the present disclosure, "normal pregnancy" refers to the pregnancy during which no significant pregnancy complications occur, the pregnancy complications including, but not limited to, gestational hypertension, gestational diabetes, twin-to-twin transfusion syndrome, fetal intrauterine growth restriction, HELLP syndrome, preeclampsia, severe preeclampsia, secondary preeclampsia, atypical preeclampsia, and eclampsia.

In the present disclosure, "control pregnancy" refers to the pregnancy without preeclampsia, severe preeclampsia, secondary preeclampsia, atypical preeclampsia, or eclampsia. To wit, the "control pregnancy" used herein may include "normal pregnancy".

In the present disclosure, "placenta-derived gestational disorder" refers to a disorder developed during pregnancy due to a developmental defect or loss of function of the placental tissue. Exemplarily, the "placenta-derived gestational disorder" used herein is selected form gestational diabetes, twin-to-twin transfusion syndrome, fetal intrauterine growth restriction, gestational hypertension, eclampsia, preeclampsia, severe preeclampsia, secondary preeclampsia, atypical preeclampsia, or HELLP syndrome.

In the present disclosure, "gestational diabetes" means that a woman, who does not have diabetes before pregnancy, has symptoms of hyperglycemia during pregnancy.

In the present disclosure, "twin-to-twin transfusion syndrome" is the commonest complication occurring in monochorionic diamniotic twins. Because the monochorionic diamniotic twins share one placenta, in which the networks of a large number of blood vessels are anastomosed with one another, the blood of the two fetuses communicate directly, with one fetus being a donor twin with growth retardation, anemia, oliguria, oligohydramnios, or hypotension, while the other fetus being a recipient twin with plethora, polyuria, polyhydramnios, cardiomegaly, hypertension, or edema. The donor twin may die in the womb due to intrauterine growth distress, and the recipient twin may also die from heart failure.

In the present disclosure, "fetal growth restriction" means that the fetal weight is less than the tenth percentile or less than two standard deviations of the average weight for the same gestational age.

In the present disclosure, "gestational hypertension" refers to onset of diastolic blood pressure ≥90 mm Hg and (or) systolic blood pressure ≥140 mm Hg (measured at intervals between 6 hours and 1 week) after 20 weeks of gestational age without proteinuria, neurological symptoms, symptoms of liver or kidney damage, or symptoms of HELLP syndrome.

In the present disclosure, "preeclampsia" refers to new onset of systolic blood pressure ≥140 mm Hg and (or) diastolic blood pressure ≥90 mm Hg after 20 weeks of gestational age, proteinuria ≥300 mg/24 h, or qualitative test (+). In the present disclosure, preeclampsia may include severe preeclampsia, secondary preeclampsia, and atypical preeclampsia.

In the present disclosure, "severe preeclampsia" refers to onset of systolic blood pressure ≥160 mm Hg and (or) diastolic blood pressure ≥110 mm Hg after 20 weeks of gestational age, 24-hour proteinuria level of greater than or equal to 2.0 g and (or) qualitative test (++) or more, together with at least one of: serum creatinine >106 $\mu$mol/L or elevated than before, blood platelets <100,000/mm$^3$, or developing microangiopathic hemolytic anemia, elevated lactate dehydrogenase or liver enzymes, with headache or other neurological or visual symptoms, persistent epigastric discomfort, or pulmonary edema.

In the present disclosure, "secondary preeclampsia" refers to onset of systolic blood pressure ≥140 mm Hg and (or) diastolic blood pressure ≥90 mm Hg before pregnancy or before 20 weeks of gestational age, and onset of preeclampsia or eclampsia during pregnancy.

In the present disclosure, "atypical preeclampsia" refers to occurrence of symptoms of gestational hypertension with one of: neurological symptoms, hemolysis, elevated liver enzymes, and low counts of platelets, but without proteinuria (abbreviated from preeclampsia without symptoms of renal impairment), or with proteinuria (more than two rapid strip tests being positive) along with one of: neurological symptoms, hemolysis, elevated liver enzymes, low counts of platelets, but without hypertension (abbreviated from preeclampsia without symptoms of hypertension).

In the present disclosure, "HELLP syndrome" refers to a syndrome characterized by hemolysis, elevated liver enzymes, and low counts of platelets during pregnancy, which is a serious complication of gestational hypertension.

In the present disclosure, "transposon", also known as a mobile gene or a jumping gene, is a DNA sequence that may be inserted and cleaved in a genome and can change its own location.

"Sequence identity" and "percent identity" used herein refer to the percentage of nucleotides that are the same (i.e., identical) between two or more polynucleotides. The sequence identity between two or more polynucleotides can be determined by aligning the nucleotide sequences of polynucleotides and scoring the number of positions at which nucleotides are identical in the aligned polynucleotides, and comparing the number of these positions with the number of positions at which nucleotides are different in the aligned polynucleotides. Polynucleotides may differ at one position by, e.g., containing a different nucleotide (i.e., substitution or mutation) or deleting a nucleotide (i.e., a nucleotide insertion or a nucleotide deletion in one or two polynucleotides). The sequence identity may be calculated by dividing the number of positions at which nucleotides are identical by the total number of polynucleotides. For example, the percent identity may be calculated by dividing the number of positions at which nucleotides are identical by the total number of nucleotides in the polynucleotides, and multiplying the result by 100.

In some inventions, the present disclosure relates to the stringency of hybridization conditions used to define the degree of complementarity between two polynucleotides. Optionally, the aforesaid polynucleotides may be selected from DNA. "Stringency" used herein refers to the temperature and ionic strength conditions during hybridization and the presence or absence of some organic solvents. The higher the stringency, the higher the degree of complementarity between the target nucleotide sequence and the labeled polynucleotide sequence. "Stringent conditions" refers to the temperature and ion conditions under which only nucleotide sequences with high-frequency complementary bases hybridize. The term "hybridization under high-stringency or very high-stringency conditions" used herein describes the conditions for hybridization and washing. Guidance for hybridization reactions is described in Current Protocols in Molecular Biology, John Wiley & Sons, N.Y. (1989), 6.3.1-6.3.6.

In the present disclosure, "high-stringency hybridization conditions" means that following the standard DNA blotting procedures, a probe of at least 100 nucleotides in length pre-hybridizes or hybridizes for 12 to 24 hours at 42°C in 5X SSPE (saline sodium phosphate EDTA), 0.3% SDS, 200 $\mu$g/ml of sheared and denatured salmon sperm DNA, and 50% formamide. Finally, the vector material is washed three times at 65°C with 2X SSC and 0.2% SDS, each for 15 min.

In the present disclosure, "very high-stringency hybridization conditions" means that following the standard DNA blotting procedures, a probe of at least 100 nucleotides in length pre-hybridizes or hybridizes for 12 to 24 hours at 42°C in 5X SSPE (saline sodium phosphate EDTA), 0.3% SDS, 200 $\mu$g/ml of sheared and denatured salmon sperm DNA, and 50% formamide. Finally, the vector material is washed three times at 70°C with 2X SSC and 0.2% SDS, each for 15 min.

In the present disclosure, "whole-genome bisulfite sequencing" is also known as WGBS. The sequencing is based on the principle of treating a sample to be tested with bisulfite to convert the unmethylated C bases in the genome into U and then into T by PCR amplification, so that they are distinguished from the C bases originally with methylation modifications, and then aligned with the reference sequence by the high-throughput sequencing technology, thereby determining whether methylations occur at the CpG/CHG/CHH loci. This is particularly suitable for drawing whole-genome DNA methylation profiles at single-base resolution.

In the present disclosure, "cell-free DNA", also known as cfDNA, is the partially degraded endogenous DNA that is free from extracellular parts in human circulating blood, urine, and other body fluids. cfDNAs are mostly 200 bp or less in length and present in plasma at very low concentrations (about 1 to about 100 ng/mL).

In the present disclosure, "genomic DNA", also known as "gDNA", refers to the total DNA content of an organism in the haploid state.

In the present disclosure, "locus" corresponds to a single locus, which may be a single base position or a group of related base positions, such as a CpG locus.

In the present disclosure, "DNA methylation" refers to a process of transferring a methyl group from S-adenosyl methionine (SAM) as a methyl donor to a specific base under the catalysis of DNA methyltransferases in an organism. In mammals, methylation mainly takes place at the 5'C-terminal of nucleotide cytosine residues. On human genomes, a large amount of DNA methylations take place on cytosines in CpG dinucleotides, where C is cytosine, G is guanine, and p is a phosphate group. DNA methylation may also take place on cytosine in nucleotide sequences such as CHG and CHH, where H is adenine, cytosine, or thymine. DNA methylation may also take place at, e.g., N6-methyladenine, other than cytosines. In addition, DNA methylations may further be in the form of 5-hydroxymethylcytosine, etc. In most cases, DNA methylations are induced by methylation modifications on oppolocus DNA strands or other CpG loci near DNA bases.

In some cases, "DNA methylation" may be used as a noun, referring to a methylated DNA fragment.

In the present disclosure, "demethylation" refers to biochemical erasure of the methylation modification to the C base of the CpG locus on the original DNA fragment. During human embryonic development, the erasure process is realized mainly by specifically catalyzing the oxidization of 5-methylcytosine by a TET family oxidase to form 5-hydroxymethylcytosine, 5-formylcytosine, and 5-carbonylpyrimidine in this order, then forming uracil through the catalytic deamination reaction of APOBEC family enzymes, and finally erasing the uracil at this locus through replication and correction by DNA polymerases, and replacing it with the original cytosine.

In the present disclosure, *"de novo* methylation" refers to a process of DNA methylation occurring on a DNA fragment that does not originally have any methylation modification. In particular, this process is not the same as the DNA methylation induced by the methylation modification to the oppolocus DNA strand caused by semi-conservative replication.

In the present disclosure, "hyper-methylation" means that more than 50%, preferably more than 90%, more preferably more than 95% of the C bases at CpG loci on a DNA fragment are modified with methylation. In the present disclosure, "hyper-methylation" and "high methylation" have the same meaning and are interchangeable.

In the present disclosure, "hypo-methylation" means that less than 50%, preferably less than 10%, more preferably less than 5% of the C bases at CpG loci on a DNA fragment are modified with methylation.

In the present disclosure, "DNA methylated locus" refers to a single or multiple base position(s) at which DNA methylation modifications may occur, for example, a CpG locus, a CHG locus, or a CHH locus. In some instances, DNA methylated loci are equivalent to CpG loci.

In the present disclosure, "DNA differentially methylated locus", also known as a "DNA methylation modification difference locus", refers to a single base position where the degrees of DNA methylation modifications are different in two groups of biological samples, for example, a CpG locus, a CHG locus, or a CHH locus.

In the present disclosure, "DNA differentially methylated region", also known as a "DNA methylation difference region" or "DNA methylation modification difference region", refers to a genomic region consisting of a plurality of adjacent base positions where the degrees of DNA methylation modifications are different in two groups of biological samples. Of these, the distance between any two adjacent base positions does not exceed 20,000 nucleotides (bases). In the present disclosure, "DNA methylation modification" may be expressed as a single base methylation modification, or may be expressed as a mean of methylation modifications of multiple bases on a continuous region, or may be expressed as a methylation haplotype, or may also be expressed as a methylation monosomy.

In the present disclosure, "DNA methylation modification level" may be described by overall quantitative parameter $\beta$ or by the abundance of the methylation haplotype.

In the present disclosure, the overall quantitative parameter $\beta$ of the DNA methylation modification level may be expressed as a mean methylation modification level of C bases, namely:

$$\beta = \frac{\text{the number of C with methylation modifications}}{\text{the number of C with methylation modifications + the number of C without methylation modifications}}.$$

The above-mentioned formula is applied to either a single base or a genomic region consisting of a plurality of adjacent bases.

In the present disclosure, "methylation haplotype", also known as "methylation monosomy", refers to a combination of methylation modifications at two or more consecutive C-base loci on a single DNA fragment. Due to the relevance of the DNA methylation modifications on the same chromosome (DNA strand), the methylation modifications to a plurality of adjacent C-base loci may be correlated with one another, forming a certain fixed form. Therefore, the DNA methylation haplotype is an interlocking pattern of DNA methylation modifications on the same chromosome.

In the present disclosure, "abundance of methylation haplotype" refers to the proportion of a specific methylation haplotype obtained from statistical analysis of the methylation haplotype information. That is, abundance of methylation haplotype = the number of DNA fragments matching a specific methylation haplotype/ the total number of DNA fragments fully covering the genomic region with this methylation haplotype.

In the present disclosure, "methylated regions with a placenta-derived gestational disorder" and "differentially methylated regions (DMRs) specific to a placenta with a placenta-derived gestational disorder" share the same meaning, and can be interchangeable.

In the present disclosure, "Pearson correlation coefficient" can be used to measure the degree of correlation between two random variables by dividing the standard deviation of the two random variables by the covariance. Pearson correlation coefficient is a value between -1 and 1. If the linear relationship between two variables is strengthened, the correlation coefficient tends to be 1 or -1. If one variable increases and the other also increases, this indicates that they are positively correlated and the correlation coefficient is greater than 0. If one variable increases but the other decreases, this indicates that they are negatively correlated and the correlation coefficient is less than 0. If the correlation coefficient is equal to 0, this indicates that they do not have a linear relationship.

In the present disclosure, "similarity" can be used to measure the quantitative similarity of two samples or two types of samples. In particular, it can be expressed quantitatively as the probability that a sample is surely from a certain type of specific samples, as calculated by a statistical principle. In other words, it corresponds to the probability that a sample randomly selected from a group of samples of a specific type is equivalent to a given target sample.

In the present disclosure, "higher similarity" or "significantly higher" means that when the probability that a sample is from the specific samples of type A rather than from the specific samples of another type (e.g., rather than from the specific samples of type B) exceeds 90%, preferably exceeds 95%, more preferably exceeds 99%, the statistic value of similarity is significantly higher than expected; "lower similarity" or "significantly lower" means that when the probability that a sample is from the specific samples of type A rather than from the specific samples of another type (e.g., rather than from the specific samples of type B) is less than 10%, preferably less than 5%, more preferably less than 1%, the statistic value of similarity is significantly lower than expected.

In the present disclosure, the method for measuring the similarity between two samples may be selected from Bayesian analysis, correlation, binomial distribution, beta distribution, negative binomial distribution, hypergeometric distribution, Normal distribution, Poisson distribution, Komolgorov-Smirnov test (KS test), and Shapiro-Wilk test. The method for measuring the proportion of sample similarity (i.e., the probability that a sample is more likely from the specific samples of type A rather than the specific samples of type B) may be selected from likelihood ratio test and chi-square test. Without loss of generality, a person skilled in the art should be easily aware that such analysis may be conducted by any other appropriate mathematics statistics method based on the specific DNA methylated markers and principles and methods of statistics provided herein.

In the present disclosure, "EP-007 probe or probe set" is composed of a series of end-labeled DNA fragments of about 50 bp to about 70 bp in length, which is capable of capturing and enriching the genomic regions listed in Attached List 1 herein. For a specific region as listed in the Attached List 1, the EP-007 probe or probe set includes a combination of whole-genome methylation or demethylation modifications to all CpG loci over this specific region. Namely, for any CpG locus on any region as listed in the Attached List 1, the EP-007 probe or probe set includes not only complementary sequences when the locus is an unmethylated cytosine, but also complementary sequences when the locus is a methylated cytosine. Exemplarily, the probes in the "EP-007 probe or probe set" used herein are 50, 51, 52, 53, 54, 55, 56, 57, 58, 59, 60, 61, 62, 63, 64, 65, 66, 67, 68, 69, or 70 bp in length.

In one embodiment of the present disclosure, the DNA methylation status may be analyzed by other techniques, such as microarray, real-time PCR, digital real-time PCR, and mass spectrometry (Plongthongkum et al., Nature Reviews Genetics, 2014, 15 (10):647-61; Consortium et al., Nature Biotechnology, 2016, 34 (7):726). A person skilled in the art should be easily aware that, based on the specific DNA methylated markers provided herein, any method or reagent that can be used to detect the DNA methylation status may be used to detect, monitor, and/or predict whether a subject has a placenta-derived gestational disorder.

The bisulfite conversion-based methylation detection method has become a conventional technique for detecting the DNA methylation status, and is well known to a person skilled in the art. In one specific embodiment of the present disclosure, the EZ DNA Methylation-Goldbisulfite conversion kit from the Zymo Research Corporation is used as a reagent for detecting the DNA methylation status, to enable the bisulfite conversion of the sample DNA.

In one embodiment of the present disclosure, the inventors have discovered that it is possible to compare the methylation regions in the placental sample to be tested with those in the placenta in control pregnancy, determine a mean methylation level or an abundance of methylation haplotype in a differentially methylated region specific to the placenta with a particular placenta-derived gestational disorder in the sample to be tested by comparing the placental sample to be tested with the placental sample from a pregnancy with or without the particular placenta-derived gestational disorder, and then analyze the similarity between the placental sample to be tested and the placenta with or without the particular placenta-derived gestational disorder based on the mean methylation level or the abundance of methylation haplotype, thereby determining whether the source of the placental sample to be tested is related to the particular placenta-derived gestational disorder.

In one specific embodiment of the present disclosure, the inventors have discovered that compared with the placenta in the control pregnancy, the placenta with the placenta-derived gestational disorder has a series of differentially methylated regions (DMRs) specific to the placenta with the placenta-derived gestational disorder, namely, the methylation regions with the placenta-derived gestational disorder.

In another embodiment of the present disclosure, the inventors have discovered that by comparing the mean methylation levels in the differentially methylated regions (DMRs) specific to the placenta with the placenta-derived gestational disorder, the placenta in normal pregnancy can be distinguished from the placenta from a pregnant woman with the placenta-derived gestational disorder selected from: gestational diabetes (GDM), twin-to-twin transfusion syndrome (TTTS), fetal intrauterine growth restriction, gestational hypertension (GHT), eclampsia, preeclampsia (PE), severe preeclampsia, secondary preeclampsia, atypical preeclampsia, or HELLP syndrome, thereby determining whether the subject has the placenta-derived gestational disorder.

In one specific embodiment of the present disclosure, the placenta-derived gestational disorder is preeclampsia. Exemplarily, according to the relative methylation levels (compared, in the same region, with the regional methylation levels in the placenta in control pregnancy), the differentially methylated regions (PE-DMRs) specific to the preeclamptic placenta may be classified into preeclampsia-specific hyper-methylated regions (PE-hyper-DMRs, i.e., the genomic regions with an elevated methylation modification level in the preeclamptic placenta) and preeclampsia-specific hypo-methylated regions (PE-hypo-DMRs, i.e., the genomic regions with a decreased methylation modification level in the preeclamptic placenta). Furthermore, the inventors have discovered that by comparing the mean methylation levels in PE-DMRs, it is possible to distinguish germ cells or embryonic tissues at different developmental stages, including, but not limited to, ovum, sperm, 8-cell cleavage stage, morula, blastocyst-stage inner cell mass, and fetus at 6 weeks of gestation.

In one embodiment of the present disclosure, the inventors have further studied PE-DMRs and discovered that PE-hyper-DMRs are mostly distributed in intergenic regions and introns. Moreover, the inventors have discovered a higher proportion of PE-hyper-DMRs on a series of human endoretroviruses (ERVs) in intergenic regions or introns.

In one specific embodiment of the present disclosure, ERV enriching preeclampsia-specific hyper-methylated regions includes LTR12C, LTR12E, LTR13A, HERVE-int, LTR12D, and LTR13. In a more specific embodiment, the ERV significantly enriching preeclampsia-specific PE-hyper-DMRs is LTR12C.

In spite of not wishing to be bound by a single theory, there are PE-hyper-DMRs that are in a hypo-methylation status in *Homo sapiens* spermatoblasts, but are in a hyper-methylation status in the homologous regions in *Pan troglodytes*. That is, the PE-hyper-DMRs are enriched in the hypo-methylated regions specific to *Homo sapiens* gametes. Furthermore, the chromatin accessibility to the PE-hyper-DMRs are specifically increased at the 8-cell stage of *Homo sapiens* embryonic development, that is, the PE-hyper-DMRs are enriched in the functional regions of the genome that play important roles in the specific stage of embryonic development.

In one embodiment of the present disclosure, the inventors have discovered that in the preeclamptic placenta, a change in the methylation status of LTR12C is strongly correlated with the change in the chromatin accessibility status of its surrounding genes.

Moreover, PE-hypo-DMRs are enriched in introns of functional genes, introns of non-coding RNAs, 5'-untranslated regions, 3'-untranslated regions, and downstream regions of genes.

In one embodiment of the present disclosure, the inventors have discovered that PE-hypo-DMRs are significantly enriched in the *de novo* methylation region of the placental lineage, suggesting that the preeclamptic placenta is significantly defective in the course of *de novo* methylation. Furthermore, the chromatin accessibility to the PE-hypo-DMRs is specifically increased at the morula stage and blastocyst stage and in the placenta during the *Homo sapiens* embryonic development, and the expression of their surrounding genes is strongly increased at these stages of embryonic development, particularly in the differentiation process of extravillous trophoblasts (EVTs). That is, PE-hypo-DMRs are enriched in functional regions of the genome that play important roles in cell differentiation and placental development.

In another embodiment of the present disclosure, the inventors have discovered that in the placental tissue, there are strongly correlated CpG loci in the preeclampsia-specific methylation regions. The methylation modifications carried on DNA fragments, from which such type of genomic regions are derived, can form a characteristic combination, i.e., a preeclampsia-specific methylation haplotype. The inventors have further discovered that the methylation patterns of the preeclampsia-specific methylation haplotypes differ markedly and consistently between the preeclamptic placenta and the placenta in control pregnancy. Meanwhile, such kind of methylation haplotypes is not present in the plasma of normal non-pregnant women.

In another embodiment of the present disclosure, the inventors have discovered that the preeclampsia-specific methylation haplotype may be detected in and only in cfDNA fragments in the plasma of pregnant women afflicted with preeclampsia.

In another embodiment of the present disclosure, the inventors have discovered that the difference in the abundance of such kind of methylation haplotypes in the plasma of pregnant women is in fact an indicator for the difference in the developmental and health conditions of placentas. To wit, it is possible to determine whether a subject pregnant woman has a placenta-derived disease by comparing the abundance of methylation haplotype in the plasma of the subject pregnant woman with that in the placenta with the placenta-derived gestational disorder and in the placenta in control pregnancy.

In one specific embodiment of the present disclosure, when the abundance of the preeclampsia-specific methylation haplotype in plasma is significantly lower than the abundance in plasma of pregnant women with preeclampsia in a statistics sense and the abundance of the methylation haplotype specific to the control pregnancy is significantly higher than the abundance in plasma of pregnant women with preeclampsia in a statistics sense, the placenta may be considered to be in good developmental and health conditions. However, when the abundance of the preeclampsia-specific methylation haplotype is relatively increased, the placenta may be considered to be dysplastic, and placental-derived diseases such as preeclampsia are more likely to occur. Thus, the preeclampsia-specific methylation haplotype discovered by the inventors may be used to predict the risk of preeclampsia.

In one embodiment of the present disclosure, the inventors have discovered that by comparing the sample to be tested with the placental samples with or without the placenta-derived gestational disorder to determine the mean methylation level of the sample to be tested in the differentially methylated regions specific to the placenta with the placenta-derived gestational disorder; and then by comparing the similarity between the detection results of the sample to be tested and the placental sample without the placenta-derived gestational disorder, it is possible to determine whether the subject has the placenta-derived gestational disorder.

To be specific, if the similarity between the detection results is lower (i.e., the statistic value of similarity is significantly lower than expected), or the similarity between the detection results of the sample to be tested and the placental sample with the placenta-derived gestational disorder is higher (i.e., the statistic value of similarity is significantly higher than expected), the subject has the placenta-derived gestational disorder. If the similarity between the detection results of the sample to be tested and the placental sample without the placenta-derived gestational disorder is higher (i.e., the statistic value of similarity is significantly higher than expected), or the similarity between the detection results of the sample to be tested and the placental sample with the placenta-derived gestational disorder is lower (i.e., the statistic value of similarity is significantly lower than expected), the subject does not have the placenta-derived gestational disorder.

The higher similarity (or significantly higher) or the lower similarity (or significantly lower) is determined by the following criterion: if the probability that this sample is from a certain group of the samples is less than 10%, preferably less than 5%, more preferably less than 1%, the similarity between the detection results is considered to be lower; if the probability that this sample is from a certain group of the samples is more than 90%, preferably more than 95%, more preferably more than 99%, the similarity between the detection results is considered to be higher.

In another embodiment of the present disclosure, the inventors have discovered that by comparing the detection result of the DNA methylation haplotype in the sample to be tested or of the abundance of the DNA methylation haplotype with the detection result of a DNA methylation haplotype in a placental sample with or without the placenta-derived gestational disorder or of the abundance of the DNA methylation haplotype, it is possible to determine whether the subject has the placenta-derived gestational disorder.

To be specific, if the similarity between the detection results of the sample to be tested and the placental sample without the placenta-derived gestational disorder is lower (i.e., the statistic value of similarity is significantly lower than expected), or the similarity between the detection results of the sample to be tested and the placental sample with the placenta-derived gestational disorder is higher (i.e., the statistic value of similarity is significantly higher than expected), the subject has the placenta-derived gestational disorder. If the similarity between the detection results of the sample to be tested and the placental sample without the placenta-derived gestational disorder is higher (i.e., the statistic value of similarity is significantly higher than expected), or the similarity between the detection results of the sample to be tested and the placental sample with the placenta-derived gestational disorder is lower (i.e., the statistic value of similarity is significantly lower than expected), the subject does not have the placenta-derived gestational disorder.

The higher similarity (or significantly higher) or the lower similarity (or significantly lower) is determined by the following criterion: if the probability that this sample is from a certain group of the samples is less than 10%, preferably less than 5%, more preferably less than 1%, the similarity between the detection results is considered to be lower; if the probability that this sample is from a certain group of the samples is more than 90%, preferably more than 95%, more preferably more than 99%, the similarity between the detection results is considered to be higher.

In one embodiment of the present disclosure, the inventors have developed a prediction model for the risk of preeclampsia using the plasma from 22 pregnant women at 11 to 18 gestational weeks. Specifically, cfDNAs are extracted from the plasma of pregnant women; the cfDNA fragments treated with sulfite are captured by a specific probe; and the DNA fragments containing the preeclampsia-specific methylation regions are enriched and subjected to high-throughput sequencing. The similarity between the cfDNA sample and the preeclamptic placenta and the placenta in control pregnancy is calculated to predict the risk of preeclampsia.

In one embodiment of the present disclosure, the risk of preeclampsia can be predicted by calculating the Pearson correlation coefficient between the cfDNA sample and any type of placental samples.

In one specific embodiment of the present disclosure, the Pearson correlation coefficient can be used to describe the degree of correlation between the preeclampsia-specific methylation haplotype in cfDNA from a subject pregnant woman and the placenta in control pregnancy and the preeclamptic placenta, i.e., r(CTRL) and r(PE). Further, r(CTRL) is compared with r(PE) to evaluate the risk of the subject pregnant woman being afflicted with preeclampsia during the pregnancy. If r(CTRL) > r(PE), the risk of the subject pregnant woman being afflicted with preeclampsia during the pregnancy is low; if r(CTRL) < r(PE), the risk of the subject pregnant woman being afflicted with preeclampsia during the pregnancy is high; if r(CTRL) = r(PE), the risk of the subject pregnant woman being afflicted with preeclampsia during the pregnancy is a grayscale critical value.

Furthermore, in a retrospective clinical trial of the present disclosure, the detection and statistical method has achieved a sensitivity of 97% and specificity of 89% for the detection of pregnant women with preeclampsia among 159 peripheral blood samples from pregnant women. Besides, the correlation coefficient may also be used to predict the risk of pregnant women being afflicted with gestational hypertension, preeclampsia, severe preeclampsia, and secondary preeclampsia during the pregnancy. Moreover, the correlation coefficient shows a good linear correlation with the highest systolic blood pressure during the first stage of labor for pregnant women, and this correlation coefficient may be used to predict the highest systolic blood pressure during labor.

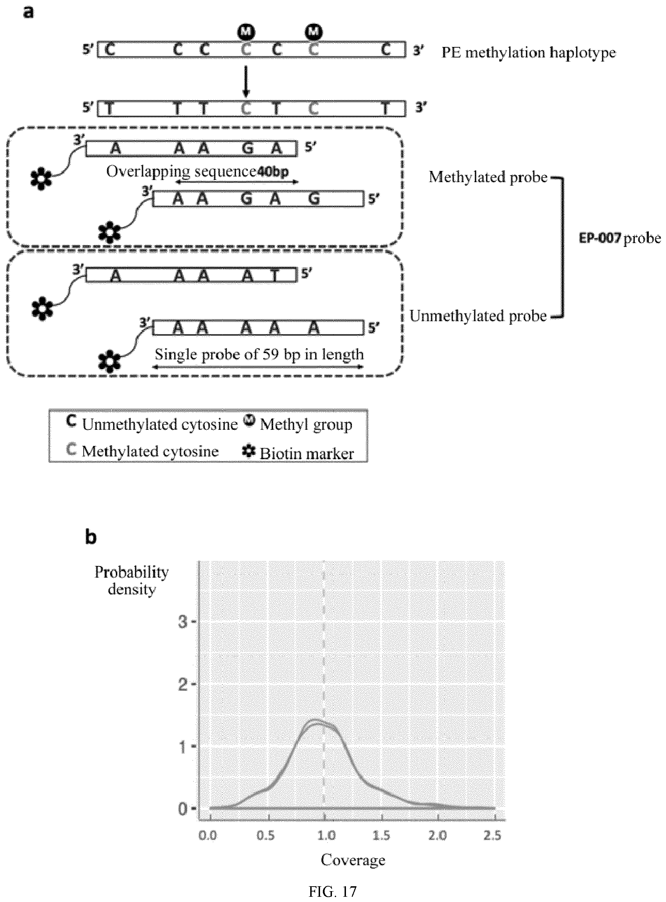

In one specific embodiment of the present disclosure, the "EP-007 probe or probe set" is composed of a series of 50- to 70-bp DNA fragments labeled with biotin at the 3'-end. It has the following four types of sequences (the design principle is as shown in FIG. 17a of the present disclosure):

> 1. the sequences inversely complementary to the sequences in which C is replaced with T as compared to the sequences of the genomic regions listed in Attached List 1;
> 2. the sequences in which C is replaced with T as compared to the sequences of the genomic regions listed in Attached List 1;
> 3. the sequences inversely complementary to the sequences in which C is replaced with T except at CpG loci as compared to the sequences of the genomic regions listed in Attached List 1;
> 4. the sequences in which C is replaced with T except at CpG loci as compared to the sequences of the genomic regions listed in Attached List 1.

In one specific embodiment of the present disclosure, the labeling may be applied at the 3'-end.

In one specific embodiment of the present disclosure, the labeling may include, but is not limited to, the labeling methods such as biotin labeling, avidin labeling, and fluorescent labeling, as long as the labeling enables the "EP-007 probe or probe set" to be detected by a detecting instrument. Exemplarily, in case of the fluorescent labeling, the detecting instrument is selected from fluorescence-detectable devices.

Since the sequences of the genomic regions listed in Attached List 1 in the present disclosure have been recited, a person skilled in the art may design a suitable EP-007 probe or probe set based on the design principle of the EP-007 probe or probe set as described above.

Examples

Other purposes, features, and advantages of the present disclosure will become apparent from the following detailed descriptions. It should be understood, however, that the detail descriptions and specific examples (while representing the specific embodiments herein) are provided for illustrative purposes only. This is because various variations and modifications made within the spirit and scope of the present disclosure will become apparent to a person skilled in the art upon reading the detailed descriptions.

All of the molecular biology methods used herein can be found in publications, e.g., Current Protocols in Molecular Biology (Wiley) and Molecular Cloning: A Laboratory Manual (Cold Spring Harbor Laboratory Press).

All of the reagents used in the present examples are commercially available, unless otherwise emphasized.

The aspects involved herein may be implemented by utilizing any one of appropriate biological materials, and the examples described herein are for illustrative purposes only. It would not be difficult for professionals in this field to realize the detection described herein using other biological materials or a combination of several of the biological materials including, but not limited to, the materials such as blood, urine, feces, saliva, oral swabs, cervical secretions, cervical smears, amniocentesis, fetal villi samples, and fetal circulating cells.

The experiments involved herein may be carried out by any one of appropriate DNA methylation modification assays, and the examples described herein are for illustrative purposes only. It would not be difficult for professionals in this field to realize the detection described herein by other methods or a combination of several of the methods including, but not limited to, the methods such as sulfite conversion, methylation modification by enzymatic conversion, Sanger sequencing, qPCR, digital PCR, time-of-flight mass spectrometry, methylation-specific PCR, methylation-specific restriction enzyme digestion, reduced representation bisulfite sequencing, whole-genome bisulfite sequencing, hybrid capture sequencing, amplicon sequencing, inverse PCR, dot blot, biochip hybridization, single-molecule sequencing, nanopore sequencing, loop-mediated isothermal amplification (LAMP), CRISPR enzyme-linked amplification reaction, and antibody cross-linking enzyme-linked amplification reaction.

The post-detection analysis involved herein may be implemented by any one of appropriate mathematical methods, and the examples described herein are for illustrative purposes only. It would not be difficult for professionals in this field to realize the analysis described herein by other methods or a combination of several of the methods including, but not limited to, t test, Z test, hypergeometric test, Fourier analysis, Wavelet analysis, Principal Component Analysis (PCA), t-Distributed Stochastic Neighbor Embedding (tSNE), Non-Negative Matrix Factorization (NMF), Support Vector Machine (SVM), k-Nearest Neighbor (KNN), k-means, Linear Model (LM), Generalized Linear Model (GLM), Gaussian Mixed Model (GMM), Neural Networks (NN), Random Forest (RF), Autoencoder, Deep Neural Networks (DNN) and variants thereof.

Example 1: Extraction of gDNA from maternal placental tissues using Qiagen kit

**Experimental Materials**

Frozen placental tissues (collected from Guangdong Maternal and Child Health Hospital, Clinical Research Approval No.: YL No. [201701044]);
Absolute ethanol (Beijing Chemical Works, 00500);
Qiagen gDNA extraction kit for animal tissues (Qiagen, 69504);
Qubit™ dsDNA HS Assay Kit (Thermo Fisher Scientific, Q32851);
λDNA HindIII digest (Takara, 3403).

**Experimental Methods**

Digestion of placental tissue: About 25 mg of placental tissues was weighed, and digestive juices (180 μL of

buffer ALT + 20 $\mu$L of protease K) were formulated. They were mixed and then incubated at 65°C for 4 h until the placental tissues were completely digested.

Cell lysis: After vortex, the digested product was added with a lysis buffer AL, absolute ethanol, and RNaseA, and incubated at room temperature for 10 min.

gDNA binding: All of the incubated samples were transferred to an adsorption column and centrifuged, and the liquid waste was discarded.

Washing and elution: The adsorption membrane was washed with buffer AW1 and buffer AW2 in sequence, and then added with an eluent to extract gDNA.

## Experimental Results

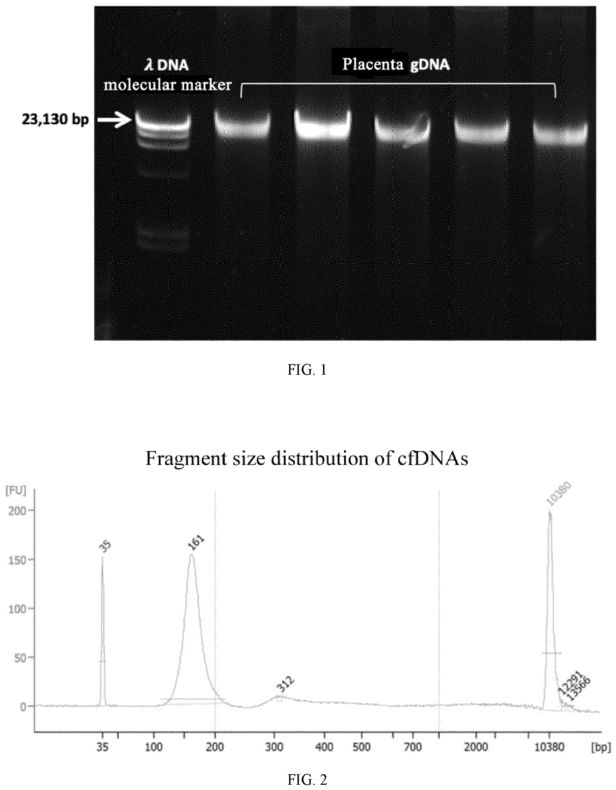

As shown in FIG. 1, the first lane from the left in FIG. 1 is the DNA molecular marker, i.e., the λDNA product (Takara) enzyme digested by Hind III. The size of the top band of the DNA molecular marker is 23,130 bp. The second to sixth lanes from the left are gDNAs extracted from different placental tissues. No obvious diffusion was observed from distribution of electrophoretic bands as shown in FIG. 1, indicating that its integrity was good.

Based on the invention described in Example 1, 8640 ng of gDNAs was extracted from about 25 mg of placental tissues. The average concentration of gDNAs was determined to be 86.4 ng/$\mu$L by Qubit™ dsDNA HS Assay Kit. The gDNA bands in the figure were concentrated at about 20 kb, indicating that the fragment size was about 20 kb.

## Example 2: Extraction of gDNA from maternal plasma using MagMAX cfDNA extraction kit

### Experimental Materials

Peripheral blood stored in blood collection tubes such as Apostle MiniMax™ cfDNA non-invasive blood collection tubes (Apostle, Inc. Silicon Valley, California.) and Cell-Free DNA BCT CE blood collection tubes (Streck, Inc., La Vista, NE) (collected from Guangdong Maternal and Child Health Hospital, Clinical Research Approval No.: YL No. [201701044]);

Protease K (Qiagen, 19133);
SDS (Sigma, 74255-250G);
Dynabeads™MyOne™ Silane (Thermo Fisher Scientific, 37002D);
MagMAX™ Cell Free DNA Lysis/Binding Solution (Thermo Fisher Scientific, A33600);
MagMAX™ Cell Free DNA Wash Solution (Thermo Fisher Scientific, A33601);
Absolute ethanol (Beijing Chemical Works, 00500);
Qubit™ dsDNA HS Assay Kit (Thermo Fisher Scientific, Q32851);
EB buffer (Qiagen, 19086);
DNase/RNase-free water (Solarbio, R1600).

### Experimental Methods

Sample preparation: The cfDNA blood collection tubes containing peripheral blood were centrifuged in a centrifuge to stratify the peripheral blood and separate the plasma layer.

Plasma lysis: A lysis reaction solution (containing 0.4 mg/ml of protease K, plasma, and 1% SDS) was prepared, vortexed, centrifuged, and incubated at 60°C for 20 min. After incubation, the lysis reaction solution was cooled at room temperature.

Binding of cfDNA to beads: A binding premix solution was prepared at a ratio of MyOne beads: binding solution = 3:250, and mixed thoroughly with the plasma lysate by vortex shaking.

Washing of beads: After adsorption by placing the reaction tube on a magnetic stand, the beads were washed twice with a wash solution and then washed twice with 80% absolute ethanol. After washing of the beads, the absolute ethanol was removed. The reaction tube was left at the magnetic stand, opened, left to stand still, and dried.

Elution of cfDNA: After dried, the beads were eluted with an EB buffer and transferred into a new centrifuge tube for storage.

### Experimental Results

From 2 mL of plasma, a total of 10 ng of cfDNAs was extracted at a concentration of 0.5 ng/$\mu$L (measured by the Qubit™ dsDNA HS Assay Kit). The fragment size distribution of cfDNAs in the samples was determined by an Agilent 2100 Bioanalyzer.

FIG. 2 shows the fragment size distribution of cfDNAs as determined by an Agilent 2100 Bioanalyzer. The peaks at 35 bp and 10,380 bp are DNA molecular markers. The main peak of cfDNA is at approximate 161 bp.

As shown in FIG. 2, the samples from which cfDNAs were extracted were free of gDNA contamination, and the main peak of their fragment size distribution is at 161 bp, which is basically consistent with the theoretical value.

Example 3: Construction of gDNA or cfDNA library based on DNA methylation library construction method for Tequila V3

**Experimental Materials**

7.5-10 ng of cfDNA or 200 ng of gDNA;
EZ DNA Methylation-Gold™ Kit (ZymoReserch, D5006);
Tequila V3 DNA Methylation Library Kit (Euler Genomics, EU-TQ-Methy);
Agencourt AMPure XP Beads (Beckman, A63882);
EB buffer (Qiagen, 19086);
DEPC water (Solarbio, R1600-500);
Absolute ethanol (Beijing Chemical Works, 00500).

**Experimental Methods**

Bisulfite conversion: According to the instruction manual for EZ DNA Methylation-Gold™ Kit, a CT conversion reagent was prepared, and the initial amount of cfDNA or gDNA as described above was subjected to CT conversion. The above cfDNA or gDNA was obtained by the method described in Example 2.

End repair: Tequila V3 DNA Methylation Library Kit was used to prepare an end-repair reaction mix 1 in accordance with Table 1. The corresponding PCR program was as follows: 37°C 30 min, 95°C 10 min, heat dome 105°C, volume 20 $\mu$L.

Table 1 Addition Volumes of Reagents in Reaction Mix for Repair

| Reaction System | Volume of Single Sample $\mu$L |
|---|---|
| cfDNA or gDNA | 17 |
| TQRB1 buffer | 2 |
| TQRE1 enzyme | 1 |
| Total volume | 20 |

Ligation reaction 1: Tequila V3 DNA Methylation Library Kit was used to prepare a reaction mix 2 on ice in accordance with Table 2. The corresponding PCR program was as follows: 37°C 30 min, 95°C 5 min, heat insulation at 10°C, heat dome 105°C, volume 30 $\mu$L.

Table 2 Addition Volumes of Reagents for Ligation Reaction 1

| Reaction System | Volume of Single Sample $\mu$L |
|---|---|
| Previous reaction | 20 |
| NF-H$_2$O | 1.5 |
| TQLB1 buffer | 4 |
| TQLR1 reagent | 2 |
| TQLR2 reagent | 1.5 |
| TQLE1 enzyme | 1 |
| Total volume | 30 |

Linear amplification: Tequila V3 DNA Methylation Library Kit was used to prepare a reaction mix 3 in accordance with Table 3. The corresponding PCR program was as follows: 95°C, 3 min, 12X[95°C 30 s; 60°C 30 s; 68°C 1 min], 68°C 5 min, thermal insulation at 16°C, heat dome 105°C, volume 100 μL.

Table 3 Addition Volumes in Reaction Mix for Linear Amplification Reaction

| Reaction System | Volume of Single Sample μL |
| --- | --- |
| Previous reaction | 30 |
| DEPC water | 45 |
| TQAB1 | 20 |
| TQAR1 | 2 |
| TQAR2 | 2 |
| TQAE1 | 1 |
| Total volume | 100 |

Bead-based purification 1: Tequila V3 DNA Methylation Library Kit, 138 μL of TQSB reagent, and 27 μL of XP beads were mixed well, then added with a PCR product obtained by linear amplification, incubated at room temperature, and placed on a magnetic stand for adsorption until the liquid was clear. Afterwards, the supernatant was removed, and a mixture of 180 μL of TQSB reagent and 100 μL of DEPC water to re-suspend the beads. After incubation at room temperature, absorption was performed over the magnetic stand until the liquid was clear. Afterwards, the supernatant was removed, and the beads were washed with 80% ethanol, followed by adding an eluent.

DNA denaturation: The PCR program was as follows: 95°C 10 min, heat dome 105°C, volume 10 μL. After the program operated for 5 min, the 0.2-mL PCR tube containing the sample was removed immediately from the PCR amplifier, inserted immediately on ice, and left for 2 min.

Ligation reaction 2: Tequila V3 DNA Methylation Library Kit was used to prepare a reaction mix 4 on ice in accordance with Table 4. The PCR program was as follows: 25°C 15 min, heat dome 35°C, volume 20 μL.

Table 4 Addition Volumes of Reagents for Ligation Reaction 2

| Reaction System | Volume of Single Sample μL |
| --- | --- |
| Product of previous step | 6.6 |
| TQTHB 1 buffer | 10 |
| TQTHR1 reagent | 2.4 |
| TQHE1 enzyme | 1 |
| Total volume | 20 |

Bead-based purification 2: To the reaction product of the previous step, 17 μL of XP beads was added, mixed thoroughly with vortex, left to stand still at room temperature, and incubated for 5 min. After incubation, the mixture was adsorbed over a magnetic stand until the liquid was clear, and the supernatant was discarded. After washed twice with 80% ethanol, the beads were dried at room temperature and eluted with DEPC water.

PCR amplification: The PCR amplification system was as listed in Table 5. The corresponding reaction program was as follows: 98°C 45 s, 12X[98°C 15 s, 60°C 30 s, 72°C 30 s], 72°C 5 min, thermal insulation at 10°C, heat dome 105°C, volume 100 μL.

Table 5 PCR Amplification System

| Reaction System | Volume of Single Sample μL |
| --- | --- |
| Eluent in previous reaction | 73 |
| TQAB1 reagent | 20 |
| TQAR1 reagent | 2 |
| TQIE2 enzyme | 1 |
| Index T5XX | 2 (added separately) |

(continued)

| Reaction System | Volume of Single Sample μL |
|---|---|
| Index Q7XX | 2 (added separately) |
| Total volume | 100 |

Bead-based purification 3: To the PCR product of the previous step, 17 μL of XP beads was added, mixed thoroughly with vortex, left to stand still at room temperature, and incubated for 5 min, and then placed on a magnetic stand for adsorption until the liquid was clear. After the liquid waste was aspirated and discarded, the beads were washed twice with 80% ethanol. After the liquid waste was aspirated and discarded, the beads were dried at room temperature and then eluted with DPEC water to obtain a pre-library.

**Experimental Results**

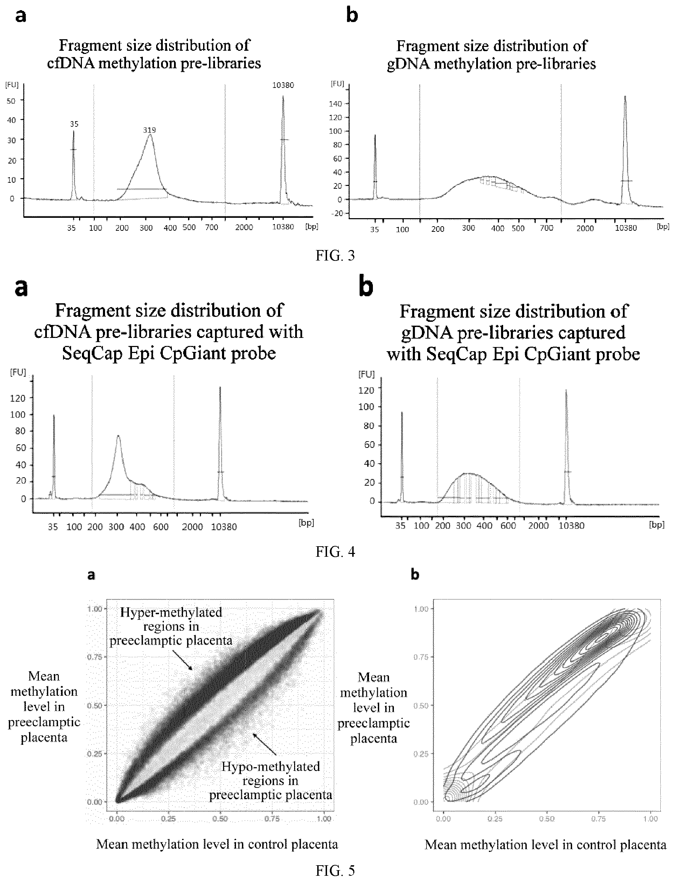

The experimental results were as shown in FIG. 3, showing the fragment size distribution of cfDNAs as determined by the Agilent 2100 Bioanalyzer. The peaks at 35 bp and 10,380 bp are DNA molecular markers.

From an initial amount 10 ng of cfDNA, 61.8 ng/μL of pre-library (determined by Qubit™ dsDNA HS Assay Kit) was obtained by this experimental method. The main peak of the fragment size distribution of the pre-library was at about 320 bp. That is, the fragment size of the cfDNA pre-library was about 320 bp (see FIG. 3a).

From an initial amount 200 ng of placenta gDNA, 70.8 ng/μL of pre-library could be obtained (determined by Qubit™ dsDNA HS Assay Kit). The main peak of its fragment size distribution was at about 300 bp. That is, the fragment size of the gDNA pre-library was about 300 bp (see FIG. 3b).

Example 4: Enrichment of whole-genome gDNA/cfDNA methylated loci using SeqCap EpiGiant probe

**Experimental Materials**

500 ng of pre-library product for methylation library construction;
Hybrid Elution Kit of Non-Invasive Preeclampsia Early Screening Kit (Euler Genomics, EU-TQ-MV1);
SeqCap EpiGiant Enrichment Probe (Roche, 07138911001);
Agencourt AMPure XP Beads (Beckman, A63882);
Dynabeads™ M-270 Streptavidin (Thermo Fisher ScientificScientific, 65306).

**Experimental Methods**

Evaporation: In accordance with Table 6, an evaporation system was prepared in a 1.5-mL EP tube using a hybrid elution kit of non-invasive preeclampsia early screening kit, and the EP tube was placed in a vacuum concentrator and evaporated at 60°C with the cap open. The library samples were constructed by the method described in Example 3.

Table 6 Evaporation System

| Reaction System | Volume of Single Component μL |
|---|---|
| Library sample | 500 ng |
| TQBLKC reagent | 2 |
| TQBLKD reagent | 4 |
| TQBLKCHE reagent | 2.5 |

Hybridization reaction: In accordance with Table 7, a hybridization system was prepared in a 200-μL PCR tube using a hybrid elution kit of non-invasive preeclampsia early screening kit, left to stand for 10 min after mixing well with vortex, and thereafter denatured in a PCR amplifier at 95°C for 10 min. At 8 min of denaturation, the PCR tube containing 4.5 μL of probe was placed in the PCR amplifier at 95°C to preheat the probe. After denaturation, the probe was charged into the hybridization system mixture at 47°C. After mixing with vortex, the PCR tube was placed in the PCR amplifier at 47°C (heat dome temperature: 57°C) and hybridized for 72 h.

Table 7 Hybridization System

| Reaction System | Volume of Single Sample μL |
|---|---|
| Evaporated sample from previous step | / |
| TQHS1 | 7 |
| TQHS2 | 3.5 |

Capture and washing: 100 μL of streptavidin-modified beads (Dynabeads™ M-270 streptavidin) equilibrated at room temperature and 200 μL of bead washing buffer (1X TQWS5 reagent) were measured and mixed thoroughly with the reaction product from previous step, and then placed on a magnetic stand for absorption until the liquid was clear. The liquid was aspirated and discarded. After washed once with 200 μL of bead washing buffer, the beads were re-suspended with an equal volume of bead washing buffer, and placed on a magnetic stand for absorption until the liquid was clear. After the liquid waste was aspirated and discarded, the beads were incubated in the PCR amplifier for 45 min.

Elution: To the product from the previous step, 180 μL of 1X TQWS4 preheated at 65°C was added using a hybrid elution kit of non-invasive preeclampsia early screening kit, and subjected to vibration incubation at 65°C for 5 min before absorbing over a magnetic stand until the liquid was clear. This step was repeated once. The liquid waste was aspirated and discarded and 180 μL of 1X TQWS1 was added and mixed well by vibration at room temperature for 2 min before absorbing over a magnetic stand until the liquid was clear. The liquid waste was aspirated and discarded and 180 μL of 1X TQWS2 was added and mixed well by vibration at room temperature for 1 min before absorbing over a magnetic stand until the liquid was clear. The liquid waste was aspirated and discarded and 180 μL of 1X TQWS3 was added and mixed well by vibration at room temperature for 1 min before absorbing over a magnetic stand until the liquid was clear. The liquid waste was aspirated and discarded and 23 μL of TQNF reagent was added and mixed well by vibration.

Amplification of capture product: In accordance with Table 8, an amplification system of the capture product was prepared using a hybrid elution kit of non-invasive preeclampsia early screening kit. The corresponding PCR program was as follows: 98°C 45s, 8X[98°C 15 s; 60°C 30 s; 72°C 30 s] 72°C 2 min, thermal insulation at 16°C, heat dome 105°C, volume 50 μL.

Table 8 Amplification System of Capture product

| Reaction System | Volume of Single Sample μL |
|---|---|
| TQAE2 enzyme | 25 |
| TQPR3 reagent | 2 |
| Capture product (containing beads) | 23 |
| Total volume | 50 |

Purification after amplification: The hybrid elution kit of non-invasive preeclampsia early screening kit was used, and the above reaction product was placed on a magnetic stand for absorption until the liquid was clear and afterwards, the supernatant was was drawn and mixed with 90 μL of XP beads by vortex. After left to stand at room temperature for 5 min, the mixture was absorbed over a magnetic stand until the liquid was clear. The liquid waste was aspirated and discarded. The beads were washed twice with 80% ethanol, and air dried at room temperature with the cap open. After drying, the beads were added with 25 μL of TQEB reagent, and incubated at room temperature for 5 min before absorbing over a magnetic stand until the liquid was clear. The clear liquid was drawn into a new EP tube, thereby obtaining a purified capture product.

**Experimental Results**

From 500 ng of pre-library for methylation library construction, 4.46 ng/μL of library could be enriched after capture by the SeqCap EpiGiant probe, as determined by the Qubit™ dsDNA HS Assay Kit.

FIG. 4 shows the fragment size of the libraries captured by the SeqCap EpiGiant probe, as determined by Agilent 2100 Bioanalyzer. The size of the above fragment is about 300 bp. Specifically, FIG. 4a shows the library constructed based on placenta gDNAs, which has a fragment size of about 300 bp. FIG. 4b shows the library constructed based on cfDNAs in the plasma, which has a fragment size of about 300 bp.

Example 5: Quality test on data produced by Illumina second-generation high-throughput sequencing platform for methylation libraries

**Experimental Methods and Results**

The DNA pre-library was sequenced in accordance with the instructions of the Illumina platform kit. In the raw reads, the proportion of Q30 was greater than 80%. Samples were demultiplexed using bcl2fastq (0.2.1). According to the quality control criterion, the unknown demultiplexing fragment count was <5%, and the average fragment length was >160 bp. The Euler-PhiX flow was used for quality control of the PhiX library, and the criterion of acceptability was error rate <0.001%. Fastqc (3.2) was used for quality control of sublibraries. The quality control criteria for libraries were as follows: Q30 >80%, repeating fragments <=10%, and GC ratio = 30%-35%. As for the cfDNA libraries, the KS test on their fragment length distribution and that of the standard cfDNAs shows that P>0.5, and the KS test on their fragment end base distribution and that of the standard cfDNAs show that P>0.5. CHG>THG and CHH>THH were sampled and found that their conversion rates were ≥99%.

Example 6: Preeclamptic placenta-specific differentially methylated regions

**Experimental Materials**

Placentas in control pregnancy (collected from Peking University Third Hospital, Clinical Research Ethics Approval Document No.: IRB00001052-16009);
Preeclamptic placentas in pregnant women (collected from Peking University Third Hospital, Clinical Research Ethics Approval Document No.: IRB00001052-16009);

DNA methylation database *of Homo sapiens* spermatoblasts (CRA000114);
DNA methylation database *of Pan troglodytes* spermatoblasts (GSE30340);
ATAC-seq database *of Homo sapiens* embryo at 2-cell stage (SRP163205, SRP112718);
ATAC-seq database *of Homo sapiens* embryo at 4-cell stage (SRP163205, SRP112718);
ATAC-seq database *of Homo sapiens* embryo at 8-cell stage (SRP163205, SRP112718);
ATAC-seq database of inner cell mass of *Homo sapiens* blastocyst-stage embryo (SRP163205, SRP112718);
ATAC-seq database of trophoblasts of *Homo sapiens* blastocyst-stage embryo (SRP163205, SRP112718);
ATAC-seq database *of Homo sapiens* embryonic stem cells (SRP112718);
HBSS (Thermo Fisher Scientific, 14025076);
Protease inhibitor (Roche, 5056489001);
0.4% Trypan Blue (Solarbio, C0040);
60% Iodixanol (Sigma, D1556);
TruePrep DNA Library Prep Kit (Vazyme, TD501-02);
TruePrep Index Kit V2 for Illumina (Vazyme, TD202);
Oligo Clean & Concentrator (Zymo Research, D4061);
Agencourt AMPure XP beads (Beckman, A63882);
RNase-free water (Solarbio, R1600-500);
70um Cell strainer (BD Falcon, 352350).

**Experimental Methods**

**Analysis of DNA methylation level**

The procedures for extraction of placenta gDNA, capture by SeqCap EpiGiant probes, and high-throughput sequencing were the same as those described in Example 1, Example 4, and Example 5.
Analysis of the methylation level at CpG loci: The coverage and methylation level at each CpG locus were extracted using PileOMeth (version 0.1.13). Conditions for quality control: The coverage at a single CpG locus was required to be ≥200X. Each of the methylated loci was analyzed using the R language (3.5.2) to screen out the preeclampsia-specific methylation modification loci. Each of the CpG methylation modification loci was subjected to statistical detection by the t test, and required to meet the following conditions: the distribution of its methylation modifications in preeclamptic placental samples was significantly different from that of methylation modifications in control placental samples, i.e., p < 0.001; at the same time, the distribution of its methylation modifications in preeclamptic placental samples was significantly different from that of methylation modifications in plasma samples from healthy non-pregnant women, i.e., p < 0.001. The methylation levels at the CpG loci, obtained from the above analysis, were significantly and consistently different among different groups of samples, i.e., groups of preeclamptic placentas, control gestational placentas, and plasma from non-pregnant women. The CpG loci obtained from the above analysis were methylation modification loci specific to preeclamptic placentas (for the details, see Attached List 1).

The regions where the methylation modification loci specific to preeclamptic placentas were located were combined, and the methylation levels were averaged across the whole region. In control placentas and preeclamptic placentas, each of the regions was analyzed using the R language (3.5.2), and the mean methylation level of each of the regions was detected by the t test.

**Experimental process for chromatin accessibility sequencing analysis (ATAC-seq)**

Tissue homogenization: 20 mg of frozen tissues were weighed and put in a pre-cooled glass homogenizer, to which 1 ml of pre-cooled 1x HB buffer (as listed in Table 10, in which the 1x HB buffer was further prepared based on the 1.022xHB buffer listed in Table 9) was added, followed by grinding the tissues. The ground tissue homogenates were filtered through a 70-$\mu$m cell strainer and transferred into a low-adsorption tube, and centrifuged at 4°C for 5 min. The supernatant was discarded. The tissue homogenates were re-suspended by adding a fresh HB buffer.

Table 9 Preparation of 1.022xHB Buffer

| 1.022 x HB Buffer (50 ml System) | | |
|---|---|---|
| Reagent | Final Concentration | Volume Added ($\mu$l) |
| 1M sucrose | 0.25M | 12775 |
| 1M KCl | 0.06M | 3066 |
| 5M NaCl | 0.015M | 153.3 |
| 1M MgCl2 | 0.005M | 255.5 |
| 1M Tris-HCl pH 7.5 | 0.01M | 511 |
| NF-H2O | ---- | 33239.2 |
| Total volume ($\mu$l) | | 50000 |

Table 10 Preparation of 1xHB Buffer

| 1x HB Buffer (50 ml System) | | |
|---|---|---|
| Reagent | Final Concentration | Volume Added ($\mu$l) |
| 1.022x EL HB buffer | 1x | 48923.7 |
| 1M DTT | 0.001 | 50 |
| 100 mM spermidine | 0.5 | 250 |
| 20% NP-40 | 0.3 | 750 |
| Protease inhibitor | ---- | 1 piece |
| NF-H2O | ---- | 33239.2 |
| Total volume ($\mu$l) | | 50000 |

Iodixanol gradient centrifugation: An equal volume of 50% iodixanol was added to the re-suspension to prepare 30% and 40% iodixanol, and construct a density gradient centrifugation system for nuclei enriched on 25%/30%/40% iodixanol, which was centrifuged at 3000 rcf at 4°C for 20 min. After centrifugation, the nuclear layer at the interface of 30% and 40% iodixanol was aspirated. The RSB buffer (Table 11) was added to dilute the nuclei.

Table 11 Preparation of RSB Buffer

| 1x RSB Buffer (50 ml System) | | |
|---|---|---|
| Reagent | Final Concentration | Volume Added (μl) |
| 1M Tris-HCl pH 7.5 | 0.01M | 500 |
| 5M NaCl | 0.01M | 100 |
| 1M MgCl2 | 0.003M | 150 |
| Protease inhibitor | ---- | 1 piece |
| NF-H2O | ---- | 49250 |
| Total volume (μl) | | 50000 |

Tn5 transposase digestion-based library construction and purification: After centrifugation at 500 rcf at 4°C for 10 min, the Tn5 transposase digestion was carried out according to the instructions of the VazymeTD501-02 TruePrep-DNALibraryPrepKit. The enzyme-digested product was purified according to the Zymo D4061 Oligo Clean & Concentrator kit. The purified product was subjected to Index PCR according to TruePrep Index Kit V2 for Illumina. The PCR product was subjected to library purification with XP beads.

**ATAC-seq data analysis**

The coverage was extracted with deeptools (version 3.3.0). R Software (version 3.5.2) was used to analyze the coverage values of transcription factors and TSS region. The peak of the accessible chromatin enrichment region was analyzed using macs2 Software (version 2.2.4) and Genrich Software (version 0.6). For the peaks of the accessible chromatin enrichment regions extracted from the eclamptic placenta and the control placenta, diffBind and NarrowPeaks (R language package from Bioconductor) were used to analyze their differential regions and perform enrichment analysis (statistically analyzing the data using the R language).

**Experimental Results**

**Screening of preeclampsia-specific DNA methylated regions**

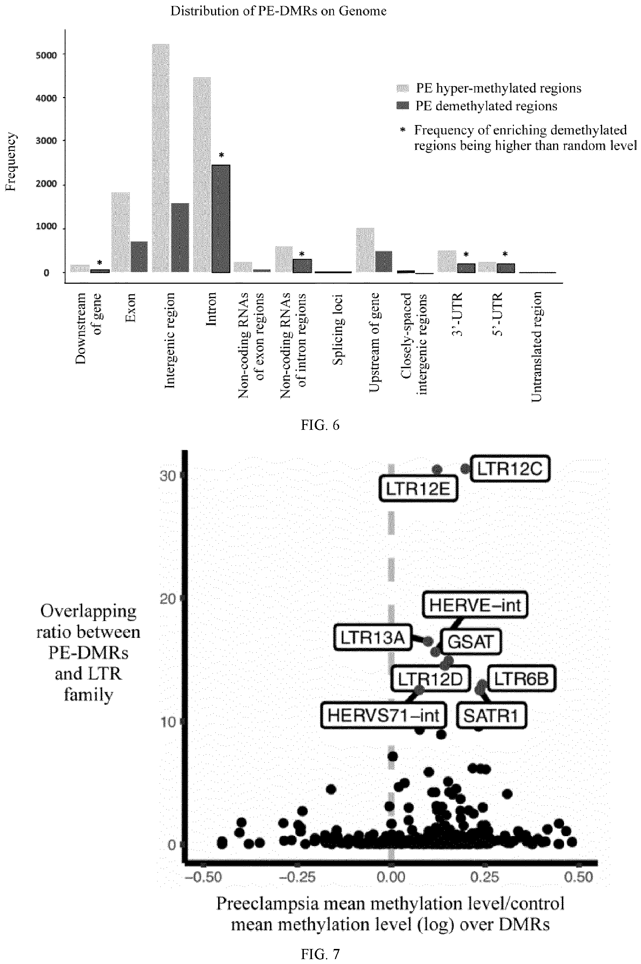

As shown in FIG. 5a, the x-axis represents the mean methylation level of the placenta-specific differentially methylated regions in a placenta in normal pregnancy, and the y-axis represents the mean methylation levels of the same region in preeclamptic placentas. The light-gray dots were concentrated on the straight line y=x, indicating that the mean methylation levels of these methylated regions were consistent in the preeclamptic placentas and in the placentas in normal pregnancy, i.e., non-differential regions. The distribution of black dots deviated obviously from the straight line y=x, indicating that there were differentially methylated regions with significantly statistical differences in the preeclamptic placentas and in the placentas in normal pregnancy.
The density distribution curves in FIG. 5b depict the differentially methylated regions of FIG. 5a. Therefore, FIG. 5b shows the density distribution curves of the non-differential regions (light gray) and the methylated regions with significantly statistical differences (dark gray) as shown in FIG. 5a.
In other words, as shown in FIG. 5, the comparison between the whole-genome methylation levels in the preeclamptic placentas and in the placentas in control pregnancy revealed that there were a large number of preeclampsia-specific differentially methylated regions (PE-DMRs), which could be classified into, according to the difference in methylation levels of PE-DMRs, preeclampsia-specific hyper-methylated regions (PE-hyper-DMRs) and preeclampsia-specific hypo-methylated regions (PE-hypo-DMRs) (for the details, see Attached List 2).
As shown in FIG. 6, PE-DMRs, when aligned to the whole genome, were found to be concentrated in the gene regulatory regions, such as non-coding transcription regions (5'UTR/3'UTR), intergenic regions, introns, and some of exon regions. A further statistical analysis of enrichment showed that PE-hypo-DMRs were significantly enriched in introns, 5'UTR/3'UTR, and intron regions of non-coding RNAs; while PE-hyper-DMRs were mostly distributed in intergenic regions and intron regions, their distribution patterns were random.
As shown in FIG. 7, the alignment of the PE-hyper-DMRs with the transposable elements (TEs) in human genome revealed that the PE-hyper-DMRs were significantly enriched in LTR12 family transposons. In particular, about 30% of LTR12C transposons contained PE-hyper-DMRs.

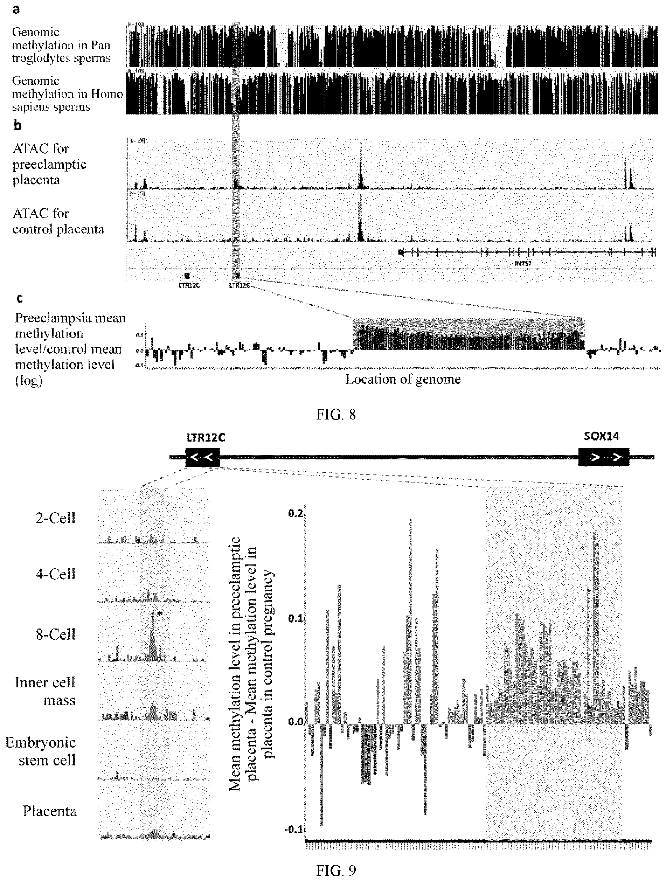

FIG. 8a shows the DNA methylation patterns in *Pan troglodytes* and *Homo sapiens* spermatoblasts. FIG. 8b shows the comparison of chromatin accessibility to the placenta in control pregnancy and the preeclamptic placenta.

FIG. 8c shows the methylation patterns of the region in the placenta in control pregnancy and the preeclamptic placenta.

To wit, by comparing the DNA methylation modification levels of the LTR12C regions with the preeclampsia-specific hyper-methylation characteristics or the homologous regions thereof in the preeclamptic placentas, the placentas in control pregnancy, the *Homo sapiens* spermatoblasts, and the *Pan troglodytes* spermatoblasts, it was found that there was LTR12C in the preeclamptic hyper-methylated region, and this region was in a hyper-methylation status in *Pan troglodytes* spermatoblasts while it was in a hypomethylation status in *Homo sapiens* spermatoblasts (FIG. 8a). That is, the hypo-methylated LTR12C specific to *Homo sapiens* spermatoblasts was in a hyper-methylation status in preeclamptic placentas (FIG. 8c). The DNA methylation status of the hypo-methylated LTR12C specific to *Homo sapiens* spermatoblasts was altered in preeclamptic placentas, accompanied by the change in chromatin accessibility (FIG. 8b).

As shown in FIG. 9, the analysis of the whole-genome chromatin accessibility to different developmental stages (2-cell stage, 4-cell stage, 8-cell stage, inner cell mass at the blastocyst stage, embryonic stem cell, and placenta) of an embryo showed that the chromatin accessibility to the genes in vicinity of the preeclamptic hyper-methylated region was specifically increased at 8-cell stage.

Example 7: Screening of *de novo* methylation regions specific to placental lineage

**Experimental Methods and Results**

Analysis of methylation levels at CpG loci: The coverage and methylation level were extracted from each of CpG loci using PileOMeth (version 0.1.13). Conditions for quality control: the coverage at a single CpG locus was required to be ≥200X. Each of the methylated loci was analyzed using the R language (3.5.2) to screen out the preeclampsia-specific hyper-methylation modification locus, which was in a hypomethylation status in leukocyte of healthy non-pregnant women. Each of the CpG methylation modification loci was subjected to statistical detection by the t test, and required to meet the following conditions: the distribution of its methylation modifications in the placental sample in control pregnancy was significantly different from that of methylation modifications in the sample from leukocyte of healthy non-pregnant women, i.e., $p < 0.001$.

Based on the differentially modified methylation locus screened by the above analysis, the regions where it was located were combined, and the methylation levels were averaged across the whole region, thereby screening out a placenta-specific hyper-methylated region. Similarly, by comparing the loci with differentially methylated modifications in DNAs from HUVEC (a differentiated human-derived placental cell line) and hESC1 (an undifferentiated human-derived embryonic stem cell line), it was possible to screen out the locus that was hyper-methylated in HUVEC and hypo-methylated in hESC1 could be screened out. The differentially methylated loci were statistically detected by the t test, which had to satisfy $p < 0.001$. The regions where differentially modified methylation loci screened were located were combined, and the methylation levels were averaged across the whole region, thereby screening out a HUVEC-specific hyper-methylated region. The intersection of the placenta-specific hyper-methylated region and HUVEC-specific hyper-methylated region was the *de novo* methylation region specific to placental lineage.

Example 8: Enrichment of preeclampsia-specific hypo-methylated regions in *de novo* methylation region of placental lineage

**Experimental Materials**

Placentas in control pregnancy (collected from Peking University Third Hospital, Clinical Research Ethics Approval Document No.: IRB00001052-16009);

Preeclamptic placentas in pregnant women (collected from Peking University Third Hospital, Clinical Research Ethics Approval Document No.: IRB00001052-16009);

ATAC-seq database *of Homo sapiens* embryo at 2-cell stage (SRP163205, SRP112718);

ATAC-seq database *of Homo sapiens* embryo at 4-cell stage (SRP163205, SRP112718);

ATAC-seq database *of Homo sapiens* embryo at 8-cell stage (SRP163205, SRP112718);

ATAC-seq database of inner cell mass of *Homo sapiens* blastocyst-stage embryo (SRP163205, SRP112718);

ATAC-seq database *of Homo sapiens* embryonic stem cells (SRP112718);

ATAC-seq database of trophoblasts of *Homo sapiens* blastocyst-stage embryo (SRP163205, SRP112718);

Single cell RNA sequencing database *of Homo sapiens* placental tissues (E-MTAB-6701, E-MTAB-6678, E-MTAB-7304).

**Experimental Methods and Results**

Placental *de novo* methylation regions and PE-DMRs were intersected to sort out the preeclampsia-specific differentially methylated regions that executed the placental *de novo* methylation, i.e., ExE-DN PE-DMRs, and the preeclampsia-specific differentially methylated regions that did not execute the placental *de novo* methylation, i.e., Not-ExE-DN PE-DMRs.

FIG. 9 shows the mean regional methylation levels of the preeclampsia-specific differentially methylated regions that executed the placental *de novo* methylation, ExE-DN PE-DMRs (dark gray) and the preeclampsia-specific differentially methylated regions that did not execute the placental *de novo* methylation, Not-ExE-DN PE-DMRs (light gray). The y-axis represents the log value of the ratio of the mean regional methylation level in the preeclamptic placenta to that in the placenta in normal pregnancy. That is, y=0 represents that in a certain region, there is no difference between the mean methylation levels in the preeclamptic placenta and in the placenta in normal pregnancy.

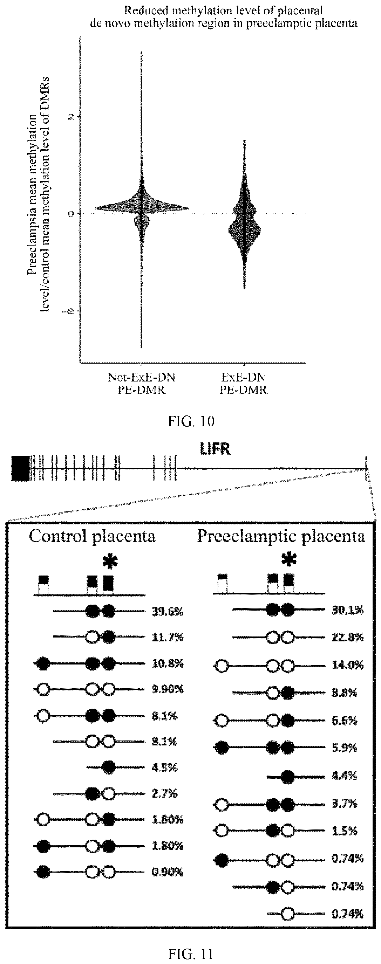

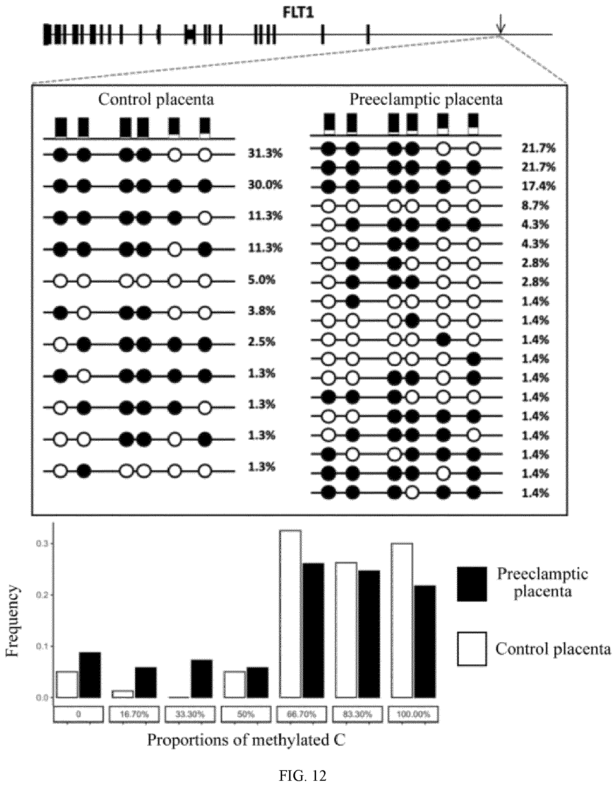

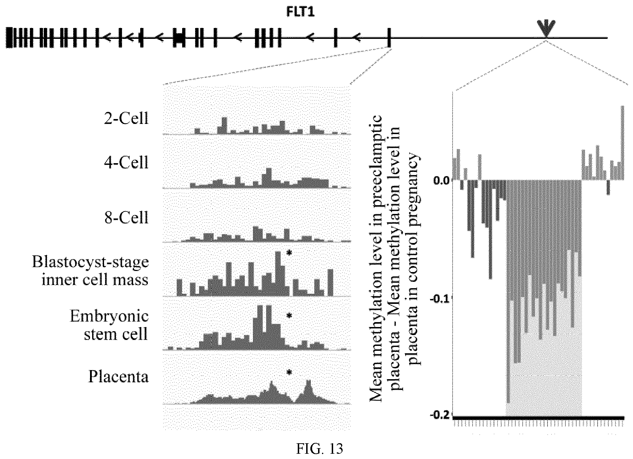

To wit, the methylation level of ExE-DN PE-DMRs in the preeclamptic placenta was significantly decreased in comparison to the placenta in normal pregnancy (FIG. 10). By aligning the preeclampsia-specific hypo-methylated regions with the placental *de novo* methylation regions, it was found that 52% of the preeclampsia-specific hypo-methylated regions overlapped the placental *de novo* methylation regions, so the preeclamptic placentas were obviously defective in the course of *de novo* methylation to the placental lineage. Two examples of preeclampsia-specific *de novo* methylation defects in the placental lineage were as shown in FIGS. 11 and 12. The analysis of the whole-genome chromatin accessibility to different developmental stages (2-cell stage, 4-cell stage, 8-cell stage, inner cell mass at the blastocyst stage, embryonic stem cell, and placenta) of an embryo showed that the chromatin accessibility to the genes in vicinity of the preeclampsia-specific hypo-methylated region was specifically increased in morula, blastocyst, or placenta, as shown in FIG. 13.

Time-series analysis of single-cell RNA expression: The RNA expression data were extracted using Monocle (version 0.2.0). The data were subjected to dimensionality reduction by the principal component analysis method (reduce_dimension function, Monocle) and the UMAP method. For each cell, the mean expression levels of its homologous genes (genes within the range of 50 kbp base pairs near the PE-hypo-DMR or PE-hyper-DMR position) were calculated as the expression levels of PE-hypo-DMR genes and PE-hyper- DMR genes.

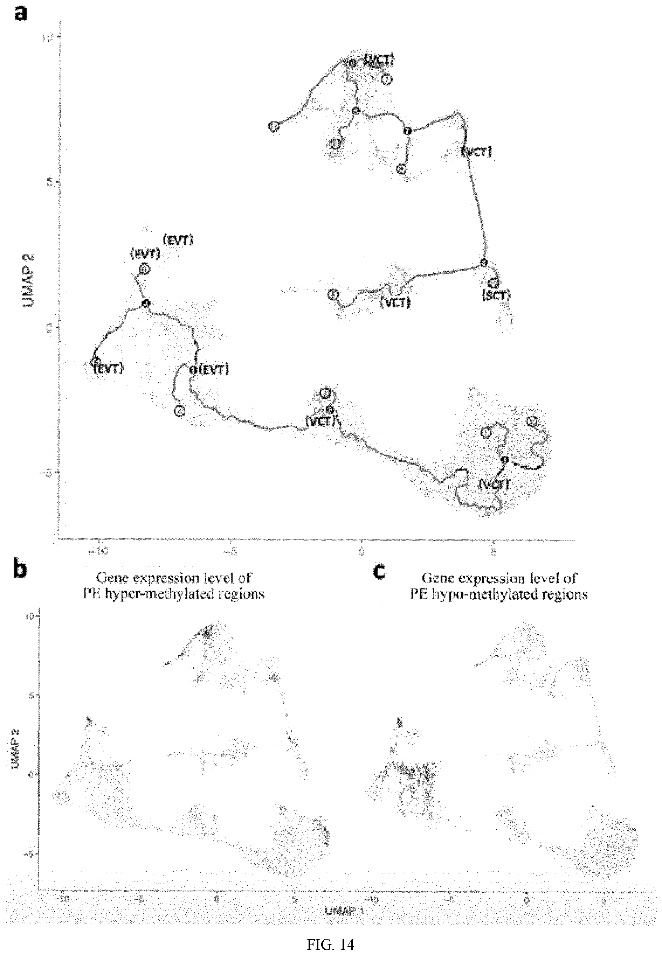

By analyzing the gene expression levels during the differentiation of placental villous cytotrophoblasts (VCTs) into syncytiotrophoblasts (SCTs) or extravillous trophoblasts (EVTs), it was found that, as shown in FIG. 14, the genes in vicinity of the preeclampsia-specific hypo-methylated regions were mostly specifically expressed in EVT cells, while the genes in vicinity of the preeclampsia-specific hyper-methylated regions were mostly specifically expressed in VCT cells.

Example 9: Distinguishing germ cells or embryonic tissues at different developmental stages by the mean methylation level of preeclampsia-specific methylated regions

**Experimental Materials**

DNA methylation database *of Homo sapiens* ova (CRA000114)
DNA methylation database *of Homo sapiens* sperms (CRA000114)
DNA methylation database of *Homo sapiens* embryo at 8-cell cleavage stage (CRA000114)
DNA methylation database *of Homo sapiens* morula-stage embryo (CRA000114)
DNA methylation database of *Homo sapiens* embryonic tissues from inner cell mass of *Homo sapiens* blastocyst-stage embryo (CRA000114)
DNA methylation database *of Homo sapiens* fetuses at 6 gestational weeks (CRA000114)
Preeclamptic placentas in pregnant women (collected from Peking University Third Hospital, Clinical Research Ethics Approval Document No.: IRB00001052-16009)
Placentas in normal pregnancy (collected from Peking University Third Hospital, Clinical Research Ethics Approval Document No.: IRB00001052-16009)

**Experimental Methods and Results**

The experimental methods for performing the DNA methylation sequencing on placentas and the data analysis were the same as those described in Example 4, Example 5, and Example 6.

PE-hyper-DMRs and PE-hypo-DMRs could be obtained through the experiments and analyses described in the above-mentioned examples. Statistical analysis of the mean methylation levels of the above-mentioned regions in the methylation databases of human ovum, sperm, 8-cell cleavage stage, morula, inner cell mass at the blastocyst stage, and fetuses at 6 gestational weeks showed that the early embryonic development process following the gametogenesis involved a process of from extensive whole-genome demethylation (represented by 8-cell cleavage stage, morula, blastocyst) to *de novo* methylation (represented by normal placenta and embryo). On the trajectory formed in the course of epigenetic changes, epreeclamptic placentas could be well distinguished from early embryos/ fetuses based on the mean methylation levels of the preeclampsia-specific hyper-methylated regions and hypo-methylated regions.

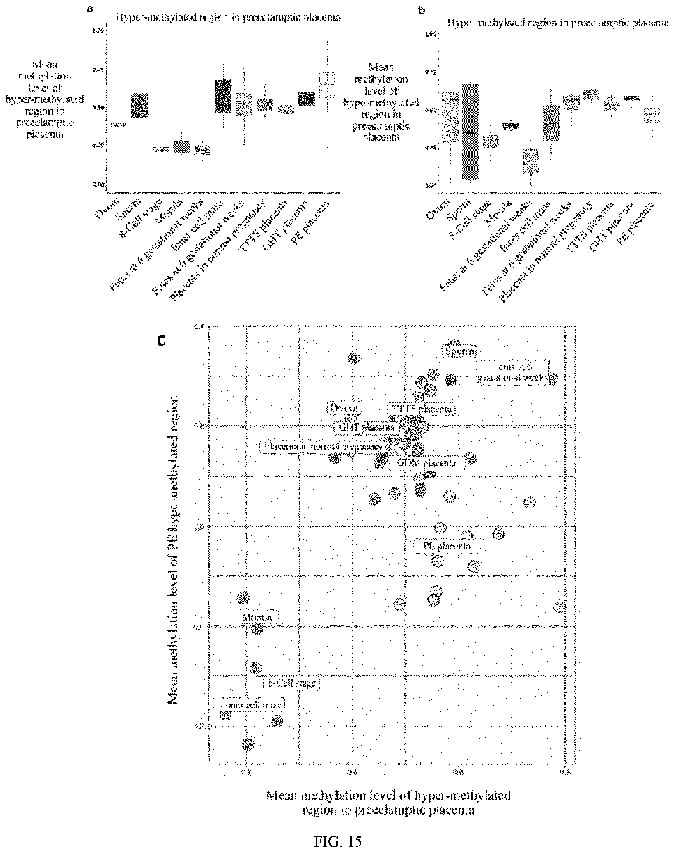

FIGS. 15a and 15b show the mean methylation levels of the preeclampsia-specific hyper-methylated and hypo-methylated regions in embryos (or tissues) at different developmental stages and placentas with different pregnancy complications, respectively. FIG. 15c shows a scatter plot drawn for germ cells or embryonic tissues at different developmental stages with the mean methylation level of the preeclampsia-specific hyper-methylated regions as the x-axis and the mean methylation level of the preeclampsia-specific hypo-methylated regions as y-axis. The preeclamptic placentas, placentas without the placenta-derived gestational disorder, sperms, ova, 8-cell stage, morula, and blastocyst could be well distinguished.

To wit, as shown in FIG. 15, the result further demonstrated that PE-hyper-DMRs and PE-hypo-DMRs were specific to preeclamptic placentas.

Example 10: Distinguishing placentas with different pregnancy complications by the mean methylation level of preeclampsia-specific methylated regions

**Experimental Materials**

Gestational placenta with gestational hypertension
Gestational placenta with gestational diabetes
Gestational placenta with twin-to-twin transfusion syndrome
Preeclamptic placenta
Placenta in normal pregnancy
(All of the above placentas were collected from Peking University Third Hospital, Clinical Research Ethics Approval Document No.: IRB00001052-16009.)

**Experimental Methods and Results**

The experimental methods for performing the DNA methylation sequencing on gestational placentas with preeclampsia, gestational hypertension, gestational diabetes, and twin-to-twin transfusion syndrome, and placenta in normal pregnancy as well as the data analyses were the same as those described in Example 4 and Example 6.

PE-hyper-DMRs and PE-hypo-DMRs could be obtained through the experiments and analyses described in the above-mentioned examples. Statistical analysis of the mean methylation levels of the above regions in the placentas with gestational hypertension, gestational diabetes, and twin-to-twin transfusion syndrome, placentas without the placenta-derived gestational disorder, and preeclamptic gestational placentas showed that the mean methylation levels of the preeclampsia-specific hyper-methylated regions and hypo-methylated regions could be used to distinguish different pregnancy complications (FIG. 15c). Therefore, there were specific methylation modification differences in the PE-hyper-DMRs and PE-hypo-DMRs in preeclamptic placentas.

Example 11: Differences in methylation haplotypes in preeclampsia group and control pregnancy group

**Experimental Materials**

Placenta in control pregnancy
Gestational placenta with preeclampsia
Plasma from healthy non-pregnant women
Control maternal plasma
Preeclamptic maternal plasma
(All of the above samples were collected from Guangdong Maternal and Child Health Hospital, Clinical Research Approval No. YL No. [201701044].)

**Experimental Methods and Results**

Extraction of placental tissue gDNA/plasma cfDNA and construction flow of DNA library were roughly the same as those described in Example 1, Example 2, Example 4, and Example 5.

Methylation haplotypes were extracted within the preeclampsia-specific differentially methylated regions obtained from the above analysis. Each of the haplotypes was analyzed using the R language (3.5.2) to screen out the preeclampsia-specific methylation haplotype, which was required to satisfy the condition that the frequency of this haplotype occurring in the preeclamptic placenta was at least 20 times that in the control placenta, or that the frequency of this haplotype occurring in the control placenta was at least 20 times that in the preeclamptic placenta.

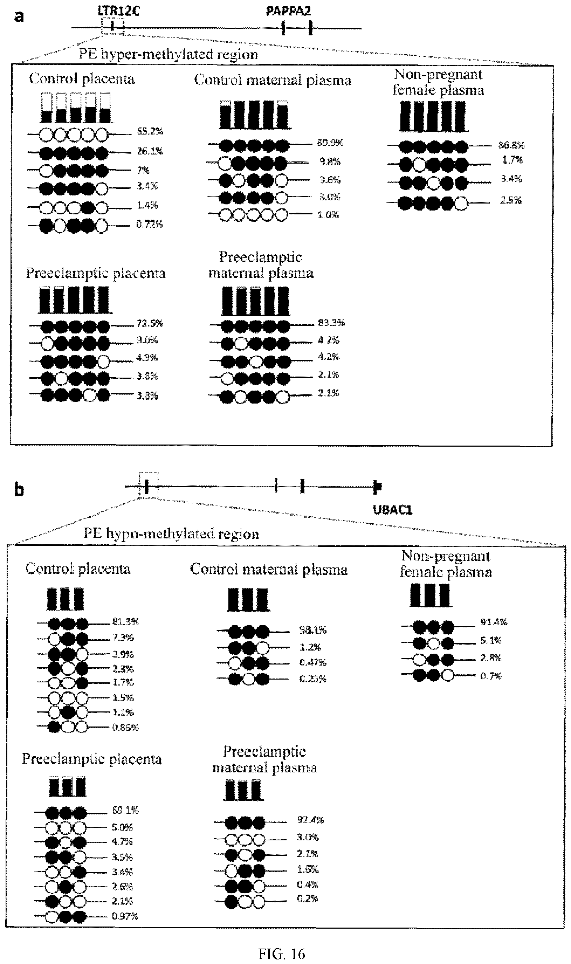

The screened methylation haplotypes were listed in Table 3. There were significant and consistent differences in the frequencies of the preeclampsia-specific methylation haplotypes listed in this table occurring in the control gestational placentas and preeclamptic placentas, e.g., as shown in FIG. 16. FIGS. 16a and 16b show the methylation haplotypes in the preeclampsia-specific hyper-methylated region and hypo-methylated region, respectively.

Example 12: Design and verification of EP-007 probe or probe set

**Experimental Materials**

Synthesized EP-007 probe or probe set;
KAPA double-strand DNA library kit (KAPA HTP Lib PrepKit, KK8235);
Agencourt AMPure XP beads (Beckman, A63882);
Dynabeads™ M-270 streptavidin (Thermo Fisher Scientific, 65306).

**Experimental Methods and Results**

As shown in FIG. 17a, the EP-007 probe or probe set was designed based on the following basic principle: The probe was composed of several sequences that were 59 bp in length and complementary to the sequences of the methylation haplotype-specific loci after sulfite sequencing. For each of the CpG loci of each preeclampsia-specific methylation haplotype, one methylated (G) probe and one unmethylated (A) probe corresponding to this locus would be synthesized. The two probes covering adjacent loci contained 40-bp overlapping sequences. The probes had a biotin label at the 3'-end.

Probe design was conducted on the genomic region where the preeclampsia-specific methylation haplotypes (Attached List 3) obtained from the above analysis were located to synthesize a DNA probe complementary thereto (Twist Inc., U.S.A.). As shown in FIG. 17a, a 59-bp DNA complementary strand was basically designed.

Exemplarily, in the present example, the sequence of the target region desired to be detected was: >13 :29140769-29140827

CAGGCAACCTGTAAAGGAGGGCCAACTGCGACTGCCTGGCCCACTCCCAGAG

ACCAGAA

(SEQ ID NO: 1).

This sequence was part of the upstream region of the FLT1 gene. Based on the design principle of probes described in the present disclosure, the probes designed for the aforementioned regions were as follows:

1. 5'-TAGGTAATTTGTAAAGGAGGGTTAATTGCGATTGTTTGGTTTATTTTTAG AGATTAGAA-3' (i.e., CpG -> C; non-CpG->T) (SEQ ID NO: 2)

2. 5'-TAGGTAATTTGTAAAGGAGGGTTAATTGTGATTGTTTGGTTTATTTTTAG AGATTAGAA-3' (i.e., all C->T) (SEQ ID NO: 3)

3. 5'-GTTCGTTGGATATTTTTTTTCGGTTGACGTTGACGGATCGGGTGAGGGTT TTTGGTTTT-3' (i.e., reverse complement. CpG -> C; non-CpG->T) (SEQ ID NO: 4)

4. 5'-GTTTGTTGGATATTTTTTTTTGGTTGATGTTGATGGATTGGGTGAGGGTTT TTGGTTTT-3' (i.e., reverse complement. All C->T)) (SEQ ID NO: 5)

The synthesized DNA probes were subjected to double-stranded library construction and sequencing according to the operation instructions of the KAPA double-stranded DNA library construction kit. As listed in Table 12, the proportion of data less than 1X coverage was almost 0 and the normalized mean coverage of 0.2 or less was almost 0, so the probe could cover the selected region well.

Table 12 Sequencing Results of Double-Stranded Library Construction for EP-007 probe or probe set

|  | Sequencing after Double-Stranded Library Construction for EP-007 Probe or Probe Set | |
| --- | --- | --- |
|  | Duplicate-1 | Duplicate-2 |
| Total base (Gb) | 0.8557455 | 0.7458039 |
| Mean coverage | 1029.837458 | 964.1954077 |
| Fraction of target bases <1X | 0 | 0.00006945 |
| Normalized mean coverage <0.2 | 0.002020202 | 0.002020202 |

Meanwhile, FIG. 17a shows the sequencing results after double-stranded library construction for the EP-007 probe or probe set. The x-axis represents the ratios of the coverage of different probe fragments to the mean coverage, and the y-axis represents the distribution density. The coverage distribution is in line with the Poisson distribution. Therefore, the synthetic quality of the EP-007 probe or probe set is unbiased and reliable.

Example 13: Optimization of hybridization time and hybridization and eluting temperatures of EP-007 probe or probe set

**Experimental Materials**

500 ng of pre-library product for methylation library construction;
Hybrid Elution Kit of Non-Invasive Preeclampsia Early Screening Kit (Euler Genomics, EU-TQ-MV1);
Agencourt AMPure XP beads (Beckman, A63882);
Dynabeads™ M-270 streptavidin (Thermo Fisher Scientific, 65306).

**Experimental Methods**

The experimental methods were basically the same as those described in Example 4.

**Experimental Results**

Hybridization and eluting temperatures

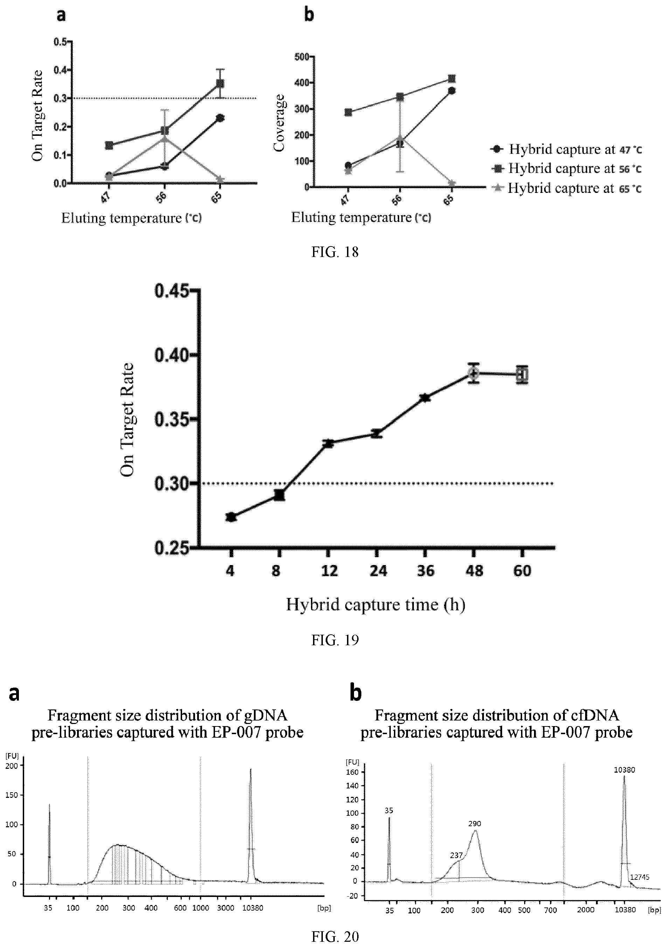

FIGS. 18a and 18b show the comparisons of the on target rates and the mean coverage in the sequencing results obtained at different combinations of the hybridization temperatures (47°C, 56°C, and 65°C) and the eluting temperatures (47°C, 56°C, and 65°C), respectively. The circle represents hybridization at 47°C, the square represents hybridization at 56°C, and the triangle represents hybridization at 65°C.

By comparing the on target rates and the mean coverage in the sequencing results of the library products at different combinations of the hybridization temperatures (47°C, 56°C, and 65°C) and the eluting temperatures (47°C, 56°C, and 65°C), it was concluded that the capture effect was optimal in case of hybridization at 56°C and elution at 65°C. That is, the on target rate was greater than 30% (as shown in FIG. 18a) and the mean coverage was the highest in case of the same data size (as shown in FIG. 18b).

Hybridization time

FIG. 19 shows the on target rates of the library products when hybridized at the eluting temperature of 56°C for different hybridization time (4 h, 8 h, 12 h, 24 h, 36 h, 48 h, and 60 h). It was found that the on target rate reached a sequencing result of greater than 30% when the hybridization time was 12 h.

The results show that the on target rate of greater than 30% may be obtained when the hybridization time is 12 h or longer.

Example 14: Capture of preeclamptic methylation modification difference loci in gDNA or cfDNA using EP-007 probe or probe set

**Experimental Materials**

    500 ng of pre-library product for methylation library construction;
    Hybrid Elution Kit of Non-Invasive Preeclampsia Early Screening Kit (Euler Genomics, EU-TQ-MV1);
    Agencourt AMPure XP beads (Beckman A63882);
    Dynabeads™ M-270 streptavidin (Thermo Fisher Scientific, 65306).

**Experimental Methods**

The experimental methods were basically the same as those described in Example 4.

**Experimental Results**

FIG. 20 shows the fragment size distribution of the library after probe capture as determined by Agilent 2100 Bioanalyzer. The peaks at 35 bp and 10,380 bp are DNA molecular markers. FIG. 20a shows the fragment size of the library constructed based on placenta gDNA, and FIG. 15b shows the fragment size of the library constructed based on cfDNA in plasma.

That is to say, from 500 ng of pre-library for methylation library construction, 2.98 ng/μL of library could be enriched after capture with the EP-007 probe or probe set. Determined by Agilent 2100 Bioanalyzer, the fragment size of the library constructed based on placenta gDNA and the fragment size of the library constructed based on cfDNA in plasma were both about 300 bp, as captured with the EP-007 probe or probe set.

Example 15: Construction of preeclampsia risk prediction model

**Experimental Materials**

Peripheral blood drawn from the control pregnant women and preeclamptic pregnant women at 10 to 38 gestational weeks and stored in Apostle MiniMax™ cfDNA non-invasive blood collection tubes (Apostle, Inc. Silicon Valley, California.); (collected from Guangdong Maternal and Child Health Hospital, Clinical Research Approval No.: YL No. [201701044])

    Protease K (Qiagen, 19133);
    SDS (Sigma, 74255-250G);
    Dynabeads™MyOne™ Silane (Thermo Fisher Scientific, 37002D);
    MagMAX™ Cell Free DNA Lysis/Binding Solution (Thermo Fisher Scientific, A33600);
    MagMAX™ Cell Free DNA Wash Solution (Thermo Fisher Scientific, A33601);
    Absolute ethanol (Beijing Chemical Works, 00500);

EB buffer (Qiagen, 19086);
DNase/RNase-free water (Solarbio, R1600);
EZ DNA Methylation-Gold™ Kit (Zymo Research, D5006);
Tequila V3 DNA Methylation Library Kit (Euler Genomics, EU-TQ-Methy);
EB buffer (Qiagen, 19086);
SeqCap EpiGiant Enrichment Probe (Roche, 07138911001);
Agencourt AMPure XP beads (Beckman, A63882);
Dynabeads™ M-270 streptavidin (Thermo Fisher Scientific, 65306);
ILLUMINA NextSeq PE150 sequencing kit.

**Experimental Methods and Results**

The plasma from 22 pregnant women was subjected to experiments in molecular biology and analysis of the DNA methylation level. The experimental methods were the same as those described in Examples 2, 3, 5, 9, and 12. There were 6 pregnant women and 16 preeclamptic pregnant women.

In the control pregnancy group and the preeclampsia group, the methylation levels of each of the preeclampsia-specific methylation haplotype regions in Attached List 3 were averaged within the groups to obtain matrices M (CTRL) and M (PE), and calculate the Pearson correlation coefficients r (CTRL) and r (PE) of the control pregnancy group and the preeclampsia group by the leave-one-out method.

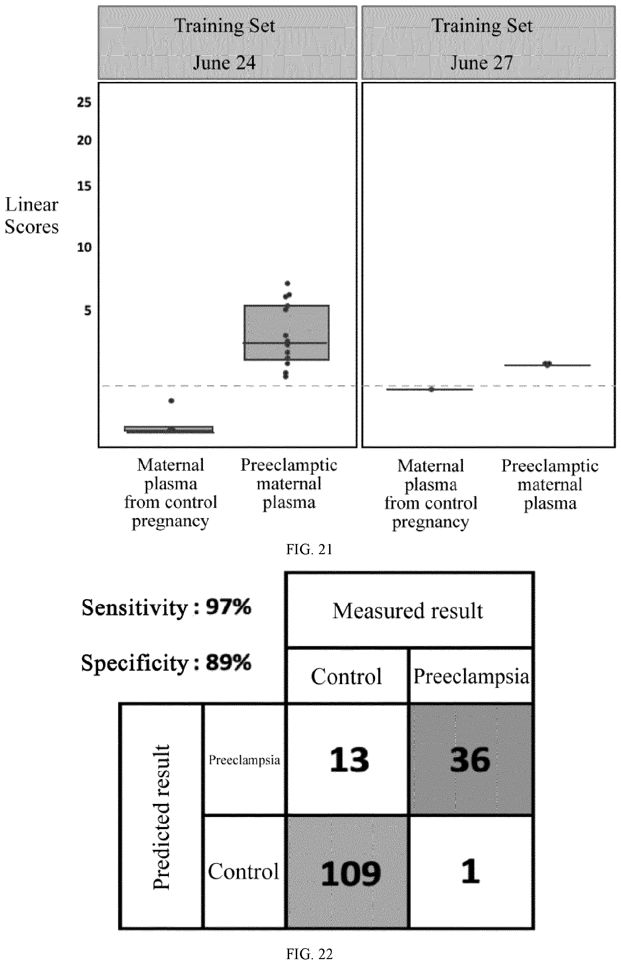

As shown in FIG. 21, a linear regression analysis between r(CTRL) and r(PE) and the clinical diagnosis of 22 pregnant women was made to obtain linear scores, which could be used to evaluate the degree of preeclampsia-associated placental dysplasia, that is, the degree of placental dysplasia in pregnant women afflicted with preeclampsia was high, and its linear score was high accordingly; however, the placental development of the control pregnancy was relatively good, so its linear score was low.

FIG. 16 shows linear scores statistically calculated by correlation and linear regression after analyais of the methylation levels of PE-DMRs.

Example 16: Verification of preeclampsia risk prediction model by retrospective clinical samples

**Experimental Materials**

The experimental materials were the same as those used in Example 13, in which the peripheral blood samples drawn from a total of 159 pregnant women with preeclampsia or control pregnancy at 10 to 38 gestational weeks (Guangdong Maternal and Child Health Hospital, YL No. [201701044]).

**Experimental Methods and Results**

In four batches, four experimenters conducted experiments in molecular biology and analysis of haplotypes in 159 pregnant women by the same experiment methods as described in Examples 2, 3, 5, 9, and 12.

For the preeclampsia-specific methylation haplotype regions listed in Attached List 3, the mean methylation level of the subject in each region was calculated to obtain the matrix M (test), and the Pearson correlation coefficients between M (test) and M (CTRL) and M (PE), i.e., r (CTRL) and r (PE), were calculated, respectively. Further, r (CTRL) was compared with r (PE) to predict the risk of preeclampsia. If r (CTRL) > r (PE), the risk of preeclampsia was high; if r (CTRL) < r (PE), the risk of preeclampsia was low.

As shown in FIG. 22, the results show that this method may differentiate pregnant women with preeclampsia from pregnant women of control pregnancy, with the sensitivity of 97% (36/37) and the specificity of 89% (109/122).

Example 17: Distinguishing different severities of subtypes of preeclampsia by the preeclampsia risk prediction model

**Experimental Materials**

The experimental materials were the same as those used in Example 13, in which the peripheral blood samples drawn from a total of 159 pregnant women with preeclampsia or control pregnancy at 10 to 38 gestational weeks (Guangdong Maternal and Child Health Hospital, YL No. [201701044]).

**Experimental Methods and Results**

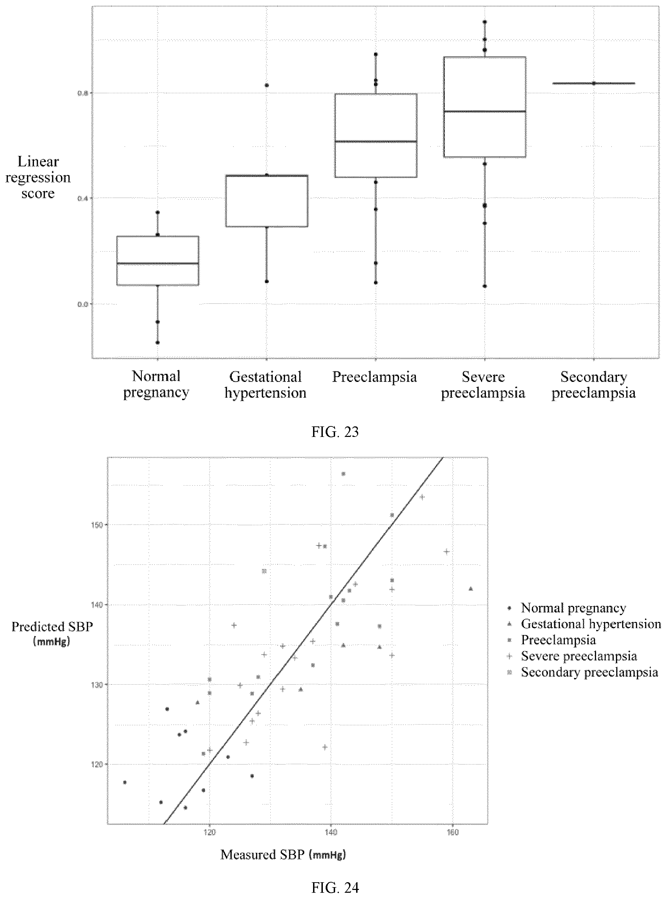

The experimental methods were roughly the same as those used in Example 15. For the maternal plasma from 159 subjects, the correlation between r (CTRL) and r (PE) and the clinical outcomes showed that gestational hypertension, preeclampsia, severe preeclampsia, and secondary preeclampsia could be better distinguished (FIG. 23).

Example 18: Prediction of the highest pressure in the first stage of labor of pregnant women by the preeclampsia risk prediction model

**Experimental Materials**

The experimental materials were the same as those used in Example 13, in which the peripheral blood samples drawn from a total of 181 pregnant women with preeclampsia or control pregnancy at 10 to 38 gestational weeks (collected from Guangdong Maternal and Child Health Hospital, Clinical Research Approval No.: YL No. [201701044]).

**Experimental Methods and Results**

The regression statistical analysis was performed on r (CTRL) and r (PE) of 22 pregnant women and the highest pressure in the first stage of labor in the corresponding clinical outcomes, to establish a model between the similarity of the subject placenta to the control pregnancy and preeclamptic placenta and the highest systolic blood pressure in the first stage of labor of pregnant women. Then, the model was used to predict the highest systolic blood pressure in the first stage of labor of 159 pregnant women. As shown in FIG. 24, the highest systolic blood pressure in the first stage of labor predicted by the model was in a good linear relationship with the highest systolic blood pressure measured in clinical.

The above-mentioned examples of the present disclosure are provided by way of instances only for clearly illustrating the present disclosure, and are not intended to limit the embodiments of the present disclosure. For a person skilled in the art, other variations or modifications in different forms may also be made to the above descriptions. It is unnecessary and impossible to exhaust all embodiments here. Any modifications, equivalent replacements, improvements, etc. made within the spirit and principle of the present disclosure shall be included within the protection scope of the claims of the present disclosure.

**Attached List 1**

Genomic regions of chromosome 1:
10609-10732;854755-855015;855281-855548;865578-865745;906938-907140;918299-91
8633;933296-933610;981271-981434;1002867-1003145;1064840-1065000;1091548-1091808 ;1096501-109690
8;1098952-1099212;1100360-1100496;1103155-1103312;1104187-1104346 ;1141776-1142016;1145641-11459
09;1222110-1222220;1267412-1267789;1268092-1268275 ;1268747-1268985;1269212-1269412;1353574-1353
851;1356727-1356902;1377431-1377557 ;1487465-1487644;1713644-1713847;1906534-1906862;1910292-191
0489;1953059-1953419 ;2064041-2064202;2066274-2066422;2124985-2125125;2160972-2161105;2161785-21
62095 ;2164535-2164639;2262419-2262565;2270684-2270893;2279479-2279833;2307620-2307890 ;2316165-
2316792;2339934-2340225;2350002-2350312;2365498-2365658;2379747-2379888 ;2382636-2382868;238784
5-2387952;2391121-2391407;2401568-2401734;2403576-2403735 ;2408494-2408653;2409799-2410059;24113
77-2411499;2415770-2416030;2425732-2426018 ;2435099-2435257;2437018-2437230;2500798-2500922;2527
240-2527344;2719832-2719954 ;2722273-2722421;2766756-2766872;2774240-2774347;2777158-2777281;277
9044-2779211 ;2782993-2783117;2792661-2792830;2799264-2799432;2827786-2827930;2835104-2835267 ;2
835823-2835958;2836448-2836736;2844082-2844430;2844871-2845339;2845494-2845639 ;2846855-2846999;
2850500-2850638;2850819-2850952;2859522-2859835;2883701-2883805 ;2886654-2886923;2899449-289965
6;2918366-2918705;2919692-2919797;2938885-2939081 ;2980518-2980846;3006206-3006330;3007620-30077
69;3026100-3026234;3032651-3032868 ;3042333-3042541;3044643-3045005;3051474-3051627;3057365-3057
479;3059136-3059331 ;3059717-3059866;3079075-3079335;3080079-3080462;3080672-3080780;3081185-308
1373 ;3089024-3089321;3103043-3103153;3111175-3111310;3115439-3115689;3128184-3128426 ;3128962-3
129092;3130390-3130559;3134161-3134277;3135301-3135443;3141877-3141993 ;3143654-3143914;3144710-
3144820;3147865-3148134;3153144-3153293;3159082-3159352 ;3162051-3162160;3182821-3183261;318454
6-3184823;3191481-3191660;3192387-3192628 ;3193002-3193115;3193864-3194003;3195486-3196098;32038
66-3204440;3206252-3206382 ;3207299-3207630;3208767-3209099;3213487-3213635;3233906-3234049;3234
220-3234322 ;3265820-3266044;3272171-3272296;3274547-3274736;3276377-3276803;3277493-3277636 ;32
80492-3280771;3282727-3282940;3289767-3290050;3294220-3294333;3302198-3302324 ;3308428-3308688;3
319265-3319553;3321194-3321504;3325631-3325772;3327972-3328117 ;3331049-3331336;3332089-3332295;

3342161-3342279;3351106-3351393;3354296-3354480 ;3377807-3377935;3378264-3378459;3417006-3417390;3420736-3420941;3421689-3421949 ;3425534-3425794;3431290-3431550;3459805-3460107;3460418-3460678;3464723-3464983 ;3495625-3495795;3504103-3504260;3512718-3512860;3556937-3557273;3669230-3669375 ;4275026-4275244;4391775-4392024;4736060-4736244;4770676-4770847;5893828-5893942 ;5917797-5917916;5932351-5932480;6088559-6088667;6111671-6111814;6134181-6134558 ;6148978-6149125;6159517-6159648;6169924-6170086;6294636-6294911;6341139-6341272 ;6530699-6530818;6638413-6638578;6664102-6664268;7081270-7081375;7111114-7111237 ;7723484-7723664;7724019-7724165;7797229-7797491;8086837-8087054;8274043-8274190 ;8298426-8298685;8403907-8404167;8510416-8510608;10689960-10690091; 10917318-10917578;11473186-11473367;11938458-11938578;12251597-12252008;12609996-12610208;13910569-13910698;14156736-14156846;14309883-14310030;15541294-15541554; 16061427-16061554;16321032-16321158;16554135-16554558;16861392-16861519;16950647-16950793; 17019523-17019823;17026318-17026455;18060459-18060568;18149518-18149705;18686617-18686743;18691983-18692140;19054751-19054854;19665069-19665244;20005721-20005854;21755768-21755917;22927195-22927331;23519947-23520057;26136828-26136971;26503622-26503725;27677607-27677867;27849101-27849266;27994887-27995147;29189324-29189642;30240188-30240341;32169629-32169747;32221936-32222059;32936900-32937089;34420860-34421008;34639624-34639745;36644255-36644374;38181454-38181574;41114654-41114914;41131945-41132084;41253449-41253606;41583010-41583158;41889160-41889394;43392604-43392818;43533970-43534316;43814208-43814324;44031196-44031306;44068789-44068892;45082810-45083346;45271751-45271858;45279449-45279745;46858809-46858941;48452689-48452811;48764353-48764476;53386630-53386996;53528963-53529073;53742195-53742451;53904603-53904799;53935873-53936011;54059896-54059999;54775727-54775970;55521708-55522062;55844167-55844286;56323691-56323793;58521893-58522110;61519888-61520012;62662133-62662237;62783533-62783678;63784342-63784449;68515754-68516070;73571459-73571578;74663494-74663641;74713906-74714135;75590829-75590933;76262373-76262838;82039775-82040055;86577457-86577618;86988863-86989062;90409071-90409331;91189205-91189331;91191796-91192088;94205105-94205238;96135602-96135711;107587472-107587734;110993523-110993625;113291201-113291309;119535588-119535693;119542118-119542296;119549295-119549402;120377669-120377779;144994520-144994637;145092216-145092576;147737221-147737391;149146695-149146855;149148152-149148289;149148886-149149036;149286330-149286476;149857877-149858023;150953712-150953943;151509682-151509791;151810377-151810525;153581750-153581873;153599383-153599573;153934210-153934328;154516404-154516523;155156325-155156430;156047075-156047186;159683404-159683510;159683776-159683891;160178004-160178283;160336399-160336590;160389227-160389339;161947938-161948198;162337162-162337441;166765956-166766137;173579033-173579162;173887148-173887285;179334176-179334289;181098503-181098635;182810641-182810747;194326822-194327068;196547397-196547498;196945529-196945637;197744907-197745026;200005266-200005406;200010631-200010788;200969705-200969906;201475326-201475452;201476557-201476665;202172867-202173002;204531685-204531837;207262319-207262429;207992427-207992724;210857019-210857247 ;212062720-212063173;214170992-214171132;214638070-214638191;217310965-217311204;218524557-218524685;220552393-220552515;221065070-221065171;222717221-222717387;223567849-223568034;225566690-225566949;225706939-225707215;226651855-226651998;226825434-226825539;227921627-227921778;227947136-227947322;227974825-227975111;228079042-228079222;228289732-228289880;228473852-228474036;228503487-228503829;228528780-228529062;228558892-228559169;230367477-230367737;230513970-230514117;234667362-234667642;235587072-235587249;236228789-236228910;236273193-236273312;238847990-238848099;244080100-244080219;245851484-245851695;246860128-246860388;247542223-247542331;247802774-247803023

Genomic regions of chromosome 10:

415821-415938;461809-462031;577179-577311;579226-579335;664696-664804;665113-665313;1198688-1198835;1209314-1209498;1228965-1229068;1230849-1230965;1235997-1236180;1245359-1245473;1256493-1256597;1259812-1259956;1260331-1260513;1401818-1402007;1402293-1402426;1506157-1506282;1511206-1511344;1547357-1547625;1583222-1583482;1692977-1693174;1725096-1725230;1789125-1789253;2487078-2487194;3168614-3168777;3172197-3172481;3300397-3300522;3627729-3627972;3724435-3724678;3805226-3805441;3870105-3870305;5580951-5581070;5631674-5631971;5702083-5702221;6919371-6919485;7454580-7454898;8100114-8100286;8100506-8100668;8116869-8117072;10308512-10308678;119234

74-11923604;11934141-11934282;19777919-19778061;22158229-2215849
1;22624674-22624790;23369135-23369236;23480697-23480815;23484032-23484141;249094
56-24909607;25010711-25011039;25755061-25755264;30346647-30346789;30720559-3072
0774;35080974-35081093;35258200-35258474;35928604-35928713;38069487-38069801;426
44799-42645192;43247943-43248070;43393702-43393833;43447011-43447171;43697849-4
3698051;44198410-44198517;45701113-45701247;45799169-45799349;46961780-46961986;
49673519-49673645;49954066-49954186;50506754-50507035;54714822-54714950;6202350
8-62023688;63422511-63422630;65647086-65647213;67189225-67189336;68411485-68411
658;71601337-71601482;71892007-71892320;72015285-72015462;72218065-72218443;7272
7774-72727913;73394970-73395113;73497243-73497523;73521638-73521755;73575412-73
575520;73769037-73769219;77794599-77794758;79996996-79997144;81444843-81445113;8
1904171-81904480;82023899-82024011;82169057-82169163;83068999-83069136;83263584
-83263745;86004952-86005070;88703075-88703322;90488114-90488446;91296311-912965
10;92592305-92592433;94180416-94180559;94820953-94821086;95123660-95123879;95205
504-95205611;98144368-98144497;99160096-99160215;100227560-100227832;101281002-101281262;10128
2013-101282145;102046685-102046827;102748540-102748734;102778604
-102778717;102822249-102822357;102985788-102985915;103530134-103530370;10353922
8-103539367;103599571-103599692;104535275-104535406;104913266-104913526;1053619
62-105362081;106011700-106011830;106014437-106014881;106074268-106074603;110672
138-110672313;111216768-111216874;118032337-118032898;118434498-118434636;11889
1118-118891266;120359308-120359441;120543311-120543444;121295914-121296095;1223
49130-122349232;123371156-123371303;124638868-124638991;125114476-125114594;126
256988-126257240;126281463-126281600;126296075-126296434;126711969-126712220;12
6712372-126712559;129144179-129144307;129535641-129535901;130274349-130274549;1
30684697-130684873;130855094-130855278;130868340-130868448;130870128-130870256;
131354666-131354783;131529354-131529516;131639526-131639751;131706743-131706892 ;131731385-131
731626;132154687-132154824;133204168-133204317;133208394-13320852
8;133208761-133208879;133644552-133644781;133793645-133793783;133874566-1338748
33;133921213-133921429;133947644-133947755;133948074-133948227;133949165-133949
269;133950676-133950967;133953845-133954068;133961848-133962037;133981439-13398
1571;133981975-133982102;133983793-133983964;134014432-134014623;134016056-1340
16336;134016854-134017006;134018801-134019026;134019524-134019727;134021284-134
021525;134022557-134022746;134034573-134034936;134040213-134040456;134041500-13
4041684;134053767-134053915;134055143-134055267;134062149-134062260;134073893-1
34074009;134085786-134085950;134095204-134095725;134107089-134107228;134117537-134117650;13415
0489-134150734;134188750-134189010;134219125-134219410;134224956
-134225261;134341216-134341361;134495624-134495769;134527948-134528050;13460845
4-134608563;134610501-134610634;134621538-134621689;134624109-134624244;1346473
89-134647533;134692498-134692617;134692993-134693128;134694298-134694491;134698
676-134698863;134729845-134730203;134732799-134733122;134735972-134736112;13473
8977-134739370;134740633-134740899;134790766-134790938;134791519-134791769;1347
93947-134794059;134806480-134806584;134837866-134838078;134838342-134838596;134
842578-134842818;134844342-134844924;134861023-134861180;134875725-134875916;13
4881949-134882099;134884125-134884280;134894796-134895108;134896348-134896638;1
34908186-134908325;134913507-134913620;134913890-134914022;134928095-134928246;
134928613-134928749;134938102-134938249;134945401-134945727;134973001-134973107 ;134979125-134
979261;134980505-134980628;134980789-134981053;134981669-13498201
0;134993121-134993323;134996378-134996494;134998356-134998578;135001547-1350016
73;135005324-135005461;135016400-135016541;135022993-135023127;135026828-135026
943;135032460-135032633;135105810-135106070
Genomic regions of chromosome 11:
433315-433423;460626-461036;491225-491374;562173-562629;563859-564043;569264-569546;598324-59869
0;800391-800661;840419-840522;841236-841377;1009590-1009740;1
025794-1026026;1027555-1027730;1077546-1077671;1213283-1213534;1215670-1215847;1
216193-1216453;1238618-1238853;1252151-1252272;1258450-1258590;1263257-1263411;1
263729-1264000;1264798-1265058;1275970-1276123;1326436-1326546;1331584-1331785;1
464311-1464480;1471563-1471675;1661630-1661737;1824048-1824490;1892042-1892375;1
918275-1918482;1940911-1941171;1948916-1949221;1976431-1976556;1977556-1977702;1 983001-1983194;2016351-2016626;2150583-2150730;2152311-2152513;2154047-2154952;2156307-2156544;2308361-2308488;2573990-2574106;2594839-2594942;2610068-2610195;2828647-2828779;2924858-2925027;2942754-2942867;5290745-5290849;6340684-6340944;8378686-8378973;11832933-11833213;15136178-15136295;15963013-15963197;17591774-17591902;17742018-17742147;19735182-19735320;19736191-19736471;22454151-22454499;23286497-23286693;23421709-23421953;23427917-23428068;23674871-23675037;23905153-23905261;27672696-27672807;32450424-32450591;35440031-35440158;35808666-35808786;35966361-35966568;39462691-39462987;41644388-41644520;44087634-44087748;44087973-44088080;44541358-44541464;45827403-45827875;46401359-46401504;46709238-46709341;47208979-47209160;47613367-47613484;47927067-47927327;56099764-56099898;57232348-57232508;58912211-58912327;59667014-59667119;60383157-60383407;61096982-61097114;61687817-61687937;61900808-61900991;61957678-61957809;63783225-63783416;63784025-63784166;64064979-64065125;64127709-64127816;64140341-64140470;64374712-64374819;64863168-64863379;64992997-64993107;65313934-65314043;65314375-65314541;65321406-65321662;65359412-65359570;65360017-65360227;65374749-65375224;65487450-65487551;65582798-65582918;65768425-65768673;66193938-66194128;66458798-66459081;66472290-66472413;66838979-66839093;67210269-67210384;67278778-67278904;67494341-67494473;68133047-68133219;68135679-68135932;68138818-68139042;68860016-68860122;69259196-69259335;69813138-69813530;70741247-70741377;71623045-71623320;72533239-72533357;74178113-74178268;74440515-74440645;76025109-76025321;76849076-76849217;80242887-80243022;80918570-80918873;81662731-81662889;82114950-82115250;85194996-85195305;85646499-85646618;88241585-88241766;89522850-89522959;92600068-92600334;93475896-93475999;94883424-94883564;112505587-112505690;113930736-113931017;114112901-114113129;114479593-114479698;114484683-114484831;116228521-116228700;117069780-117070018;117314862-117314964;117376078-117376338;117541468-117541600;119181451-119181587;119317202-119317399;119473914-119474019;119524039-119524211;120384015-120384150;121349643-121349767;123016009-123016126;124953019-124953193;126985893-126986034;127811623-127811817;128778360-128778620;129613524-129613831;129731669-129731883;129734455-129734599;130060344-130060521 ;131130899-131131039;131561146-131561268;131939119-131939245;131939970-131940174;132016219-132016419;133800709-133800949;134253340-134253625;134253820-134253954;134393379-134393576;134794919-134795051

Genomic regions of chromosome 12:

296473-296579;562849-563109;718054-718314;1059330-1059457;1937782-1937899;1948379-1948579;1973812-1973944;2215413-2215574;2787762-2787912;2788752-2788900;2800985-2801117;3309751-3309886;3310077-3310303;3529673-3529815;4543223-4543336;4554886-4555027;6729405-6729553;6745460-6745569;6946126-6946231;6959518-6959658;7062109-7062240;7071780-7071921;7167780-7167900;7583650-7583756;7781092-7781289;9892249-9892632;10251413-10251678;10264269-10264384;11760584-11760854;12419035-12419209;12618535-12618660;15654718-15654837;15743119-15743233;20094183-20094296;20267333-20267475;20268256-20268405;20705364-20705772;20935613-20935748;23439187-23439314;27924231-27924397;31270307-31270456;34471039-34471144;34475337-34475448;34478217-34478389;34489902-34490032;34491014-34491157;34516115-34516471;41086179-41086327;41966249-41966400;42979452-42979569;48921680-48922002;51717595-51717866;51718108-51718504;52258077-52258299;52309016-52309296;52408677-52408937;52540565-52540825;52636850-52637020;52789509-52789749;52994767-52995027;53453100-53453221;54408027-54408250;54422347-54422473;54441169-54441496;54447632-54448038;56031025-56031143;57290257-57290375;57351092-57351229;57569768-57569899;57599348-57599457;58000654-58000881;58013414-58013620;59689560-59689723;65218216-65218372;67072768-67073063;69198710-69199108;74564430-74564636;74564803-74564915;74796732-74796882;85187890-85188120;94533095-94533250;94580419-94580658;94676506-94676709;94939915-94940020;100041843-100041975;100823976-100824406;103696137-103696285;108985716-108985817;110270879-110271025;110506167-110506299;114236455-114236567;114263695-114263833;114877844-114877979;115131420-115131546;116354803-116354947;116808768-116809048;117579965-117580102;117627099-117627223;121343016-121343149;121661747-121661868;121890488-121890605;122356821-122356952;123215107-123215239;123738214-123738340;124393810-124394029;124773720-124773980;124870076-124870515;125217602-125217729;125271270-125271435;125627229-125627489;1276505 59-127650674;127965770-127965882;128869789-128870093;129148451-129148615;129190447-129190574;129468126-129468250;130184739-130184851;130384863-130385014;130572620-130572734;130683456-130683640;130840928-130841078;130908854-130909059;130909641-130909756;131194033-131194140;131366591-131366877;131367209-131367428;131437900-131438178;131529236-131529419;131572549-131572674;131605592-131605749;131850468-131850600;131865276-131865380;132169193-132169459;132280441-132280706;132620803-132621063;132663422-132663562;132680733-132680973;132682573-132682807;132687626-132687777;132688219-132688359;132688933-132689064;132822420-132822665 ;132824316-132824425;132837350-132837796;132839020-132839169;132848607-132848729;132849401-132849545;132851967-132852087;132863714-132864221;132880775-132881162;132895296-132895543;132898924-132899096;132952634-132952755;132956419-132956787;132980605-132980728;132981736-132981926;132982275-132982565;132987039-132987176;133004858-133004971;133012177-133012599;133056699-133056977;133100669-133100773;133101094-133101225;133120445-133120848;133123407-133123624;133135487-133135686;133159420-133159843;133160460-133160573;133161622-133162062;133177725-133177883;133197770-133198064;133336998-133337158

Genomic regions of chromosome 13:

19582812-19582925;20392317-20392577;20980095-20980206;21291152-21291297;22615324-22615469;22637826-22637943;23458123-23458364;25801840-25802080;25946831-25947200;29107238-29107373;29148868-29148980;29192850-29193054;29933449-29933584;30054342-30054522;30071293-30071553;30077289-30077510;36788555-36788815;37453407-37453512;43134712-43134866;47014718-47014820;49793020-49793280;49921802-49921924;51568062-51568207;51825835-51826017;52981271-52981395;53422319-53422461;53422649-53422791;54025812-54026149;60015420-60015540;64314939-64315074;70681866-70681992;72249231-72249343;73636077-73636209;76279069-76279346;79982788-79982944;81064032-81064280;84061235-84061374;91148199-91148442;95355521-95355646;97599730-97600054;99095683-99095796;99096364-99096516;99096713-99096865;99383487-99383747;99629851-99630223;99737372-99737705;100621635-100621800;101315183-101315464;109616321-109616435;110533158-110533302;110781474-110781749;110965644-110965914;111971757-111971961;111972733-111973057;112111066-112111209;112200412-112200534;112200745-112201013;112225448-112225606;112238555-112238873;112255469-112255588 ;112507644-112507788;112517379-112517505;112555972-112556085;112712023-112712137;112817732-112817855;112996304-112996422;113291762-113292022;113409882-113409987;113424098-113424250;113464307-113464413;113544390-113544674;113552480-113552740;113553780-113553891;113585777-113585948;113613573-113613717;113634165-113634459;113648761-113649031;113670884-113671297;113678805-113679064;113689097-113689377;113700520-113700641;113701833-113702105;113708541-113708801;113714316-113714454;113718282-113718430;113719005-113719285;113719915-113720039;113731198-113731511;113735268-113735374;113739025-113739426;113741556-113741663;113742704-113742989;113752386-113752786;113752892-113753008;113795921-113796043;113803178-113803397;113811492-113811630;113991973-113992129;114074346-114074466;114074681-114074839;114107876-114108141;114185609-114185752;114186002-114186117;114187890-114188150;114311777-114312053;114312456-114312743;114579117-114579288;114748219-114748723;114761897-114762157;114766724-114767088;114768170-114768522;114770057-114770194;114774490-114774623;114774937-114775106;114776333-114776480;114782674-114782839;114801076-114801190;114801472-114801632;114802547-114802648;114807120-114807306;114828289-114828436;114846514-114846754;114862690-114863026;114872714-114872887;114875170-114875467;114917465-114917735

Genomic regions of chromosome 14:

19552756-19552882;19888527-19888729;21469383-21469528;22370643-22370777;24546392-24546540;24779925-24780064;24780464-24780825;29228334-29228475;29254667-29254796;35871714-35871957;36987167-36987432;37127671-37127775;38067839-38067989;42075553-42075695;47289472-47289732;47669846-47669972;48095758-48095872;50789276-50789536;53684197-53684562;54860350-54860477;57833188-57833298;58864739-58864887;59065986-59066095;59112362-59112725;59261405-59261517;61103979-61104118;61110728-61110851;65289804-65289908;68038823-68038973;69256594-69256946;70039176-70039871;70316866-70317048;71634121-71634340;71635170-71635289;71720841-71721359;72757827-72757933;72976906-72977078;73396377-73396516;73706424-73706539;77730976-77731087;787097

26-78709906;81790233-81790391;82089218-82089329;82145655-8214587 8;90162180-90162322;90967496-90967644;91880085-91880203;93154317-93154424;931707 10-93170831;94173126-94173261;94306818-94306932;94393652-94393911;94417396-9441 7505;94776104-94776285;95358041-95358143;95942665-95942828;97431262-97431368;996 65186-99665321;101198727-101198831;101235838-101235964;101290704-101290986;1012 93029-101293167;101341254-101341377;101347193-101347429;101498224-101498354;101 506232-101506440;101509249-101509363;101512542-101512896;101515918-101516066;10 1618124-101618439;101967708-101967920;102027719-102028123;102053948-102054054;1 02101574-102101696;102974004-102974264;103294573-103294770;103411329-103411460; 104151982-104152124;104600454-104600634;104622058-104622185;104638075-104638634 ;104717199-104 717317;104741838-104742148;104800629-104800730;105012456-10501263 4;105103000-105103123;105154789-105154975;105156970-105157120;105196085-1051963 45;105196441-105196564;105218119-105218379;105220620-105220774;105247214-105247 320;105282932-105283053;105318278-105318380;105332541-105332644;105538413-10553 8545;105715648-105716016;105741936-105742116;105858433-105858573;105930383-1059 30533;105936287-105936429;105964494-105964647;106236068-106236301;106744195-106 744492

Genomic regions of chromosome 15:
20500097-20500216;21260243-21260383;22954676-22954817;25328405-25328598;2533 1538-25331810;25333245-25333481;25333693-25333819;25429120-25429262;25439336-25 439494;25452087-25452234;25454353-25454613;25468463-25468600;25475546-25475655;2 5961763-25962039;26107480-26107780;27494330-27494511;27786834-27786990;28197404 -28197546;28272391-28272548;28834235-28834341;29210460-29210609;29210940-292110 91;31295015-31295133;31685126-31685238;32933783-32933978;35591628-35591775;40544 352-40544515;41165190-41165335;42371635-42371842;45670999-45671279;46501861-465 01985;53090486-53090611;53095771-53096242;54754511-54754719;55881585-55881853;60 287445-60287663;61517509-61517629;62516463-62516607;63312170-63312301;63671591-63671693;655035 71-65503848;66274691-66274862;67073455-67073597;67418251-6741839 6;68096933-68097080;68549114-68549256;68624774-68624886;70168931-70169146;720951 17-72095298;72490399-72490637;72524585-72524688;74709660-74709781;75019250-7501 9393;75111015-75111157;75136035-75136181;76408348-76408488;76629837-76629951;772 72025-77272146;77320669-77320797;77324526-77324734;77861577-77861688;77928918-7 7929063;77933713-77933918;78196323-78196536;79043911-79044028;79471437-79471561; 81410799-81410916;81426635-81426854;81666392-81666512;86126117-86126407;8919877 6-89198878;89876688-89877101;89954947-89955060;90320166-90320508;90578559-90578 665;91840099-91840252;92706010-92706210;95399900-95400006;96906421-96906807;9864 7804-98647959;99966831-99967113;100672241-100672497;101169888-101170019;1016266 57-101626849;101695660-101695761;102520336-102520448

Genomic regions of chromosome 16:
351770-351955;476939-477199;507982-508180;560102-560280;585815-585961;605144-605282;646387-64651 4;672266-672502;696429-696551;727196-727298;788079-788184;798 264-798440;810594-810696;811028-811141;816004-816228;818329-818501;832991-833213; 874313-874422;874629-874894;883878-883999;891200-891402;893730-894068;894780-895 048;895260-895474;900744-900944;921005-921187;922023-922174;926158-926321;928931-929102;938037-9 38142;941822-942231;945521-945676;959675-959816;960017-960186;963 281-963404;967900-968132;968136-968396;970628-970888;1001256-1001363;1017696-101 7868;1022357-1022462;1024260-1024381;1027310-1027510;1033335-1033485;1034330-103 4448;1040572-1040708;1056983-1057140;1069552-1069816;1071036-1071349;1076847-107 6989;1077614-1077762;1112280-1112395;1116317-1116428;1128048-1128184;1129936-113 0062;1131907-1132125;1138264-1138405;1138697-1138798;1146160-1146337;1151097-115 1288;1158502-1158617;1179659-1179845;1204875-1205026;1207890-1208006;1210774-121 0887;1215465-1215641;1216737-1216876;1217148-1217292;1217827-1217971;1221814-122 2232;1236204-1236522;1244320-1244467;1256258-1256367;1257708-1257906;1261243-126 1399;1262087-1262230;1271879-1271985;1273762-1273911;1275480-1275582;1305586-130 5813;1365801-1365915;1369206-1369335;1394962-1395111;1397728-1397878;1404850-140 5063;1487459-1487703;1493756-1493860;1500343-1500507;1560800-1561275;1570743-157 1003;1585920-1586076;1795200-1795552;1796810-1797449;1813975-1814251;1843625-184 3830;2012706-2012914;2040930-2041062;2140870-2141007;2203175-2203316;2273017-227 3159;2288601-2288702;2338129-2338282;2358275-2358417;3059648-3059836;3493359-349 3504;3706674-3706785;3989432-3989550;4714033-4714152;4730391-4730767;4732926-4733257;6069932-6070116;6533180-6533460;7382335-7382477;7796774-7796925;10935153-10935344;14397504-14397942;14400873-14401025;19126144-19126395;19127248-19127359;21358305-21358443;22326413-22326543;25159910-25160172;27234298-27234558;27853395-27853526;28081140-28081267;28224136-28224249;28331351-28331466;28948278-28948409;29118820-29118983;29151048-29151200;29151523-29151674;29169963-29170230;29222347-29222448;29262792-29263025;29323906-29324009;29340601-29340743;30017955-30018195;30100355-30100511;30124686-30124904;30133122-30133311;31146861-31147128;31483162-31483317;32290058-32290182;33070350-33070866;33509682-33509903;33852490-33852800;34208387-34208655;35027326-35027548;35029898-35030009;46962905-46963079;46963989-46964158;49525631-49525806;49669731-49669842;49686461-49686668;49783345-49783635;50745959-50746088;50873851-50873953;51026677-51026962;51184280-51184462;54318157-54318314;56659375-56659635;56709801-56710036;57669429-57669569;57831641-57831859;57832134-57832309;57836787-57836913;58120934-58121038;58131326-58131428;58191975-58192104;63163378-63163649;65105709-65105857;65157743-65158003;66918914-66919049;66943845-66943952;67183642-67183774;67918699-67918926;68014177-68014303;68269362-68269515;69776038-69776369;70812830-70813090;71571085-71571203;73100458-73100568;75148407-75148550;75263716-75263905;75283549-75283661;75512693-75513169;75550021-75550165;77465154-77465272;79027500-79027745;80654677-80654787;81526928-81527067;81527249-81527524;81533151-81533275;81536133-81536289;81564093-81564528;81565706-81565886;81667103-81667207;82661521-82661623;84417988-84418092;84483628-84483766;84492560-84493006;84722861-84722990;85075042-85075233;85196346-85196615;85209162-85209325;85256212-85256366;85337322-85337537;85363003-85363117;85458281-85458415;85608003-85608311;85650374-85650557;85678088-85678415;85966415-85966519;85969300-85969411;86160817-86160950;86184977-86185116;86528271-86528385;86714536-86714745;86715028-86715325;86772057-86772158;86774040-86774154;86795398-86795511;86911938-86912087;86951582-86951715;87451112-87451214;87641064-87641189;87682628-87682743;87740820-87740930;87864445-87864567;87867921-87868087;87904158-87904547;88052922-88053108;88315446-88315572;88330720-88330954;88371318-88371488;88454810-88454952;88476959-88477074;88480700-88480885;88482183-88482352;88493595-88493702;88504983-88505222;88744579-88744891;88821809-88821926;88832470-88832814;88884969-88885095;88905033-88905257;88905408-88905745;88941486-88942093;88945738-88945937;88946849-88947069;88963090-88963306;88964439-88964944;89000094-89000311;89000900-89001002;89009874-89009998;89019476-89019670;89050730-89050833;89100795-89100933;89115674-89115834;89120615-89120840;89137796-89137959;89141880-89142023;89229268-89229369;89346234-89346386;89387014-89387519;89461615-89461907;89640889-89641060;89688060-89688504;89900088-89900250;89900350-89900765;89972422-89972926;89973141-89973267;90128717-90128834

Genomic regions of chromosome 17:

86514-86774;146378-146508;263405-263591;707114-707265;708439-708583;709220-709449;710617-710890;730169-730313;744589-744849;747395-747596;750777-750960;754213-754329;805425-805647;822721-823014;950471-950649;951755-951962;1029686-1029866;1093963-1094351;1395854-1395960;1474682-1474879;1494318-1494471;1553414-1553592;1634125-1634566;1881105-1881214;1961565-1961701;3716200-3716611;4510577-4510683;4803421-4803764;4979072-4979184;5019385-5019496;7727101-7727219;7754799-7755013;7792052-7792191;7836524-7836640;8110065-8110351;8791859-8791988;9976391-9976504;10212329-10212602;10422077-10422190;14207369-14207512;16849251-16849389;17062034-17062170;17088577-17088684;17110182-17110353;17359835-17359973;17407805-17407909;17713349-17713719;17910196-17910434;18088312-18088578;19220794-19220904;19350000-19350102;19617148-19617305;21731120-21731268;22020651-22020804;22253212-22253324;25335912-25336049;25798733-25799019;25993431-25993537;27044064-27044320;27893094-27893331;28562117-28562218;30348178-30348317;30348728-30348898;30367336-30367458;32366789-32367099;33672833-33672973;34097374-34097546;36997619-36997797;37042200-37042329;37074968-37075238;37331612-37331923;38024208-38024476;39680318-39680724;40274686-40274946;40275266-40275382;40308110-40308252;40318355-40318543;40330818-40330951;40713882-40714255;40715043-40715407;40805862-40805984;40824078-40824379;41847096-41847236;42431865-42432006;42635625-42636182;43198355-43198493;43862893-43863023;44090853-44090999;46138081-46138182;46227996-4622826 1;46607782-46608135;46673394-46673560;46799639-46799745;46804207-46804415;47113489-47113597;48628400-48628516;48653108-48653296;49746979-49747091;55474061-55474183;55533020-55533208;56621341-56621578;59534544-59534683;60729461-60729588;61995743-61995860;62019271-62019377;62066668-62066826;63225002-63225127;65052129-65052231;66375071-66375196;70113454-70113585;70637038-70637333;72345389-72345493;73088154-73088350;73126515-73126665;73696459-73696655;73719830-73720014;73720590-73720840;73739801-73739941;73754448-73754584;73805841-73806157;73914273-73914375;73996169-73996384;74005350-74005578;74133474-74133786;74222881-74223128;74908575-74908882;75385014-75385117;75385329-75385458;75522239-75522375;75558356-75558632;75797020-75797129;76183640-76183779;76417893-76418003;76422911-76423049;76425170-76425309;76425465-76425595;76472296-76472590;76967614-76967826;77030191-77030327;77111616-77111727;77224269-77224403;77393894-77394062;77419496-77419669;77460269-77460399;77680318-77680444;77685550-77685707;77808309-77808503;77817346-77817523;77834120-77834281;77884831-77885163;77924314-77924422;77948976-77949105;78064033-78064293;78090499-78090759;78293360-78293470;78417889-78418088;78444608-78444747;78748439-78748552;78937786-78937961;78939669-78939807;78940033-78940172;78999635-78999909;79017352-79017536;79030900-79031091;79045455-79045664;79096477-79096676;79099777-79100287;79108111-79108257;79228880-79229024;79388434-79388754;79430811-79431136;79432888-79433066;79477840-79478078;79494897-79495544;79495659-79495929;79514682-79514849;79680645-79680803;79827105-79827226;79827601-79827913;79936502-79936625;79957456-79957563;79961397-79961639;79970070-79970310;80022818-80023034;80038280-80038385;80278862-80278971;80290004-80290278;80337468-80337614;80350283-80350408;80351067-80351349;80393104-80393216;80615580-80615693;80621636-80621770;80654550-80654855;80794148-80794322;80798013-80798130;80834070-80834238;80841388-80841593;80845456-80845654;80867522-80867718;81014671-81014811;81042475-81042693;81042957-81043190

Genomic regions of chromosome 18:

2913099-2913359;4692168-4692315;6929695-6929973;7011217-7011517;11147324-11147438;11148502-11148704;12093605-12093787;12254608-12254799;12376166-12376287;12911025-12911263;19997912-19998018;30253077-30253179;32557856-32557978;35142911-35143018;36523511-36523656;36524696-36524815;41901006-41901266;44260325-44260450;44777838-44777945;54814561-54814670;55018649-55018791;55019621-55019881;55102008-55102159;59483326-59483510;61143964-61144293;63673366-63673522;67918103-67918363;68098182-68098309;74062554-74062691;74091419-74091536;74091849-74091962;74173808-74173967;75691288-75691435;75998530-75998790;76601754-76601899;76690786-76690906;76764164-76764328;77181332-77181458;77211083-77211208;77218368-77218521;77233391-77233493;77272517-77272669;77289042-77289214;77313759-77314027;77612132-77612280;77622411-77622671;77623511-77623889;77638029-77638277

Genomic regions of chromosome 19:

407274-407401;426996-427264;440739-441012;641771-641985;643216-643390;736374-736475;751124-751242;812514-812639;863075-863226;929476-929834;939522-939782;944234-944353;1008998-1009312;1009581-1009978;1012076-1012181;1105470-1105989;1112618-1112890;1163467-1163571;1207406-1207645;1220659-1220815;1307442-1307702;1308088-1308193;1409489-1409611;1440259-1440375;1465904-1466033;1496187-1496464;1785443-1785546;1800116-1800276;1881012-1881272;1985881-1986159;1993188-1993317;2038426-2038539;2275664-2275933;2276231-2276566;2291272-2291834;2356433-2356558;2358620-2358743;2430116-2430394;2511569-2511714;2700642-2700988;2717355-2717493;2980113-2980244;3344155-3344281;3481765-3482028;3507795-3507932;3539061-3539203;3669758-3669894;3905467-3905577;3964708-3964989;3984172-3984323;4091049-4091217;4391326-4391534;4552455-4552575;4556028-4556131;4792724-4792836;5210577-5210692;5240143-5240245;5244109-5244234;5250308-5250508;5335108-5335261;5691805-5692212;5822190-5822490;5829147-5829298;6007848-6007957;6534759-6534917;7267899-7268027;7293222-7293352;7505139-7505453;7626084-7626198;7712038-7712372;7926980-7927286;7928046-7928163;8387233-8387343;8398613-8398727;8654344-8654451;8676733-8676862;9370151-9370331;9965432-9965583;10076989-10077270;10254525-10254637;12306037-12306438;12306467-12306581;12758957-12759157;12880553-12880844;12969320-12969630;13110697-13110946;13198813-13198999;13365937-13366152;14359679-14359987;15281126-15281608;15487839-15487991;15564032-15564292;15719695-15719824;16198791-16198996;161 99301-16199472;16528768-16529028;17283537-17283942;17877723-17877846;17941985-17942360;18545021-18545140;18557105-18557380;18572345-18572546;18888694-18888799;18979170-18979504;19648029-19648456;22123507-22123704;22193425-22193710;22682209-22682341;24182604-24182719;30155520-30155714;31770037-31770174;33697975-33698181;34263975-34264112;35715867-35715996;35764609-35764806;35800808-35801031;36024802-36024954;36365483-36365713;36435460-36436056;36499325-36499477;36987989-36988110;38284033-38284143;38307971-38308263;39009920-39010101;39051851-39052224;39217542-39217681;39921424-39921539;40676400-40676563;40711801-40711929;41313752-41313856;41732534-41732669;41836965-41837171;42703790-42703900;42906601-42906740;43912178-43912594;45211154-45211314;46032452-46032732;46056784-46057103;46806880-46807331;48228952-48229220;48244318-48244419;48725593-48725714;49059650-49059790;49116355-49116496;49120443-49120595;49238619-49238762;49517745-49517860;49971643-49971789;49993157-49993268;50027789-50027925;50364420-50364533;50365147-50365360;51021468-51021728;51132585-51132706;51174888-51175001;51535251-51535466;51917729-51917918;52104320-52104624;52216745-52217059;56090047-56090307;56603156-56603394;58549185-58549455;58571667-58571816;58629729-58629901;58958222-58958364
Genomic regions of chromosome 2:
314941-315145;502994-503242;503471-503857;621816-621968;816675-816823;817583-817715;866740-866878;882039-882307;883681-883793;906893-907022;1017930-1018077;1130469-1130642;1151708-1151813;1166225-1166333;1237456-1237607;1291358-1291471;1480763-1480992;1482854-1483028;1497746-1497869;1543374-1543485;1566909-1567411;1792515-1792762;1846718-1846845;1895732-1896006;1941126-1941290;1997887-1998264;2831009-2831267;2875964-2876079;3151358-3151466;3185199-3185469;3452412-3452672;3749733-3749993;3818767-3818868;3834250-3834353;5896451-5896566;8595989-8596120;8714184-8714530;9347890-9348018;9375569-9375768;9533797-9533907;10151636-10151908;10152738-10152914;10153821-10153967;10154227-10154365;10384631-10384732;10567046-10567251;10619227-10619346;11076220-11076327;11750747-11751009;11758654-11758793;11925414-11925532;12880419-12880684;15703422-15703623;16154211-16154334;22059106-22059217;23913308-23913569;23913772-23913932;24397744-24398076;25051006-25051131;25475724-25475928;27529746-27529898;27530769-27531598;27665507-27665639;27958421-27958530;30144168-30144384;31215668-31215914;31359819-31359936;31457607-31457722;33953223-33953352;42319246-42319372;45168776-45169036;45169422-45169774;47419168-47419278;59182247-59182386;59477247-59477383;62116650-62116822;63280428-63280532;63285364-63285519;63286469-63286709;64834105-64834431;64876959-64877091;68251455-68251561;68675722-68675940;71134002-71134122;71205519-71205998;71211845-71212013;71294876-71295004;73161953-73162159;73164590-73164723;73496066-73496180;74347459-74347734;74668353-74668477;76672413-76672525;78182097-78182272;80101281-80101401;83164674-83164776;83212913-83213047;84105399-84105599;86307198-86307394;88124901-88125161;89064507-89064654;96097860-96097961;97174770-97174934;97652205-97652349;99013246-99013410;99439906-99440028;102091153-102091362;105697669-105697796;107459775-107459882;109559228-109559453;109934688-109934960 ;113379619-113379780;114048867-114049009;114325955-114326073;117813669-117813782;118617169-118617429;119591601-119591704;119606694-119606799;120190151-120190368;121116537-121116640;122088551-122088676;124441553-124441710;124445363-124445503;125745049-125745172;126595222-126595338;127801049-127801356;127963474-127963591;128389096-128389237;129131469-129131617;129661444-129661593;130691090-130691215;131520256-131520421;132220155-132220415;132724040-132724161;133426718-133426907;134024317-134024467;135568278-135568379;140167808-140167942;142022623-142022728;147344967-147345072;147345256-147345399;148444157-148444333;148584353-148584816;159726425-159726561;161127322-161127688;162275374-162275596;164204508-164204841;164960000-164960125;165874788-165874926;168572451-168572698;171679000-171679139;172430711-172430816;174890808-174890957;175191840-175192170;175412124-175412755;175595399-175595599;176932629-176932886;176964588-176964835;178972945-178973081;192745999-192746104;192746300-192746434;194367473-194367574;198571482-198571682;200213525-200213630;200331902-200332063;200334900-200335072;202004720-202004884;202737401-202737676;207305207-207305318;216274620-216274747;217674547-217674756;218843925-218844078;218988817-218988956;219128422-219128551;219219509-219219647;219256054-219256164;219735751-219736010;219818548-219818739;21 9857167-219857345;220406607-220406771;223170537-223170797;226608712-226608855;2
28185299-228185452;232546136-232546246;233245980-233246089;233251745-233251882;
233385141-233385262;233757562-233757690;235372806-235372935;236415626-236415844 ;236773979-236
774083;236777077-236777194;237068534-237068794;237964992-23796510
4;238280909-238281017;238289880-238290023;238446575-238446715;238617110-2386172
78;238621262-238621443;239048106-239048361;239048462-239048722;239317900-239318
175;239495105-239495227;239709312-239709428;239865187-239865288;239949254-23994
9449;239951168-239951316;240002094-240002278;240005916-240006019;240029685-2400
29806;240039954-240040096;240100625-240100822;240168211-240168360;240449613-240
449764;240738007-240738359;240748209-240748358;241076094-241076361;241098608-24
1098728;241185018-241185290;241197081-241197297;241389046-241389281;241390051-2
41390223;241391057-241391185;241393303-241393414;241440038-241440250;241568341-241568630;24158
6162-241586450;241613203-241613319;241807708-241807827;241835226
-241835462;241835670-241835799;241851342-241851539;241896876-241897033;24198802
0-241988125;242004860-242005162;242011111-242011216;242212500-242212617;2422948
68-242295000;242509492-242509709;242549382-242549530;242598716-242598840;242683
938-242684066;242754587-242754771;242787213-242787398;242794882-242795200;24281
3983-242814188;242815135-242815331;242833054-242833255;242833458-242833634;2429
39254-242939481;242988004-242988116

Genomic regions of chromosome 20:
824493-824665;3218090-3218216;3766241-3766611;5451914-5452066;6194166-619432
8;13279934-13280297;17557017-17557143;17595272-17595449;18024141-18024285;184896
55-18489766;22559740-22559924;29955962-29956063;30195968-30196163;30777591-3077
7894;30778219-30778329;31041369-31041476;32379831-32380033;34638218-34638423;346
99991-34700251;34995363-34995513;36148604-36148799;36151119-36151269;36153186-3
6153309;37352085-37352229;42788668-42789043;43378708-43378895;43926929-43927189;
44839110-44839279;48598339-48598445;48768685-48768804;57408794-57408913;5758297
1-57583196;58514042-58514316;59965709-59965816;59969316-59969456;60441927-60442
057;60448702-60448813;60501966-60502086;60520205-60520306;60639273-60639496;6095
3364-60953667;60971545-60971715;61041540-61041645;61151636-61152007;61153871-61
153998;61158286-61158387;61314855-61314986;61318364-61318594;61403308-61403478;6
1905169-61905384;61923786-61923900;61939628-61939773;61978048-61978194;61979533
-61979667;61986016-61986161;62002864-62003022;62031707-62031925;62032309-620324
69;62032823-62032984;62037630-62037758;62084647-62084825;62097753-62097994;62111
155-62111423;62126196-62126299;62127292-62127395;62679364-62679725;62729774-627
30203;62813809-62813966;62818658-62818811

Genomic regions of chromosome 21:
10596670-10596813;10597540-10597841;10599393-10599655;10991067-10991339;1099
4948-10995059;28515757-28515863;28516095-28516200;30372612-30372752;30451257-30
451380;33247238-33247417;33805624-33805734;34166477-34166641;37618727-37618869;3
7667907-37668053;37852547-37852666;38066839-38066972;39493180-39493503;39643732
-39643869;43185715-43185832;44106225-44106545;44352528-44352698;44369619-443698
13;44389866-44389987;44473691-44473840;44480585-44480711;44721027-44721167;44821
312-44821560;45582426-45582529;45622342-45622614;45622803-45623168;45664074-456
64211 ;45683165-45683429;45705485-45705980;45709420-45709742;45712762-45713022;45
713606-45713720;45752382-45752598;45881450-45881710;45926462-45926643;46321493-46321761;463482
36-46348364;46410851-46410953;46421181-46421290;46424706-4642494
4;46521035-46521228;46816568-46816698;46850377-46850665;46859124-46859262;469184
09-46918557;46929780-46930079;46935441-46935701;46964194-46964343;46972437-4697
2588;46972744-46972914;46973298-46973462;47286962-47287426;47288359-47288844;472
92177-47292324;47318755-47318961;47322452-47322554;47394303-47394563;47404222-4
7404424;47406763-47406966;47421037-47421389;47422541-47422683;47423533-47423693;
47453296-47453556;47533224-47533354;47545624-47545761;47551773-47552605;4756027
8-47560438;47560678-47560830

Genomic regions of chromosome 22:
16123154-16123315;16228363-16228507;17518180-17518440;17897894-17898235;1826
0220-18260480;18639239-18639444;19165207-19165314;19710954-19711270;19892663-19
892769;19960370-19961052;20133575-20133687;20134349-20134530;20716156-20716416;2 0783863-20783966;20784984-20785169;22007099-22007238;22058008-22058268;23523866 -23524229;23776101-23776234;23794252-23794534;23799716-23799835;23802510-238027 61;24115526-24115685;24224867-24225017;25575338-25575485;25761780-25761992;26148 670-26148940;28007090-28007375;28193815-28194075;28194970-28195230;29445638-294 45746;29706708-29706823;30115202-30115349;30125363-30125561;32014284-32014544;34 046406-34046577;36091773-36091963;37420264-37420524;37493705-37494160;37499342-37499452;374996 07-37499742;37608625-37608846;37678641-37678791;37771204-3777144 3;38484049-38484176;38506545-38506781;39712955-39713087;39770340-39770557;400191 15-40019304;40058029-40058146;40814826-40815387;41185198-41185342;42523474-4252 3617;44708907-44709095;45018513-45018654;45132873-45133362;46508320-46508608;467 70059-46770319;46774141-46774289;46787377-46787562;46859899-46860049;46929628-4 6929829;47513273-47513533;48977330-48977449;49138260-49138539;49141691-49141847; 49295437-49295575;49376409-49376857;49579808-49579999;49717597-49717905;4976187 5-49762158;49766838-49767010;49795411-49795573;49812104-49812350;49843537-49843 801;50158120-50158267;50219681-50219943;50470104-50470482;50479987-50480130;5072 0723-50720864;51043305-51043407;51043672-51043785;51143196-51143299
Genomic regions of chromosome 3:
9969911-9970024;10491154-10491268;12829922-12830031;12856794-12856903;130285 01-13028781;13555611-13555722;13694350-13694484;14853147-14853268;16119166-1611 9269;16925454-16925569;27353058-27353176;27754351-27754494;38175455-38175600;420 56643-42056787;42265624-42265750;48310428-48310599;48677634-48677768;49394621-4 9394731;49394890-49395074;49689805-49690101;49824444-49824559;49947831-49947935; 51746670-51746989;51749763-51749960;52099372-52099549;52478749-52478853;5303302 5-53033341;57543312-57543472;57743413-57743704;62358080-62358370;70908960-70909 102;75263610-75263718;77089354-77089475;77289659-77289795;83266692-83266798;8500 8633-85008780;87424596-87424853;93637424-93637531;96393715-96393862;99594791-99 595300;101406231-101406491;103447185-103447336;109115208-109115441;112706474-11 2706601;112998694-112998819;114647005-114647115;114903881-114904056;120170436-1 20170540;121903514-121903626;125677240-125677349;126679198-126679343;128210766-128211158;12821 1495-128211625;128212214-128212337;128336772-128336889;128998518 -128998630;129866878-129866992;134635235-134635336;138170578-138170802;14086648 7-140866669;149768332-149768452;149768479-149768601;153096365-153096626;1568379 68-156838228;157821357-157821617;159754100-159754204;161214690-161214923;168416 772-168416921;169470001-169470137;169539942-169540210;169540298-169540688;17030 3580-170303706;170626721-170626841;172858838-172859108;180042402-180042574;1814 21427-181421570;183209625-183209915;183952495-183952633;184280643-184280825;190 105485-190105745;193272561-193272870;193857480-193857624;194014564-194014723;19 5489708-195489980;195587085-195587234;195849415-195849751;195869881-195870141;1 96326326-196326457;196522757-196522934;197184326-197184440;197238913-197239159; 197388168-197388327;197401717-197401995
Genomic regions of chromosome 4:
52892-53692;298812-298963;565894-566007;619766-619869;629011-629152;631732-63 1867;660260-660466;681236-681347;686969-687078;786763-786883;793002-793262;79855 5-798662;812387-812500;815269-815389;829265-829501;843214-843380;891883-892011;94 0643-941226;943518-943706;949483-949617;954955-955152;956269-956377;983374-98367 7;1022461-1022588;1042149-1042267;1044639-1044745;1046976-1047115;1138272-113847 1;1138999-1139270;1139864-1139997;1148449-1148570;1188392-1188652;1188981-118915 4;1227449-1227729;1373404-1373684;1379901-1380064;1520095-1520242;1537143-153726 6;1538778-1538956;1542139-1542284;1543535-1543850;1559561-1559711;1562050-156221 1;1562945-1563104;1563588-1563844;1594919-1595074;1600893-1601027;1604405-160479 0;1608351-1608594;1625223-1625615;1638117-1638270;1642316-1642812;1801062-180132 7;1802984-1803161;1803393-1803513;1804705-1805046;1807244-1807998;1808506-180886 3;2062976-2063086;2820399-2820559;2929994-2930260;3043723-3043855;3312792-331290 4;3417481-3418229;3418577-3418845;3468150-3468291;3478232-3478441;3514736-351484 5;3577650-3577769;3681485-3681641;3748441-3748701;3897608-3897872;4133914-413406 0;4228200-4228343;4670855-4670986;4864487-4864600;4867099-4867212;4868385-486860 8;5021655-5021783;5458476-5458806;5972022-5972228;6010010-6010130;6024855-602501 1;6052421-6052534;6955850-6956110;6974087-6974197;7246058-7246187;7374559-737470 1;7395407-7395621;7430863-7430967;7435283-7435464;7485936-7486043;7502841-750295 2;7593377-7593525;7647724-7647930;7665769-7665911;7677795-7677935;7735266-773537 5;7738879-7738994;7769785-7769909;7967226-7967390;7986329-7986435;8024963-802508 4;8070911-8071027;8087108-8087365;8107659-8107871;8108168-8108324;8127171-812734 7;8154059-8154167;8373420-8373580;8374299-8374486;8376721-8377196;8412735-841289 1;8587077-8587184;8602797-8603057;8692749-8692927;9104452-9104724;12224977-12225 120;12461334-12461470;12539817-12539991;12611058-12611321;13536290-13536425;1776 8736-17768911;20180710-20180836;22601392-22601512;25507143-25507403;25677983-25 678180;26065540-26065976;30722129-30722345;34337439-34337587;43084455-43084573;4 3849075-43849207;44449664-44449842;45422038-45422190;45459414-45459554;45569178 -45569319;47427773-47427934;48012655-48012908;48988075-48988361;57178102-571782 44;57458900-57459016;57522762-57522952;63413896-63414008;67725717-67725857;73742 844-73742985;74223403-74223544;77869491-77869608;78077213-78077358;78387614-783 87732;78491885-78492034;78492893-78493008;85403016-85403168;85404070-85404193;87 857485-87857701;91760087-91760258;101726769-101726907;105504080-105504190;11026 7625-110267741;113333019-113333309;116121422-116121527;116600009-116600121;1180 34764-118034912;118083661-118083781;122853739-122853986;124468791-124468926;132 649265-132649525;138654749-138655195;139009765-139010429;139137951-139138211;14 0201132-140201540;140656793-140656957;142526619-142527043;146823923-146824037;1 47561948-147562082;154179298-154179410;163124587-163124852;163660909-163661039; 174429370-174429691;174445142-174445451;184828340-184828474;184908797-184908920 ;188140079-188 140439;188538704-188538807;190731588-190732233

Genomic regions of chromosome 5:

370221-370333;403141-403307;434905-435106;435326-435495;476650-476910;488444-488709;491247-49144 3;497290-497436;497884-498297;499493-499615;500090-500223;504 490-504828;539880-539984;555877-555998;570608-570712;669672-669959;676565-676669; 676942-677170;759353-759788;772459-772576;777580-777771;781019-781133;846757-846 914;851149-851370;859730-859842;962016-962151;962947-963096;980707-980842;101949 4-1019595;1034298-1034490;1051195-1051455;1053987-1054165;1069336-1069475;107375 7-1073996;1075454-1075628;1080180-1080642;1089506-1089655;1097665-1097811;110868 6-1108804;1120411-1120579;1144919-1145077;1154593-1154863;1179133-1179277;121714 5-1217405;1241164-1241348;1243809-1243949;1253658-1253926;1254254-1254729;125694 5-1257205;1266432-1266640;1282811-1283106;1283825-1284063;1284558-1284692;129144 4-1291586;1294067-1294278;1299499-1299649;1460382-1460534;1466228-1466358;149497 6-1495154;1513626-1514220;1548911-1549171;1728964-1729226;1744869-1744986;182805 4-1828171;1836672-1836932;1865373-1865566;1866120-1866464;1922890-1923026;194890 8-1949168;2094934-2095046;2128984-2129119;2137148-2137300;2137569-2137674;228916 0-2289288;2418606-2418866;2658898-2659158;2738445-2738712;2755905-2756178;322583 9-3225962;3285954-3286311;3764218-3764375;4837640-4837769;4865961-4866225;666887 6-6669007;9066642-9066792;9118929-9119249;14459296-14459397;20398668-20398841;27 120803-27120919;28809975-28810091;28810272-28810498;31076623-31076758;32522822-32522953;335025 56-33502889;35508308-35508554;35531828-35531966;37249949-3725009 2;38134910-38135176;41968320-41968580;42894344-42894448;42951511-42951795;432802 63-43280428;50260006-50260205;50262673-50262796;50673089-50673308;50674046-5067 4175;50674745-50674885;50675061-50675270;50678374-50678509;53814624-53814941;548 26510-54826825;60039895-60040165;71475162-71475304;72593842-72594009;74426799-7 4426971;76248710-76248824;78281663-78281964;84125258-84125409;84461326-84461456; 89351908-89352029;97747125-97747229;102087695-102087875;107778016-107778142;111 541377-111541637;112410762-112410874;114335370-114335475;122426703-122426804;12 6210914-126211206;127872000-127872262;129969721-129969860;130899817-130899926;1 32158772-132158986;134364413-134364714;134374532-134374648;134386361-134386497; 135416286-135416613;135692492-135692752;137225296-137225468;137577519-137577796 ;139089776-139 089946;140175775-140176602;140209246-140209798;140215809-14021610 9;140222067-140222587;140229326-140229782;140237294-140237554;140242111-1402424 71;140242621-140242939;140249614-140250359;140256898-140257234;140262975-140263 499;140559126-140559422;140559441-140559818;140595239-140595499;140751473-14075 1609;140769104-140769223;140787768-140787883;140812344-140812479;140821469-1408 21682;140864470-140864835;142940925-142941044;146086086-146086288;149339730-149 339831;149900917-149901065;150052308-150052409;153994606-153994714;154071013-154071134;160593123-160593263;166211722-166211934;168725398-168725501;169935196-169935315;170737929-170738059;170738221-170738463;170742243-170742346;170742603-170742734;170881742-170881877;171094976-171095266;171534695-171534880;171880461-171880593;172110491-172110905;172287915-172288022;172314099-172314250;172332444-172332739;172753613-172753803;173072299-173072442;173930868-173930977;174911383-174911503;175105255-175105356;175793369-175793484;176797875-176798065;176916394-176916502;178004056-178004247;178488076-178488213;179059433-179059542;179448715-179449072;179554390-179554594;179588619-179588897;179597289-179597486;180018437-180018714;180055772-180056044;180486604-180486734

Genomic regions of chromosome 6:

292330-292590;564040-564158;761168-761384;1003609-1003721;1384522-1384642;1608558-1608733;1620909-1621221;2415766-2415898;2870494-2870754;3752748-3752900;4932089-4932233;5999283-5999395;6002471-6002692;6003194-6003447;7051394-7051497;7142058-7142187;7468918-7469042;7992526-7992639;9024342-9024728;10074891-10075499;10381714-10382088;10884036-10884170;12164223-12164329;12718467-12718569;14924982-14925093;16306000-16306212;17016205-17016536;19807335-19807617;19854566-19854713;21664520-21664625;25777056-25777187;26556845-26556950;26757580-26757749;27648725-27648929;27798857-27799039;28437369-28437476;28557290-28557550;28584035-28584289;28785007-28785143;29407727-29407990;29427451-29427610;29577110-29577223;29758526-29758643;29894304-29894628;30080642-30080782;30458135-30458278;30684202-30684406;30698749-30698905;30711035-30711165;31026020-31026216;31089156-31089294;31590571-31590674;31646235-31646378;31688079-31688229;31868846-31868950;32039767-32039890;32121232-32121368;32134604-32134826;32407570-32407715;32552322-32552582;32975865-32976137;33173307-33173501;33995914-33996039;34966649-34966954;35181113-35181423;35466163-35466268;37105288-37105431;37617920-37618178;39271471-39271665;41621104-41621261;42109127-42109267;44695291-44695667;44696001-44696189;45784346-45784613;52288648-52288944;56406084-56406262;62284300-62284530;72113268-72113417;73867587-73867847;74104455-74104607;84055878-84056029;85476839-85476975;94128042-94128342;100894396-100894656;100895575-100895775;100913764-100913908;100917211-100917395;101847642-101847803;103369396-103369618;106582561-106582669;113010622-113011049;114181402-114181620;117869003-117869393;123056029-123056133;131147799-131147923;133036545-133036652;134210666-134210807;137814635-137814803;138200265-138200412;138426124-138426272;149751431-149751540;152957120-152957258;155684409-155684549;157931822-157931933;158404036-158404197;159067622-159067817;159279473-159279753;159290230-159290374;161354447-161354587;162472302-162472467;164092506-164092700;164092861-164093024;164241262-164241551;164340373-164340633;164393360-164393468;164520780-164520881;166825892-166826009;166836754-166836896;166858138-166858257;167490869-167490981;167589273-167589384;167632872-167633016;167792185-167792288;167794149-167794372;168120772-168120891;168467456-168467560;168501905-168502060;168672714-168672876;168677465-168677754;168684722-168684898;168768040-168768163;169238138-169238268;169423372-169423494;169637751-169637862;169717941-169718084;170454151-170454288;170492184-170492500

Genomic regions of chromosome 7:

386698-386958;596151-596304;612603-612753;807815-807943;948962-949230;960114-960605;975034-975156;977023-977137;998967-999165;1039512-1040386;1052734-1052862 ;1054350-1054504;1108007-1108127;1216516-1216648;1254214-1254357;1303419-1303573 ;1325729-1325862;1363532-1363643;1370450-1370612;1428796-1428958;1443617-1443735 ;1560223-1560628;1687827-1687948;1715419-1715520;1746752-1746930;1778672-1778934 ;1865481-1865642;2040728-2040921;2057276-2057403;2185785-2185923;2293292-2293408 ;2349667-2349786;2473417-2473750;2499663-2499782;2561028-2561141;2681352-2681512 ;2700602-2700707;2770756-2771231;2800689-2800825;2801053-2801398;2801900-2802117 ;2802511-2802697;2854465-2854688;2855624-2855731;2859429-2859553;2859576-2859698 ;3849467-3849603;4030594-4030700;4049514-4049729;4050653-4050894;4118483-4118682 ;4184057-4184254;4187641-4187786;4839729-4839880;4850050-4850304;4855828-4855955 ;4856863-4857088;5340275-5340607;5389025-5389305;5400678-5401127;5458718-5458848 ;5521533-5521665;6188810-6188937;6194000-6194260;6438643-6438745;9661286-9661602 ;9765690-9765800;12610787-12610904;16890861-16890974;19184228-19184331;20823895-20824145;27153159-27153314;27183259-27183370;27185135-27185252;27203958-27204179;27206076-27206237;27309236-27309346;29186889-29187107;29229739-29229873;292334 67-29233587;30635761-30635890;32802377-32802508;32802682-32802783;33080496-3308
0616;37531572-37531835;37888044-37888257;38278803-38278904;38468984-38469109;393
93472-39393621;42267257-42267625;43484502-43485093;44153233-44153493;44801600-4
4801703;47576326-47576577;49815383-49815561;50518498-50518648;50633078-50633211;
55134100-55134245;55146393-55146495;55812549-55812684;56949957-56950241;5771525
3-57715525;57929124-57929229;61821631-61821737;61822294-61822423;62574570-62574
830;63020749-63021014;63642401-63642818;63643156-63643585;65509385-65509529;6596
9911-65970038;68000505-68000693;69923828-69923959;70252256-70252421;70502616-70
502736;71202422-71202572;71407188-71407289;71876953-71877084;72813873-72814014;7
3157280-73157390;73245402-73245730;73819738-73819867;73843721-73843828;79368471
-79368717;86974636-86974834;87105172-87105432;88119761-88119870;90894982-908951
01;92238086-92238355;96626903-96627103;96650566-96650732;97912540-97912641;97978
607-97978735;98424288-98424446;99017370-99017475;99517460-99517584;99723100-997
23311;100203033-100203651;100304644-100304922;100638850-100639004;100643545-100
643783;100769873-100769978;100845605-100845856;100882954-100883078;100921843-10
0921974;101839163-101839411;101847656-101847785;101848202-101848356;102098181-1
02098322;104445585-104445689;107592541-107592723;108108891-108109021;111381499-111381600;11371
7270-113717419;114561714-114562037;120967900-120968043;127991181
-127991655;128494130-128494305;128797855-128797964;130127586-130127846;13013012
2-130130384;131477409-131477517;131831439-131831581;134233790-134233937;1349183
82-134918642;134931024-134931636;134932449-134932808;138440463-138440653;139167
426-139167533;139256205-139256604;145057765-145057874;146731529-146731630;14876
8321-148768581;149318385-149318558;149410858-149410996;149411103-149411395;1494
16720-149416880;150021487-150021599;150074779-150074900;150711033-150711221;150
715107-150715221;150778880-150779089;150811280-150811444;150820337-150820597;15
0871185-150871461;150871479-150872017;151077325-151077428;151145267-151145596;1
51300423-151300705;151393406-151393666;151433279-151433395;151546368-151546484;
151553416-151553551;153108577-153108678;153446852-153446954;154667753-154667907 ;155006030-155
006290;155010884-155011005;155191150-155191256;155325649-15532600
5;155674685-155675066;155996484-155996598;156733725-156734217;156734411-1567346
71;156796837-156797243;156798175-156798342;156804311-156804448;156810954-156811
214;157095169-157095284;157195506-157195725;157207071-157207348;157219644-15721
9796;157233133-157233247;157333722-157333853;157335384-157335700;157347662-1573
47854;157355247-157355522;157408049-157408189;157413991-157414251;157441452-157
441590;157448993-157449114;157474919-157475065;157483305-157483509;157543646-15
7543806;157601991-157602288;157678926-157679078;157694287-157694547;157849665-1
57849811;157860362-157860560;158107343-158107491;158107804-158108211;158109335-158109483;15813
3812-158133997;158277603-158277817;158349791-158349971
Genomic regions of chromosome 8:
325033-325176;655191-655314;668025-668285;748280-748404;865864-865972;914830-914945;982153-98228
0;1112787-1112903;1114396-1114539;1132826-1133109;1134434-113
4574;1140668-1140936;1200860-1201016;1263493-1263704;1276872-1277004;1385178-138
5371;1472152-1472285;1553170-1553377;1553585-1553699;1639908-1640017;1779776-177
9917;1821298-1821488;1829774-1830034;1911528-1911635;1960222-1960370;2003769-200
3919;2014314-2014500;2041819-2041946;2143616-2143777;2196631-2196817;3316846-331
6986;3855558-3855687;6420228-6420539;6637732-6637976;6930518-6930648;8639032-863
9184;8639785-8640185;10192033-10192615;10339377-10339517;10459836-10459961;10589
192-10589310;10755723-10755878;11302092-11302294;16459664-16460187;17767417-177
67677;21970244-21970385;22034616-22034894;22844224-22844507;22928561-22928662;23
423848-23424025;23559923-23560087;23564119-23564592;24918754-24918890;27327223-27327527;334232
27-33423336;35979074-35979247;37655351-37655503;37688979-3768909
2;37699311-37699479;37743949-37744084;38411582-38411683;52183857-52184049;538522
10-53852328;55533664-55533924;56363638-56363809;57350958-57351068;57361036-5736
1318;58114805-58114915;58120340-58120489;58122875-58123032;58123194-58123375;581
24249-58124374;58125408-58125615;58129539-58129651;61326287-61326429;62823811-6
2823937;65488536-65488647;72917233-72917352;73848457-73848579;74197864-74198042;
76576769-76576918;76951131-76951259;76971864-76971967;77108661-77108787;7735975
1-77359908;81523895-81524062;89340637-89340788;89688296-89688432;90624033-90624 163;95962213-95962411;96085465-96085584;97340180-97340319;99961365-99961623;1022 02802-102203072;115323639-115323782;116048978-116049122;116170821-116170926;117 962271-117962510;119964253-119964486;124730010-124730213;126709638-126709751;12 8930104-128930240;128950431-128950534;130641180-130641350;132752295-132752443;1 33141655-133141779;135490693-135490815;138135392-138135499;139096462-139096585; 142185359-142185494;142233347-142233574;142238956-142239142;142302106-142302250 ;142310897-142 311126;142350518-142350647;142437818-142437936;142981762-14298186 9;143129202-143129350;143150270-143150420;143182559-143182689;143203588-1432038 66;143207541-143207779;143214216-143214507;143237807-143237910;143254264-143254 468;143356856-143357002;143378932-143379065;143405741-143405872;143407806-14340 8066;143472969-143473155;143546760-143546877;143560731-143561040;143582378-1435 82484;143609379-143609638;143614730-143614910;143620631-143620753;143623289-143 623631;143625581-143626108;143645492-143645722;143781627-143781955;143781960-14 3782226;143879967-143880112;144140196-144140301;144163515-144163721;144238672-1 44238779;144262756-144263033;144296873-144297020;144343806-144343929;144358407-144358582;14436 1213-144361341;144680983-144681132;144801025-144801264;144815220 -144815359;144820742-144820893;144965127-144965255;144990294-144990407;14500279 3-145002935;145011866-145012151;145045850-145045956;145048324-145048432;1450515 28-145051937;145107745-145107846;145111331-145111500;145164790-145164936;145551 082-145551217;145747723-145747832;145749775-145750137;146232962-146233084
Genomic regions of chromosome 9:
4297532-4297663;4298373-4298504;4662863-4662987;6328354-6328646;8835157-8835 270;11197185-11197346;19102622-19102747;22433833-22434003;33386385-33386526;3445 7129-34457379;34623938-34624080;43134591-43134844;66837800-66837982;67340024-67 340186;72046230-72046367;72131425-72131587;78977770-78977894;84228185-84228387;8 7903840-87904107;94860379-94860687;97349536-97349722;97807640-97807900;97865073 -97865232;98477893-98478005;99463426-99463572;100614849-100614956;106761967-106 762082;106964064-106964360;107091714-107091823;107125513-107125642;109263886-10 9264032;111617732-111617992;112296520-112296746;114090182-114090286;116663391-1 16663558;116930110-116930382;122631840-122631944;124499750-124499937;126154320-126154556;12677 6221-126776323;127071526-127071681;127600928-127601523;127623377 -127623523;129100371-129100602;130700840-130701068;131156805-131156923;13201626 4-132016381;132253313-132253506;132481611-132481764;133767796-133767925;1338135 18-133813707;135374804-135374905;136501744-136501893;137111294-137111435;137620 170-137620706;137677929-137678047;137996213-137996370;138135144-138135249;13815 6801-138156937;138193645-138193784;138330182-138330329;138393583-138393685;1384 08073-138408187;138441301-138441433;138587706-138587829;138591456-138591568;138 660867-138661107;138666312-138666525;138670628-138670837;138672837-138673181;13 8674560-138674856;138680030-138680285;138836905-138837056;138838026-138838170;1 38839522-138840012;139048246-139048499;139143521-139143695;139146527-139146662; 139250789-139250973;139515702-139515832;139565084-139565239;139734956-139735106 ;139840431-139 840679;139907612-139907775;140126911-140127171;140136997-14013713 6;140174189-140174312;140217992-140218215;140329560-140329670;140419847-1404199 83;140501304-140501413;140777297-140777579;140795348-140795503;140944063-140944 202;141007494-141007680;141042575-141042717
Genomic regions of chromosome X:
7896030-7896163;22049899-22050181;22290873-22290977;27999541-27999771;30233 607-30233727;31089585-31089704;37351147-37351407;39951483-39951665;40439815-4044 0129;41334218-41334386;48367092-48367580;55115535-55115681;56057985-56058125;632 64016-63264129;64627133-64627568;66766042-66766302;70128510-70128671;73619264-7 3619532;79082990-79083123;84634307-84634451;99663831-99664091;101397267-1013975 79;114425893-114426012;124824739-124824842;129039937-129040067;131543695-131543 825;133306625-133306958;135229594-135230119;135962416-135962568;141291476-14129 1746;142840351-142840728;147582015-147582583;150151693-150152077;152907700-1529 08275;152909455-152909751;153033217-153033347;153695647-153695785;154522076-154 522200;154624596-154624745

**Claims**

1. Use of a reagent for detecting at least one specific DNA methylation modification or at least one specific DNA methylation haplotype carried in a specific human genomic region in a sample to be tested in the preparation of a reagent or kit for detecting, monitoring, or predicting whether a subject has a placenta-derived gestational disorder, wherein the specific DNA methylation modification or the specific DNA methylation haplotype is derived from methylation difference regions of a placenta of a pregnant woman in normal pregnancy and a placenta of a pregnant woman with the placenta-derived gestational disorder.

2. The use according to claim 1, wherein the sample to be tested is derived from the subj ect;

    the methylation difference region is a genomic region listed in Attached List 1;
    the specific DNA methylation modification is a methylation modification at a specific CpG locus in a genomic region listed in Attached List 1;
    the specific methylation haplotype is a DNA methylation haplotype covered by the genomic region listed in Attached List 1; and
    the specific DNA methylation modification or the specific methylation haplotype is capable of distinguishing the placenta of the pregnant woman in normal pregnancy from the placenta of the pregnant woman with the placenta-derived gestational disorder.

3. The use according to claim 1 or 2, wherein the sample to be tested is derived from the subj ect;
    the specific DNA methylation modification or the specific DNA methylation haplotype detected is located in one or more regions selected from the following regions (i) to (iii):

    (i) a region of LTR12 transposon family; preferably, the LTR12 transposon is an LTR12C transposon or an LTR12E transposon;
    (ii) an FLT1 gene and a regulatory region thereof; preferably, the FLT1 gene and the regulatory region thereof are a region between human chromosome chr13: 28800000 bp and 302000000 bp; and
    (iii) an LIFR gene and a regulatory region thereof; preferably, the LIFR gene and the regulatory region thereof are a region between human chromosome chr5: 38370000 bp and 38840000 bp.

4. The use according to any one of claims 1 to 3, wherein the sample to be tested is selected from samples derived from blood, urine, feces, saliva, oral swabs, cervical secretions, cervical smears, amniocentesis, fetal villi, or fetal circulating cells; preferably, the blood is peripheral blood, and more preferably, the peripheral blood is plasma.

5. The use according to any one of claims 1 to 4, wherein the placenta-derived gestational disorder is selected from one or more of gestational diabetes, twin-to-twin transfusion syndrome, fetal growth restriction, gestational hypertension, preeclampsia, severe preeclampsia, secondary preeclampsia, atypical preeclampsia, and HELLP syndrome; preferably, the gestational disorder is selected from one or more of preeclampsia, severe preeclampsia, secondary preeclampsia, and atypical preeclampsia.

6. The use according to any one of claims 1 to 5, wherein the reagent for detecting the methylation difference regions derived from the placenta of the pregnant woman in normal pregnancy and the placenta of the pregnant woman with the placenta-derived gestational disorder is selected from a DNA methylation status indicator; or

    a reagent for detecting presence of a DNA methylation modification in a sample from the subj ect;
    preferably, the reagent for detecting the presence of the DNA methylation modification in the sample from the subject is a reagent required for enriching characteristic DNA.

7. The use according to claim 6, wherein the DNA methylation status indicator is selected from an antibody or binding protein identifying methylated DNA, bisulfite, enzymes with DNA catalytic oxidation, enzymes with DNA deamination, or a methylation-sensitive enzyme, or a combination thereof; preferably, the methylation-sensitive enzyme is selected from a methylation sensitive restriction endonuclease, and more preferably, the methylation sensitive restriction endonuclease is selected from HpaII or BstUI, or a combination thereof.

8. The use according to claim 6, wherein the reagent required for enriching the characteristic DNA is selected from a reagent required by a hybrid capture method, a reagent required by a polymerase chain reaction amplification method, a reagent required by an anchored nucleic acid amplification method, a reagent for sequencing by synthesis, or a reagent for single-molecule sequencing, or a combination thereof.

9. The use according to claim 8, wherein the reagent required for enriching the characteristic DNA is a probe or probe set; preferably, the probe or probe set is an EP-007 probe or probe set.

10. The use according to any one of claims 1 to 9, wherein the detection is prenatal detection; preferably, the prenatal detection is non-invasive prenatal detection; and more preferably, the prenatal detection is non-invasive prenatal detection in early pregnancy.

11. The use according to any one of claims 1 to 10, wherein the DNA is gDNA or cfDNA.

12. A kit for detecting at least one specific DNA methylation modification or at least one specific DNA methylation haplotype in a sample to be tested to detect, monitor, or predict whether a subject has a placenta-derived gestational disorder, the kit comprising:

   (a) a reagent for detecting presence of a DNA methylation modification;
   optionally, the kit further comprising:
   (b) an indicator for detecting a DNA methylation status;
   wherein the specific DNA methylation modification or the specific DNA methylation haplotype is derived from methylation difference regions of a placenta of a pregnant woman in normal pregnancy and a placenta of a pregnant woman with the placenta-derived gestational disorder.

13. The kit according to claim 12, wherein the methylation difference region is a genomic region listed in Attached List 1;

   the specific DNA methylation modification is a methylation modification at a specific CpG locus in a genomic region listed in Attached List 1;
   the specific methylation haplotype is a DNA methylation haplotype covered by the genomic region listed in Attached List 1; and
   the specific DNA methylation modification or the specific methylation haplotype is capable of distinguishing the placenta of the pregnant woman in normal pregnancy from the placenta of the pregnant woman with the placenta-derived gestational disorder.

14. The kit according to claim 12 or 13, wherein the reagent for detecting the presence of the DNA methylation modification is a reagent required for enriching a characteristic DNA; preferably, the reagent required for enriching the characteristic DNA is a probe or probe set; and more preferably, the probe or probe set is an EP-007 probe or probe set.

15. The kit according to any one of claims 12 to 14, wherein the DNA methylation status indicator is selected from an antibody or binding protein identifying methylated DNA, bisulfite, enzymes with DNA catalytic oxidation, enzymes with DNA deamination, or a methylation-sensitive enzyme, or a combination thereof; preferably, the methylation-sensitive enzyme is selected from a methylation sensitive restriction endonuclease, and more preferably, the methylation sensitive restriction endonuclease is selected from HpaII or BstUI, or a combination thereof.

16. The kit according to any one of claims 12 to 15, wherein the sample to be tested is selected from samples derived from blood, urine, feces, saliva, oral swabs, cervical secretions, cervical smears, amniocentesis, fetal villi, or fetal circulating cells; preferably, the blood is peripheral blood, and more preferably, the peripheral blood is plasma.

17. The kit according to any one of claims 12 to 16, wherein the detection is prenatal detection; preferably, the prenatal detection is non-invasive prenatal detection; and more preferably, the prenatal detection is non-invasive prenatal detection in early pregnancy.

18. The kit according to any one of claims 12 to 17, wherein the DNA is gDNA or cfDNA.

19. A polynucleotide capable of:

   (i) hybridizing to a polynucleotide indicated by an EP-007 probe or probe set under high-stringency hybridization conditions or under very high-stringency hybridization conditions; or
   (ii) having a sequence reversely complementary to the sequence of the polynucleotide as shown in (i).

wherein the polynucleotide is used for detecting at least one specific DNA methylation modification or at least one specific DNA methylation haplotype in a sample.

20. The polynucleotide according to claim 19, which has a sequence having at least 90%, optionally at least 95%, preferably at least 97%, more preferably at least 98%, or most preferably at least 99% sequence identity to a nucleotide sequence indicated by the EP-007 probe or probe set or a sequence reversely complementary thereto.

21. The polynucleotide according to claim 19 or 20, wherein the sequence of the polynucleotide comprises the nucleotide sequence indicated by the EP-007 probe or probe set; preferably, the sequence of the polynucleotide is the nucleotide sequence indicated by the EP-007 probe or probe set.

22. The polynucleotide according to claim 19 or 20, wherein the polynucleotide is a DNA probe.

23. A method for detecting whether at least one DNA fragment with a characteristic of a placenta-derived gestational disorder in a specific human genomic region is present in a sample to be tested, the method comprising a detecting step for detecting:

(a) whether at least one methylation modification at a specific CpG locus with a characteristic of the placenta-derived gestational disorder or an abundance of the modification thereof is present in the sample to be tested; or
(b) whether at least one DNA methylation haplotype with a characteristic of the placenta-derived gestational disorder or an abundance of the DNA methylation haplotype is present in the sample to be tested,

wherein the methylation modification at the specific CpG locus or the DNA methylation haplotype with the characteristic of the placenta-derived gestational disorder is derived from methylation difference regions of a placenta of a pregnant woman in normal pregnancy and a placenta of a pregnant woman with the placenta-derived gestational disorder.

24. The method according to claim 23, wherein the methylation modification at the specific CpG locus with the characteristic of the placenta-derived gestational disorder takes place at a locus covered by a genomic region listed in Attached List 1; and
the DNA methylation haplotype is a DNA methylation haplotype covered by a genomic region listed in Attached List 1.

25. The method according to claim 23 or 24, wherein the specific DNA methylation modification or the specific DNA methylation haplotype detected is located in one or more regions selected from the following regions (i) to (iii):

(i) a region of LTR12 transposon family; preferably, the LTR12 transposon is an LTR12C transposon or an LTR12E transposon;
(ii) an FLT1 gene and a regulatory region thereof; preferably, the FLT1 gene and the regulatory region thereof are a region between human chromosome chr13: 28800000 bp and 302000000 bp; and
(iii) an LIFR gene and a regulatory region thereof; preferably, the LIFR gene and the regulatory region thereof are a region between human chromosome chr5: 38370000 bp and 38840000 bp.

26. The method according to any one of claims 23 to 25, which detects said (a) or (b) by a reagent for detecting presence of a DNA methylation modification, wherein the reagent for detecting the presence of the DNA methylation modification is a reagent required for enriching a characteristic DNA; preferably, the reagent required for enriching the characteristic DNA is a probe or probe set; and more preferably, the probe or probe set is an EP-007 probe or probe set.

27. The method according to any one of claims 23 to 26, wherein the method further comprises a step of extracting DNA from the sample to be tested, and the sample to be tested is a peripheral blood sample; and preferably, the peripheral blood sample is a plasma sample.

28. The method according to any one of claims 23 to 27, wherein the DNA is gDNA or cfDNA.

29. A method for detecting, monitoring, or predicting whether a subject has a placenta-derived gestational disorder, the method comprising the following steps:

(1) a detecting step of:

(a1) detecting whether at least one methylation modification at a specific CpG locus with a characteristic of the placenta-derived gestational disorder or an abundance of the modification thereof is present in the sample to be tested, wherein the DNA methylation modification to the characteristic of the placenta-derived gestational disorder is derived from methylation difference regions of a placenta of a pregnant woman in normal pregnancy and a placenta of a pregnant woman with the placenta-derived gestational disorder; or
(b1) detecting whether a DNA methylation haplotype with a characteristic of the placenta-derived gestational disorder or an abundance of the DNA methylation haplotype is present in the sample to be tested, wherein the DNA methylation haplotype is derived from methylation difference regions of a placenta of a pregnant woman in normal pregnancy and a placenta of a pregnant woman with the placenta-derived gestational disorder;

(2) a comparing step of:

(a2) comparing a detection result of the methylation modification at the specific CpG locus in the sample to be tested or of the abundance of modification thereof with a detection result of a methylation modification at a specific CpG locus in a placental sample with or without a placenta-derived gestational disorder or of an abundance of modification thereof; or
(b2) comparing a detection result of the DNA methylation haplotype in the sample to be tested or of the abundance of the DNA methylation haplotype with a detection result of a DNA methylation haplotype in a placental sample with or without a placenta-derived gestational disorder or of an abundance of the DNA methylation haplotype;

(3) a determining step of:

(i) determining that the subject has the placenta-derived gestational disorder if in (a2) or (b2), a similarity between the detection result of the sample to be tested and the detection result of the placental sample without the placenta-derived gestational disorder is lower or a statistic value of the similarity is significantly lower than expected, or a similarity between the detection result of the sample to be tested and the detection result of the placental sample with the placenta-derived gestational disorder is higher or a statistic value of the similarity is significantly higher than expected;
(ii) determining that the subject does not have the placenta-derived gestational disorder if in (a2) or (b2), a similarity between the detection result of the sample to be tested and the detection result of the placental sample without the placenta-derived gestational disorder is higher or a statistic value of the similarity is significantly higher than expected, or a similarity between the detection result of the sample to be tested and the detection result of the placental sample with the placenta-derived gestational disorder is lower or a statistic value of the similarity is significantly lower than expected; wherein the sample to be tested is derived from the subject.

30. The method according to claim 29, wherein the methylation modification at the specific CpG locus with the characteristic of the placenta-derived gestational disorder takes place at a locus covered by a genomic region listed in Attached List 1; and the DNA methylation haplotype is a DNA methylation haplotype covered by a genomic region listed in Attached List 1.

31. The method according to claim 29 or 30, wherein the methylation modification at the specific CpG locus or specific DNA methylation haplotype detected is located in one or more regions selected from the following regions (i) to (iii):

(i) a region of LTR12 transposon family; preferably, the LTR12 transposon is an LTR12C transposon or an LTR12E transposon;
(ii) an FLT1 gene and a regulatory region thereof; preferably, the FLT1 gene and the regulatory region thereof are a region between human chromosome chr13: 28800000 bp and 302000000 bp; and
(iii) an LIFR gene and a regulatory region thereof; preferably, the LIFR gene and the regulatory region thereof are a region between human chromosome chr5: 38370000 bp and 38840000 bp.

32. The method according to any one of claims 29 to 31, wherein the placenta-derived gestational disorder is selected from one or more of gestational diabetes, twin-to-twin transfusion syndrome, fetal growth restriction, gestational hypertension, preeclampsia, severe preeclampsia, secondary preeclampsia, atypical preeclampsia, and HELLP syndrome; preferably, the gestational disorder is selected from one or more of preeclampsia, severe preeclampsia, secondary preeclampsia, and atypical preeclampsia.

33. The method according to any one of claims 29 to 32, wherein a methylated region specific to the placenta-derived gestational disorder includes a hyper-methylated region and a hypo-methylated region.

34. The method according to any one of claims 29 to 33, wherein the method for determining similarity statistics adopted in step (3) is selected from: correlation, t test, Z test, hypergeometric test, Fourier analysis, Wavelet analysis, Principal Component Analysis (PCA), t-Distributed Stochastic Neighbor Embedding (tSNE), Non-Negative Matrix Factorization (NMF), Support Vector Machine (SVM), k-Nearest Neighbor (KNN), k-means, Linear Model (LM), Generalized Linear Model (GLM), Gaussian Mixed Model (GMM), Neural Networks (NN), Random Forest (RF), Autoencoder, Deep Neural Networks (DNN) and variants thereof.

35. The method according to any one of claims 29 to 34, wherein a reagent for detecting presence of a DNA methylation modification is used to detect the methylation modification at the specific CpG locus or the abundance of modification thereof; or to detect the specific DNA methylation haplotype or the abundance of the DNA methylation haplotype.

36. The method according to claim 35, in which the detection is performed by a reagent for detecting presence of a DNA methylation modification, wherein the reagent for detecting the presence of the DNA methylation modification is a reagent required for enriching a characteristic DNA; preferably, the reagent required for enriching the characteristic DNA is a probe or probe set; and more preferably, the probe or probe set is an EP-007 probe or probe set.

37. The method according to any one of claims 29 to 36, wherein the method further comprises a step of extracting DNA from the sample to be tested, and the sample to be tested is a peripheral blood sample; and preferably, the peripheral blood sample is a plasma sample.

38. The method according to claim 37, wherein the DNA is gDNA or cfDNA.

39. The method according to any one of claims 29 to 38, wherein the method further comprises a step of enriching the DNA or sequencing the DNA.

40. A device for detecting, monitoring, or predicting whether a subject has a placenta-derived gestational disorder, comprising:

   a processor; and
   a memory configured to store processor-executable instructions,
   wherein the process is configured to, when executing the processor-executable instructions, implement the method according to any one of claims 23 to 39.

41. A non-volatile computer readable storage medium having computer program instructions stored thereon, wherein the computer program instructions, when executed by a processor, implement the method according to any one of claims 23 to 39.

42. A system for detecting at least one specific methylation modification at a specific CpG locus or at least one DNA methylation haplotype in a sample to be tested, the system comprising a detecting module configured to detect:

   (a) whether at least one methylation modification at a specific CpG locus with a characteristic of the placenta-derived gestational disorder or an abundance of the modification thereof is present in the sample to be tested; or
   (b) whether at least one DNA methylation haplotype with a characteristic of the placenta-derived gestational disorder or an abundance of the DNA methylation haplotype is present in the sample to be tested, wherein the methylation modification at the specific CpG locus or the DNA methylation haplotype with the characteristic of the placenta-derived gestational disorder is derived from methylation difference regions of a placenta of a pregnant woman in normal pregnancy and a placenta of a pregnant woman with the placenta-derived gestational disorder.

43. The system according to claim 42, wherein the methylation modification at the specific CpG locus with the characteristic of the placenta-derived gestational disorder takes place at a locus covered by a genomic region listed in Attached List 1; and the DNA methylation haplotype is a DNA methylation haplotype covered by a genomic region listed in Attached List 1.

44. The system according to claim 42 or 43, wherein the specific DNA methylation modification or the specific DNA methylation haplotype detected is located in one or more regions selected from the following regions (i) to (iii):

(i) a region of LTR12 transposon family; preferably, the LTR12 transposon is an LTR12C transposon or an LTR12E transposon;
(ii) an FLT1 gene and a regulatory region thereof; preferably, the FLT1 gene and the regulatory region thereof are a region between human chromosome chr13: 28800000 bp and 302000000 bp; and
(iii) an LIFR gene and a regulatory region thereof; preferably, the LIFR gene and the regulatory region thereof are a region between human chromosome chr5: 38370000 bp and 38840000 bp.

**45.** A system for detecting, monitoring, or predicting whether a subject has a placenta-derived gestational disorder, the system comprising the following modules:

(1) a detecting module configured to detect:

(a1) whether at least one methylation modification at a specific CpG locus with a characteristic of the placenta-derived gestational disorder or an abundance of the modification thereof is present in the sample to be tested, wherein the DNA methylation modification to the characteristic of the placenta-derived gestational disorder is derived from methylation difference regions of a placenta of a pregnant woman in normal pregnancy and a placenta of a pregnant woman with the placenta-derived gestational disorder; or
(b1) whether a DNA methylation haplotype with a characteristic of the placenta-derived gestational disorder or an abundance of the DNA methylation haplotype is present in the sample to be tested, wherein the DNA methylation haplotype is derived from methylation difference regions of a placenta of a pregnant woman in normal pregnancy and a placenta of a pregnant woman with the placenta-derived gestational disorder;

(2) a comparing module configured to compare:

(a2) a detection result of the methylation modification at the specific CpG locus in the sample to be tested or of the abundance of modification thereof with a detection result of a methylation modification at a specific CpG locus in a placental sample with or without a placenta-derived gestational disorder or of an abundance of modification thereof; or
(b2) a detection result of the DNA methylation haplotype in the sample to be tested or of the abundance of the DNA methylation haplotype with a detection result of a DNA methylation haplotype in a placental sample with or without a placenta-derived gestational disorder or of an abundance of the DNA methylation haplotype;

(3) a determining module configured to determine whether the subject has the placenta-derived gestational disorder:

(i) the subject has the placenta-derived gestational disorder if in (a2) or (b2), a similarity between the detection result of the sample to be tested and the detection result of the placental sample without the placenta-derived gestational disorder is lower or a statistic value of the similarity is significantly lower than expected, or a similarity between the detection result of the sample to be tested and the detection result of the placental sample with the placenta-derived gestational disorder is higher or a statistic value of the similarity is significantly higher than expected;
(ii) the subject does not have the placenta-derived gestational disorder if in (a2) or (b2), a similarity between the detection result of the sample to be tested and the detection result of the placental sample without the placenta-derived gestational disorder is higher or a statistic value of the similarity is significantly higher than expected, or a similarity between the detection result of the sample to be tested and the detection result of the placental sample with the placenta-derived gestational disorder is lower or a statistic value of the similarity is significantly lower than expected.

**46.** The system according to claim 45, wherein the methylation modification at the specific CpG locus with the characteristic of the placenta-derived gestational disorder takes place at a locus covered by a genomic region listed in Attached List 1; and the DNA methylation haplotype is a DNA methylation haplotype covered by a genomic region listed in Attached List 1.

**47.** The system according to claim 45 or 46, wherein the methylation modification at the specific CpG locus or specific DNA methylation haplotype detected is located in one or more regions selected from the following regions (i) to (iii):

(i) a region of LTR12 transposon family; preferably, the LTR12 transposon is an LTR12C transposon or an LTR12E transposon;

(ii) an FLT1 gene and a regulatory region thereof; preferably, the FLT1 gene and the regulatory region thereof are a region between human chromosome chr13: 28800000 bp and 302000000 bp; and

(iii) an LIFR gene and a regulatory region thereof; preferably, the LIFR gene and the regulatory region thereof are a region between human chromosome chr5: 38370000 bp and 38840000 bp.

48. The method according to any one of claims 23 to 39, wherein the method comprises high-throughput sequencing.

49. The method according to any one of claims 23 to 39, wherein the method comprises qPCR or digital PCR.

50. The method according to any one of claims 23 to 39, wherein the method comprises methylation-specific PCR.

51. The method according to any one of claims 23 to 39, wherein the method comprises methylation-sensitive enzyme digestion.

FIG. 1

Fragment size distribution of cfDNAs

FIG. 2

**a**

Fragment size distribution of
cfDNA methylation pre-libraries

**b**

Fragment size distribution of
gDNA methylation pre-libraries

FIG. 3

**a**

Fragment size distribution of
cfDNA pre-libraries captured with
SeqCap Epi CpGiant probe

**b**

Fragment size distribution of
gDNA pre-libraries captured
with SeqCap Epi CpGiant probe

FIG. 4

**a**

Mean methylation level in control placenta

**b**

Mean methylation level in control placenta

FIG. 5

Distribution of PE-DMRs on Genome

**Legend:**
- PE hyper-methylated regions
- PE demethylated regions
- \* Frequency of enriching demethylated regions being higher than random level

FIG. 6

Overlapping ratio between PE-DMRs and LTR family

LTR12E
LTR12C
HERVE–int
LTR13A
GSAT
LTR12D
LTR6B
HERVS71–int
SATR1

Preeclampsia mean methylation level/control mean methylation level (log) over DMRs

FIG. 7

a

Genomic
methylation in Pan
troglodytes sperms

Genomic
methylation in Homo
sapiens sperms

b

ATAC for
preeclamptic
placenta

ATAC for
control placenta

c

Preeclampsia mean
methylation
level/control mean
methylation level
(log)

Location of genome

FIG. 8

LTR12C

SOX14

2-Cell

4-Cell

8-Cell

Inner cell
mass

Embryonic
stem cell

Placenta

Mean methylation level in preeclamptic
placenta - Mean methylation level in
placenta in control pregnancy

FIG. 9

Reduced methylation level of placental
de novo methylation region in preeclamptic placenta

FIG. 10

FIG. 11

FIG. 12

FIG. 13

FIG. 14

FIG. 15

**a**

**b**

FIG. 16

FIG. 17

FIG. 18

FIG. 19

FIG. 20

FIG. 21

FIG. 22

FIG. 23

FIG. 24

## INTERNATIONAL SEARCH REPORT

| International application No. |
| --- |
| **PCT/CN2020/120654** |

| **A.** | **CLASSIFICATION OF SUBJECT MATTER** |
| --- | --- |

C12Q 1/68(2018.01)i

According to International Patent Classification (IPC) or to both national classification and IPC

| **B.** | **FIELDS SEARCHED** |
| --- | --- |

Minimum documentation searched (classification system followed by classification symbols)

    C12Q

Documentation searched other than minimum documentation to the extent that such documents are included in the fields searched

Electronic data base consulted during the international search (name of data base and, where practicable, search terms used)

    CNABS, DWPI, SIPOABS, CNTXT, WOTXT, EPTXT, USTXT, CNKI, 万方数据资源系统, PubMed, ISI Web of Knowledge, 百度学术: applicant/inventor, 胎盘, placenta, 甲基化, methylation, 妊娠, pregnancy, 子痫, 子痫前期, eclampsia, pre-eclampsia, 妊娠糖尿病, 妊娠高血压, CpG, LTR12, FLT1, LIFR, 无细胞DNA, cfDNA

| **C.** | **DOCUMENTS CONSIDERED TO BE RELEVANT** |
| --- | --- |

| Category* | Citation of document, with indication, where appropriate, of the relevant passages | Relevant to claim No. |
| --- | --- | --- |
| X | 相玉倩 (XIANG, Yuqian). "正常和子痫前期胎盘的DNA甲基化组学研究 (Non-official translation: Multi-Omics Study of Placenta Development in Normal Pregnancies and Placenta-Associated to Preeclampsia)" 中国博士学位论文全文数据库 医药卫生科技辑 *(Chinese Doctoral Dissertations Full-Text Database, Medical and Health Sciences)*, No. 01, 15 January 2016 (2016-01-15), E068-18 | 1-51 |
| X | KULKARNI, A. et al. "Global DNA Methylation Patterns in Placenta and Its Association with Maternal Hypertension in Pre-Eclampsia" *DNA and Cell Biology*, Vol. 30, No. 2, 31 December 2011 (2011-12-31), pp. 79-84 | 1-51 |
| X | YUEN, R.KC et al. "DNA methylation profiling of human placentas reveals promoter hypomethylation of multiple genes in early-onset preeclampsia" *European Journal of Human Genetics*, Vol. 18, 05 May 2010 (2010-05-05), pp. 1006-1012 | 1-51 |

| ☑ Further documents are listed in the continuation of Box C. | ☑ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **25 December 2020** | **13 January 2021** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2020/120654**

**C.    DOCUMENTS CONSIDERED TO BE RELEVANT**

| Category* | Citation of document, with indication, where appropriate, of the relevant passages | Relevant to claim No. |
|---|---|---|
| X | BLAIR, J.D. et al. "Widespread DNA hypomethylation at gene enhancer regions in placentas associated with early-onset pre-eclampsia" *Molecular Human Reproduction*, Vol. 19, No. 10, 13 June 2013 (2013-06-13), pp. 697-708 | 1-51 |
| A | CN 109689896 A (LIFECODEXX AG) 26 April 2019 (2019-04-26) entire document | 1-51 |
| A | CN 107223159 A (LIFECODEXX AG) 29 September 2017 (2017-09-29) entire document | 1-51 |

Form PCT/ISA/210 (second sheet) (January 2015)

| **INTERNATIONAL SEARCH REPORT** | International application No. |
| --- | --- |
| | **PCT/CN2020/120654** |

| **Box No. I** | **Nucleotide and/or amino acid sequence(s) (Continuation of item 1.c of the first sheet)** |
| --- | --- |

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

| | | | | | | | | |
|---|---|---|---|---|---|---|---|---|
| **INTERNATIONAL SEARCH REPORT** | | | | | International application No. | | | |
| **Information on patent family members** | | | | | **PCT/CN2020/120654** | | | |

| Patent document cited in search report | | | Publication date (day/month/year) | Patent family member(s) | | | Publication date (day/month/year) |
|---|---|---|---|---|---|---|---|
| CN | 109689896 | A | 26 April 2019 | KR | 20180093902 | A | 22 August 2018 |
| | | | | EP | 3168309 | A1 | 17 May 2017 |
| | | | | SG | 11201803190 S | A | 30 May 2018 |
| | | | | IL | 258796 | D0 | 28 June 2018 |
| | | | | EP | 3168309 | B8 | 03 June 2020 |
| | | | | US | 2019249249 | A1 | 15 August 2019 |
| | | | | EP | 3374519 | A1 | 19 September 2018 |
| | | | | WO | 2017081047 | A1 | 18 May 2017 |
| | | | | CA | 3042946 | A1 | 18 May 2017 |
| | | | | EP | 3168309 | B1 | 08 April 2020 |
| | | | | DK | 3168309 | T3 | 22 June 2020 |
| | | | | JP | 2018533953 | A | 22 November 2018 |
| | | | | BR | 112018008532 | A2 | 30 October 2018 |
| | | | | AU | 2016351889 | A1 | 28 June 2018 |
| CN | 107223159 | A | 29 September 2017 | EP | 3140421 | B8 | 06 May 2020 |
| | | | | CA | 2985134 | A1 | 12 November 2015 |
| | | | | RU | 2016147914 | A3 | 27 December 2018 |
| | | | | EP | 3140421 | A1 | 15 March 2017 |
| | | | | EP | 3521454 | A1 | 07 August 2019 |
| | | | | JP | 6681841 | B2 | 15 April 2020 |
| | | | | WO | 2015169947 | A1 | 12 November 2015 |
| | | | | EP | 3140421 | B1 | 25 March 2020 |
| | | | | IL | 248637 | D0 | 31 January 2017 |
| | | | | SG | 11201608993 R | A | 29 November 2016 |
| | | | | US | 2017314073 | A1 | 02 November 2017 |
| | | | | RU | 2016147914 | A | 18 June 2018 |
| | | | | WO | 2015169947 | A9 | 30 December 2015 |
| | | | | AU | 2015257654 | A1 | 10 November 2016 |
| | | | | JP | 2017514499 | A | 08 June 2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Current Protocols in Molecular Biology. John Wiley & Sons, 1989, 6.3.1-6.3.6
- **PLONGTHONGKUM et al.** *Nature Reviews Genetics,* 2014, vol. 15 (10), 647-61
- **CONSORTIUM et al.** *Nature Biotechnology,* 2016, vol. 34 (7), 726
- Current Protocols in Molecular Biology. Wiley
- Molecular Cloning: A Laboratory Manual. Cold Spring Harbor Laboratory Press